# EUROPEAN PATENT APPLICATION

(11) **EP 4 086 632 A1**
(43) Date of publication of application: **09.11.2022**
(21) Application number: 20908619.8
(22) Date of filing: 30.12.2020
(51) Int. Cl.: G01N 33/68, G01N 30/72, G01N 30/96, G01N 27/64, C12Q 1/04, G01N 30/88

(54) **DIRECT DETECTION METHOD FOR PATHOGENIC STRAIN HAVING RESISTANCE TO CARBAPENEM ANTIBIOTICS**

(30) Priority: 30.12.2019 KR 20190177683; 31.01.2020 KR 20200012108
(71) Applicant: Seegene Medical Foundation, Seoul 04805 (KR)
(72) Inventor: CHUN, Jong Kee, Seoul 04805 (KR); BAEK, Je Hyun, Gwacheon-si Gyeonggi-do 13827 (KR); YANG, Won Suk, Bucheon-si Gyeonggi-do 14774 (KR); LEE, Saeyoung, Suwon-si Gyeonggi-do 16361 (KR); SUH, Hanseul, Yangju-si Gyeonggi-do 11515 (KR); JANG, Heejung, Seoul 02603 (KR); PARK, Yoon-Ha, Seoul 04805 (KR); HWANG, Seohyun, Seoul 01755 (KR)
(74) Representative: Schnappauf, Georg
(86) International application number: PCT/KR2020/019464
(87) International publication number: WO 2021/137645

(57) **Abstract**

The present invention relates to a method for detecting a pathogenic strain having resistance to carbapenem antibiotics in a biological sample. According to the present invention, it is possible to directly identify carbapenemases, specifically KPC, OXA, NDM, IMP, VIM and/or GES protein, by mass spectrometry, thereby making it possible to quickly determine not only whether a pathogenic strain has resistance to antibiotics, but also the type of protein involved in the resistance. According to the present invention, the physical and chemical properties of each carbapenemase *in vivo,* such as the unique N-terminal truncation length, methionine residue oxidation and disulfide bond formation in each type of carbapenemase, are identified and are reflected on reference mass values. Accordingly, it is possible to more closely detect the presence of an antibiotic-resistant strain with high reliability, and thus the present invention may be advantageously used to establish an appropriate strategy for antibiotic administration at an early stage of infection.

## Description

### Technical Field

The present invention relates to a method of directly detecting an *in vivo* active form of the carbapenemases KPC, OXA, NDM, IMP, VIM and/or GES by top-down mass spectrometry without pretreatment of a sample.

### Background Art

As the therapeutic efficacy of commercially available antibiotics has sharply decreased due to the continuous increase in antibiotic-resistant bacteria, studies have been actively conducted on strategies for improving the therapeutic efficacy by appropriate antibiotic administration to patients infected with pathogens and inducing reduction of antibiotic-resistant bacteria. Currently, Minimum Inhibitory Concentration (MIC) testing is being conducted to identify whether antibiotic resistance is present, but it takes 18 hours or more for microbial culture, which is an essential step, and has low accuracy, and hence it is impossible to achieve rapid identification and select an optimal antibiotic in the early stage of infection. Gene diagnosis techniques using real-time PCR, etc. are also limited in their application to rapid and accurate high-throughput diagnosis, because they require complicated and expensive sample pretreatment in the gene extraction and amplification process, advance information on the nucleotide sequence of the target gene is essential, and inaccurate information about antibiotic resistance is included due to detection of enzyme genes that have already lost antibiotic degradation activity.

Mass spectrometry methods, including MALDI-TOF, are low-cost and high-efficiency identification systems compared to sequencing methods based on PCR, and can offer an important means for rapid identification of microorganisms. In addition, using these methods, it is possible to achieve sample treatment after strain culture and stain identification within 10 minutes, and to quickly identify a strain with the same mass value by comparing mass data for unknown strains with mass data in a database established through mass spectrometry data.

However, conventional mass spectrometry methods cannot accurately determine the type of antibiotic resistance protein, and are cumbersome because they involve degrading the target resistance protein into peptide fragments using a protease, and then indirectly inferring the type of resistance protein through the mass values of these fragments. In addition, these methods have many problems in terms of reliability.

Accordingly, the present inventors have made efforts to provide a rapid and accurate diagnostic method for carbapenem-resistant strain infection by selecting proteins directly involved in resistance to beta-lactam antibiotics, specifically carbapenem antibiotics, and measuring the exact mass values of *in vivo* active forms of these selected proteins to establish accurate reference data for determining whether or not a carbapenem-resistant strain is present.

Throughout the specification, a number of publications and patent documents are referred to and cited. The disclosure of the cited publications and patent documents is incorporated herein by reference in its entirety to more clearly describe the state of the related art and the present invention.

### DISCLOSURE

### Technical Problem

The present inventors have made intensive research efforts to develop an efficient diagnostic method capable of rapidly determining infection with an antibiotic-resistant strain by detecting an antibiotic-degrading enzyme, which is involved in resistance to lactam-based antibiotics, in a sample in a simple and highly reliable manner. As a result, the present inventors have newly discovered that enzymes that degrade the beta-lactam antibiotic carbapenem, specifically KPC, OXA, NDM, IMP, VIM and GES proteins, are present in active forms due to truncation of some of N-terminal residues *in vivo,* and have found that, when active forms of these proteins, which account for the majority of carbapenem-degrading enzymes secreted by antibiotic-resistant strains, are directly identified through mass spectrometry, it is possible to establish an appropriate antibiotic administration strategy at an early stage of infection by rapidly and accurately determining not only whether the pathogenic strains are resistant to the antibiotics but also the type of protein involved in the resistance, thereby completing the present invention.

Therefore, an object of the present invention is to provide a method of detecting in a biological sample a pathogenic strain having resistance to carbapenem antibiotics, specifically a pathogenic strain expressing KPC, OXA, NDM, IMP, VIM and/or GES.

Other objects and advantages of the present invention will be more apparent by the following detailed description of the invention, the claims and the accompanying drawings.

### Technical Solution

According to one aspect of the present invention, there is provided a method for detecting in a biological sample a pathogenic strain having resistance to carbapenem antibiotics, comprising:
(a) isolating a protein expressed by a pathogenic strain in a biological sample isolated from a subject; and
(b) performing top-down mass spectrometry on the isolated protein,
wherein it is determined that the pathogenic strain having resistance to carbapenem antibiotics is present in the biological sample, when a protein having the same mass as *Klebsiella pneumoniae* carbapenemase (KPC) or OXA carbapenemase from which 21 or 22 amino acid residues at the N-terminus have been removed is detected as a result of the mass spectrometry or a protein having the same mass as at least one carbapenemase selected from the group consisting of New Delhi Metallo-beta-lactamase NDM), imipenemase (IMP), Verona integron-borne metallo-β-lactamase (VIM) and Guiana extended spectrum β-lactamase (GES), from which 18, 19, 20, 21 or 26 amino acid residues at the N-terminus have been removed, is detected as a result of the mass spectrometry.

The present inventors have made intensive research efforts to develop an efficient diagnostic method capable of rapidly determining infection with an antibiotic-resistant strain by detecting an antibiotic-degrading enzyme, which is involved in resistance to lactam-based antibiotics, in a sample in a simple and highly reliable manner. As a result, the present inventors have newly discovered that enzymes that degrade the beta-lactam antibiotic carbapenem, specifically KPC, OXA, NDM, IMP, VIM and GES proteins, are present in active forms due to truncation of some of N-terminal residues *in vivo,* and have found that, when active forms of these proteins, which account for the majority of carbapenem-degrading enzymes secreted by antibiotic-resistant strains, are directly identified through mass spectrometry, it is possible to establish an appropriate antibiotic administration strategy at an early stage of infection by rapidly and accurately determining not only whether the pathogenic strains are resistant to the antibiotics but also the type of protein involved in the resistance.

As used herein, the term "pathogenic strain" refers to any bacteria that act as the cause of an infection or disease, including, for example, but not limited to, *Staphylococcus aureus, Streptococcus, Escherichia coli, Klebsiella pneumoniae, Pseudomonas aeruginosa, Pseudomonas otitidis, Micrococcus luteus, Citrobacter koseri, Proteus mirabilis,* and *Mycobacterium ulcerans.*

As used herein, the expression "having resistance to antibiotics" means that a specific pathogenic microorganism can grow even in an environment in which antibiotics against the microorganism are present at high concentration or in an effective amount. Whether the pathogenic microorganism has antibiotic resistance may be determined by detecting the presence of an enzyme protein, which is secreted by the pathogenic microorganism and removes or reduces the activities of the antibiotics by degrading the antibiotics. For example, beta-lactam antibiotics that inhibit bacterial cell wall synthesis, such as penicillin, cephalosporin, monobactam, and carbapenem, are inactivated by β-lactamase so that they cannot inhibit pathogens expressing β-lactamase. Accordingly, the term "resistance" is used interchangeably with the terms "low therapeutic responsiveness" and "low prophylactic responsiveness".

As used herein, the term "treatment" refers to (a) inhibiting the progress of a disease, disorder or symptom; (b) alleviating a disease, disorder or symptom; or (c) eliminating a disease, disorder or symptom. Thus, the term "therapeutic responsiveness" refers to the degree to which beta-lactam antibiotics, including carbapenem, when administered in a therapeutically effective amount to a patient infected with a pathogenic strain, act as described above *in vivo.*

As used herein, the term "prevention" refers to inhibiting the occurrence of a disease or disorder in a subject that has never been diagnosed as having the disease or disorder, but is likely to have the disease or disorder. Thus, "prophylactic responsiveness" refers to the degree to which beta-lactam antibiotics, including carbapenem, when administered in a prophylactically effective amount to a normal person whose infection has not yet been confirmed, act to inhibit infection *in vivo.*

As used herein, the term "biological sample" refers to any samples, including, for example, but not limited to, blood, tissues, organs, cells or cell cultures, which are obtained from mammals, including humans, and contain or are likely to contain a pathogenic strain to be inhibited with beta-lactam antibiotics, including carbapenem.

As used herein, the term "subject" refers to a subject that provides a sample for examining the presence of a pathogenic strain to be inhibited with beta-lactam antibiotics such as carbapenem or whether the strain has resistance to the antibiotics, and ultimately refers to a subject to be analyzed for whether or not infection with the pathogenic strain having resistance to the antibiotics has occurred. Examples of the subject include, without limitation, humans, mice, rats, guinea pigs, dogs, cats, horses, cattle, pigs, monkeys, chimpanzees, baboons or rhesus monkeys, specifically humans. Since the composition of the present invention provides information for predicting not only therapeutic responsiveness but also prophylactic responsiveness to beta-lactam antibiotics such as carbapenem, the subject in the present invention may be a patient infected with the strain or may also be a healthy subject whose infection has not yet been confirmed.

As used herein, the term "top-down mass spectrometry" refers to an analysis that directly measures the mass value of a full-length protein without performing the process of fragmenting the protein into peptide fragments, and specifically, refers to an analysis in which fragmentation of the target protein is not performed before the protein sample is injected into a mass spectrometer. Another feature of the present invention is that the procedure is simplified by performing direct mass spectrometry for a full-length protein without randomly degrading the protein using protease such as trypsin, and it is possible to determine the presence of a target protein with remarkably high reliability within a much shorter time than a conventional method of indirectly identifying proteins by collecting mass information on fragments and collecting vast amounts of information on fragmentation trends of various proteins.

In the present specification, the expression "mass of the protein is the same" means that the mass value measured through the mass spectrometry method of the present invention is substantially the same as a reference mass value, for example, a value corresponding to the mass value of a carbapenemase whose amino acid sequence and molecular weight are known and from which 18, 19, 20, 21, 22 or 26 amino acid residues at the N-terminus have been removed. "Substantially the same" means that, for example, the measured Da value or m/z × z value is within the range of the reference mass value ± 10, more specifically within the range of the reference mass value ± 7, even more specifically within the range of the reference mass value ± 5, most specifically within the range of the reference mass value ± 3. The mass value of the carbapenem degrading enzyme, which is a criterion for determining whether the mass value is substantially the same as a reference mass value, includes a mass value of a state in which 1 to 3 methionine residues exist in an oxidized state of (i.e., increased by 16, 32 or 48 from a known mass value) or in which a disulfide bond is formed between two Cys residues (i.e., decreased by 2 from a known mass value).

According to a specific embodiment of the present invention, the method of the present invention further comprises a step of performing ion exchange chromatography on the protein isolated in step (a).

As used herein, the term "ion exchange chromatography" refers to a separation and purification method of separating a charged target substance from a heterogeneous mixture using a phenomenon in which ions or charged compounds bind to an ion exchange resin by electrostatic force. Ion exchange chromatography has ion exchange resins to which various functional groups bind, in which the anion exchange resin has a positively charged functional group and thus combines with a negatively charged target substance in the mixture by electrostatic attraction, and the cation exchange resin binds specifically to a positively charged target substance

According to a specific embodiment of the present invention, the ion exchange chromatography is any one selected from the group consisting of anion exchange chromatography, cation exchange chromatography, and a sequential combination thereof.

The anion exchange resin used in the present invention may have, for example, a diethylaminoethyl (DEAE) or quaternary ammonium functional group, but is not limited thereto, and any conventional cationic functional group that provides a positive charge to the support may be used without limitation. Strongly basic anion exchange groups include, for example, Q Sepharose Fast Flow, Q Sepharose High Performance, Resource Q, Source 15Q, Source 30Q, Mono Q, Mini Q, Capto Q, Capto Q ImpRes, Q HyperCel, Q Cermic HyperD F, Nuvia Q, UNOsphere Q, Macro-Prep High Q, Macro-Prep 25 Q, Fractogel EMD TMAE(S), Fractogel EMD TMAE Hicap (M), Fractogel EMD TMAE (M), Eshmono Q, Toyopearl QAE-550C, Toyopearl SuperQ-650C, Toyopearl GigaCap Q-650M, Toyopearl Q-600C AR, Toyopearl SuperQ-650M, Toyopearl SuperQ-650S, TSKgel SuperQ-5PW (30), TSKgel SuperQ-5PW (20) and TSKgel SuperQ-5PW, but are not limited thereto, and any anion exchange resins known in the art may be used.

The cation exchange resin used in the present invention may have, for example, a sulfone group or a carboxy group, but is not limited thereto, and any conventional cationic functional group that provides a negative charge to the support may be used without limitation. For example, the cation exchange resin may be selected from the group consisting of Fractogel, CM (carboxymethyl), SE (sulfoethyl), SP (sulfopropyl), P (phosphate), S (sulfonate), PROPAC WCX-10^{™} (Dionex), Capto S, S-Sepharose FF, Fractogel EMD SO3M, Toyopearl Megacap II SP 550C, Poros 50 HS, Poros XS, and SP-sepharose matrix, but is not limited thereto. Specifically, SP (sulfopropyl) resin may be used. As column buffer, equilibration buffer, wash buffer and elution buffer known in the art, such as sodium phosphate buffer, citrate buffer, and acetic acid buffer, may be used.

Such ion exchange chromatography may be appropriately performed depending on the charges of the proteins to be separated/purified and the order of the proteins to be separated. For example, in order to sequentially separate a positively charged protein and a negatively charged protein, cation exchange chromatography may be performed, followed by anion exchange chromatography.

According to a specific embodiment of the present invention, step (a) of the present invention is performed by adding a surfactant to the biological sample.

As described above, in the present invention, direct mass spectrometry of a full-length protein is possible by top-down mass spectrometry without a fragmentation process using a protease. The present inventors have found that, when a surfactant is added to the biological sample to be analyzed, the intact full-length protein present in the cell membrane or cytoplasm may be encapsulated, so that the target protein may be quickly and accurately identified without randomly degrading the protein by an enzyme.

In the present invention, ionic, nonionic and zwitterionic surfactants may all be used without limitation as long as they are general surfactants capable of forming micelles sufficient to encapsulate full-length proteins. Specifically, the surfactant used in the present invention is an ionic surfactant or a nonionic surfactant.

Examples of ionic surfactants that may be used in the present invention include, but are not limited to, sodium deoxycholate (DOC), Medialan A, Quaternium-60, cetylpyridinium chloride, cetylpyridinium bromide, cetyltrimetylammonium chloride, cetyltrimetylammonium bromide, and Gardinol.

Examples of nonionic surfactants that may be used in the present invention include, but are not limited to, n-octyl-β-D-glucopyranoside (OG), n-octyl-β-D-thioglucopyranoside (OTG), octyl glucose neopentyl glycol (OGNG), n-dodecyl-β-D-maltopyranoside (DDM), and n-dodecyl-β-D-thiomaltopyranoside (DDTM).

More specifically, step (a) may be performed by additionally adding a lysis buffer to the biological sample. That is, the surfactant of the present invention may be used together with a lysis buffer in the step of lysing the cells in order to isolate the protein expressed by the pathogenic strain. Specifically, the lysis buffer may be a volatile buffer.

Examples of volatile buffers that may be used in the present invention include, but are not limited to, ammonium bicarbonate, acetic acid, formic acid, ammonia, ammonium carbonate, and pyridine/triethanolamine. In addition, it is possible to use any buffer that evaporates easily into the atmosphere due to its low boiling point while maintaining hydrogen ion concentration in the sample within a certain range.

According to a specific embodiment of the present invention, the method of the present invention further comprises a sonication step between step (a) and step (b).

According to a specific embodiment of the present invention, step (b) is performed using a mass spectrometry method selected from the group consisting of matrix-assisted laser desorption/ionization time of flight (MALDI-TOF) mass spectrometry, surface enhanced laser desorption/ionization time of flight (SELDI-TOF) mass spectrometry, electrospray ionization time-of-flight (ESI-TOF) mass spectrometry, liquid chromatography-mass spectrometry (LC-MS), and liquid chromatography-mass spectrometry/mass spectrometry (LC-MS/MS). More specifically, it is performed using MALDI-TOF mass spectrometry.

MALDI-TOF mass spectrometry is a method in which a sample supported by a matrix is desorbed and ionized by irradiation with a laser, and then the molecular weights of the generated ions are analyzed by measuring the time (Time-of-Flight) taken for the ions to reach a detector. According to this method, it is possible to quickly and accurately measure the mass of a large biomolecule such as a protein, because fragmentation of the target material does not occur. When the ionized molecule is accelerated by an electric field and the flight time is measured, a mass-to-charge ratio (m/z) is generated, and the molecular weight of the target material may be determined through this m/z value. For example, when m/z=30,000 (z=+1) or 15,000 (z=+2), the molecular weight becomes m/z × z = 30,000.

According to a specific embodiment of the present invention, the carbapenemase is a KPC protein, and it is determined that, when a protein having the same mass as the KPC protein from which 21 amino acid residues at the N-terminus have been removed is detected as a result of the mass spectrometry, a pathogenic strain having resistance to carbapenem antibiotics is present in the biological sample.

According to a specific embodiment of the present invention, the carbapenemase is an OXA protein, and it is determined that, when a protein having the same mass as the OXA protein from which 22 amino acid residues at the N-terminus have been removed is detected as a result of the mass spectrometry, a pathogenic strain having resistance to carbapenem antibiotics is present in the biological sample.

According to a specific embodiment of the present invention, the carbapenemase is an NDM protein, and it is determined that, when a protein having the same mass as the NDM protein from which 19 or 20 amino acid residues at the N-terminus have been removed is detected as a result of the mass spectrometry, a pathogenic strain having resistance to carbapenem antibiotics is present in the biological sample.

According to a specific embodiment of the present invention, the carbapenemase is an IMP protein, and it is determined that, when a protein having the same mass as the IMP protein from which 18 to 20 amino acid residues at the N-terminus have been removed is detected as a result of the mass spectrometry, a pathogenic strain having resistance to carbapenem antibiotics is present in the biological sample.

According to a specific embodiment of the present invention, the carbapenemase is a VIM protein, and it is determined that, when a protein having the same mass as the VIM protein from which 25 or 26 amino acid residues at the N-terminus have been removed is detected as a result of the mass spectrometry, a pathogenic strain having resistance to carbapenem antibiotics is present in the biological sample.

According to a specific embodiment of the present invention, the carbapenemase is a GES protein, and it is determined that, when a protein having the same mass as the GES protein from which 18 amino acid residues at the N-terminus have been removed is detected as a result of the mass spectrometry, a pathogenic strain having resistance to carbapenem antibiotics is present in the biological sample.

According to the present invention, the present inventors have found that, in order for a carbapenemase to have an activity of degrading carbapenem *in vivo,* the carbapenemase should be present in a state in which some of the amino acid residues at the N-terminus have been removed (active form), and the length of the N-terminal residue to be removed to maintain activity differs depending on the type of enzyme. Thus, in order to accurately determine that a strain that has infected the subject actually has resistance to carbapenem, determination should be made based on whether or not the same mass value as the KPC protein from which 21 amino acid residues at the N-terminus have been removed and/or the OXA protein from which 22 amino acid residues at the N-terminus have been removed is detected, and as described below, whether or not the same mass value as the following protein is detected: the NDM protein from which 19 or 20 amino acid residues at the N-terminus have been removed, the IMP protein from which 18 to 21 amino acid residues at the N-terminus have been removed, the VIM protein from which 25 or 26 amino acid residues at the N-terminus have been removed, and/or the GES protein from which 18 amino acid residues at the N-terminus have been removed. The present invention provides significantly improved accuracy and diagnostic reliability compared to a conventional art in which the full-length amino acid mass values of these known carbapenemases are mechanically detected.

According to a specific embodiment of the present invention, it is determined that, when one or more mass values (m/z × z) selected from the group consisting of 28720, 28746, 28737, 28780, 28678, 28728, 28704, 28806, 28736, 28738, 28688, 28562, 28877, 28676, 28686, 28733, 28690, 28557, 28718, 28963, 28716, 28588, 29105, 28795, 28825, 28769, 29631, 28760, 28656, 28776, 28975, 29086, 28748, 30433, 28730 and values within the ranges of these values ±5 are detected as a result of the mass spectrometry, a pathogenic strain having resistance to carbapenem antibiotics is present in the biological sample. More specifically, in this case, the pathogenic strain present in the sample is a KPC protein-producing strain.

According to the present invention, the mass values listed above are the mass values of KPC subtype proteins from which 21 amino acid residues at the N-terminus have been removed. As described above, the present inventors have found that all subtype proteins of KPC, a carbapenemase, maintain carbapenem-degrading activity only when 21 amino acid residues at the N-terminus have been removed *in vivo.* Thus, it is determined that, when any one or more mass values corresponding thereto are detected as a result of mass spectrometry, the subject has been infected with a strain expressing one or more of the KPC subtype proteins, that is, a pathogenic strain having resistance to carbapenem antibiotics.

According to a specific embodiment of the present invention, the mass values (m/z × z) additionally include a mass value that increased by 16 or 32 from each mass value.

More specifically, the mass values (m/z × z) additionally include a mass value that decreased by 2 from each mass value.

As shown in the Examples below, the present inventors have found that one of three methionine residues (Met49, Met116 and Met151) in the KPC protein may exist in an oxidized state. Thus, it may be determined that, even when molecular weights that increased by one oxygen atom (+16) from the above-listed mass values are measured, a strain expressing the KPC protein is present in the sample. In addition, the present inventors also found that the KPC protein can exist in a state in which there is a disulfide bond between Cys68 and Cys237. Thus, it may be determined that, even when molecular weights that decreased by the detachment of two hydrogen atoms (-2) from the above-listed mass values due to the disulfide bond are measured, a strain expressing the KPC protein is present in the sample, whereby it is possible to more closely detect the presence of an antibiotic-resistant strain.

According to a specific embodiment of the present invention, it is determined that, when one or more mass values (m/z × z) selected from the group consisting of 28147, 28098, 28117, 27679, 28172, 28158, 28282, 28032, 28048, 28252, 27673, 28190, 28260, 27718, 28126, 27900, 27653, 28002, 28175, 28149, 27877, 28161, 27955, 28151, 28131, 28215, 28191, 27978 and values within the ranges of these values ±5 are detected as a result of the mass spectrometry, a pathogenic strain having resistance to carbapenem antibiotics is present in the biological sample. More specifically, in this case, the pathogenic strain present in the sample is an OXA protein-producing strain.

According to the present invention, the mass values listed above are the mass values of OXA subtype proteins from which 22 amino acid residues at the N-terminus have been removed. The present inventors have found that OXA subtype proteins maintain carbapenem-degrading activity only when 22 amino acid residues at the N-terminus have been removed *in vivo.* Thus, it is determined that, when any one or more mass values corresponding thereto are detected as a result of mass spectrometry, the subject has been infected with a strain expressing one or more of the OXA subtype proteins, that is, a pathogenic strain having resistance to carbapenem antibiotics.

According to a specific embodiment of the present invention, the mass values (m/z × z) additionally include a mass value that increased by 16, 32 or 48 from each mass value.

As shown in the Examples below, the present inventors have found that one to three of six methionine residues (Met115, Met138, Met195, Met237, Met239 and Met241) in the OXA protein may exist in an oxidized state. Thus, it may be determined that, even when molecular weights that increased by one oxygen atom (+16), two oxygen atoms (+32) or three oxygen atoms (+48) from the above-listed mass values are measured, a strain expressing the OXA protein is present in the sample, whereby it is possible to more closely diagnose infection with an antibiotic-resistant strain.

According to a specific embodiment of the present invention, it is determined that, when one or more mass values (m/z × z) selected from the group consisting of 26439, 26413, 26438, 26421, 26435, 26467, 26420, 26363, 26587, 26407, 26479, 26381, 26449, 26434, 27043, 26448, 26416, 26465, 26453, 26455, 26466, 26510, 26484, 26509, 26492, 26506, 26538, 26491, 26434, 26658, 26478, 26550, 26452, 26520, 26505, 27114, 26519, 26487, 26536, 26524, 26526, 26537 and values within the ranges of these values ±5 are detected as a result of the mass spectrometry, a pathogenic strain having resistance to carbapenem antibiotics is present in the biological sample. More specifically, in this case, the pathogenic strain present in the sample is an NDM protein-producing strain.

According to the present invention, the mass values listed above are the mass values of NDM subtype proteins from which 19 amino acid residues at the N-terminus have been removed. It may be determined that, when any one or more mass values of these mass values are detected as a result of mass spectrometry, the subject has been infected with a strain expressing one or more of the NDM subtype proteins, that is, a pathogenic strain having resistance to carbapenem antibiotics.

As used herein, the term "mass value (m/z × z)" refers to the average molecular weight of a protein to be detected or a Dalton value representing the average molecular weight. However, when the same protein is detected using other reference mass value (e.g., monoisotopic mass value) which can be inferred through this mass value (m/z × z), this detection is considered to be the same as detection performed in the present invention. For example, when whether or not there is infection with the NDM-expressing strain is determined based on the monoisotopic mass (26493.16) of NDM-1, this determination is the same as determining whether or not whether or not there is infection with the NDM-expressing strain, using 26510 m/z × z, which is an average molecular weight that can be easily inferred by those skilled in the art from the monoisotopic mass value, as a reference value.

According to a specific embodiment of the present invention, the mass values (m/z × z) further include a mass value that increased by 16 or 32 from each mass value.

As shown in the Examples below, the present inventors have found that one or two methionine residues among seven methionine residues (39, 67, 126, 129, 245, 248 and 265) in the NDM protein exist in an oxidized state. Accordingly, it may be determined that, even when molecular weights that increased by one oxygen atom (+16) or two oxygen atoms (+32) from the above-listed mass values are measured, a strain expressing the NDM protein is present in the sample, whereby it is possible to more closely detect the presence of an antibiotic-resistant strain.

More specifically, the mass values (m/z × z) further include mass values that increased by 14, 28 or 42 from each mass value.

As described below, the present inventors have found that the NDM protein may exist in a state in which one, two, or three methylations have occurred therein. Thus, it may be determined that, even when molecular weights that increased by 1 methylation (+14), 2 methylations (+28) or 3 methylations (+42) from the above-listed mass values are measured, a strain expressing the NDM protein is present in the sample.

More specifically, the mass values (m/z × z) further include a mass value that increased by 238 from each mass value.

As shown in the Examples below, peaks corresponding to the palmitoylated protein type were observed in the NDM protein. Thus, it may be determined that, even when molecular weights that increased by the mass value (+238) caused by palmitoylation are measured, a strain expressing the NDM protein is present in the sample, whereby it is possible to more closely diagnose infection with an antibiotic-resistant strain.

According to a specific embodiment of the present invention, it is determined that, when one or more mass values (m/z × z) selected from the group consisting of 25113, 25151, 25011, 25080, 25020, 25083, 25025, 24952, 25192, 25161, 24973, 24988, 25078, 25365, 25268, 25006, 25186, 24994, 25043, 24945, 25208, 25000, 24980, 25128, 25216, 24983, 25115, 25112, 25116, 25414, 25205, 25041, 25139, 25254, 25050, 24910, 25101, 25021, 25212, 25073, 24961, 25105, 24831, 25353, 25234, 24995, 25071, 25094, 25351, 25174, 25156, 25199, 25129, 24981, 25018, 25335, 25232, 24872, 24982, 25204, 24796, 25259, 25214, 25085, 25135, 25131, 25141, 25145, 25172, 25126, 24990 and values within the ranges of these values ±5 are detected as a result of the mass spectrometry, a pathogenic strain having resistance to carbapenem antibiotics is present in the biological sample. More specifically, in this case, the pathogenic strain present in the sample is an IMP protein-producing strain.

According to the present invention, the mass values listed above are the mass values of IMP subtype proteins from which 18, 19, 20 or 21 amino acid residues at the N-terminus have been removed. It may be determined that, when one or more of these mass values are detected as a result of mass spectrometry, the subject has been infected with a strain expressing one or more the IMP subtype proteins.

According to a specific embodiment of the present invention, it is determined that, when one or more mass values (m/z × z) selected from the group consisting of 25322, 25515, 25488, 25391, 25339, 25516, 25464, 25485, 25527, 25531, 25414, 25455, 25421, 25499, 25542, 25129, 25405, 25534, 25446, 25472, 25444, 25298, 25338, 25367, 25355, 25508, 25264, 25306, 25336, 25352, 25543, 25407, 25268, 25419, 25514, 25501, 25487, 25445, 25341, 25364, 25424, 25458, 25491, 25513, 25348, 25518, 25350 and values within the ranges of these values ±5 are detected as a result of the mass spectrometry, a pathogenic strain having resistance to carbapenem antibiotics is present in the biological sample. More specifically, in this case, the pathogenic strain present in the sample is a VIM protein-producing strain.

According to the present invention, the mass values listed above are the mass values of VIM subtype proteins from which 25 or 26 amino acid residues at the N-terminus have been removed. It may be determined that, when one or more of these mass values are detected as a result of the mass spectrometry, the subject has been infected with a strain expressing one or more the VIM subtype proteins.

According to a specific embodiment of the present invention, it is determined that, when one or more mass values (m/z × z) selected from the group consisting of 29217, 29274, 29186, 29216, 29247, 29246, 29259, 29203, 29231, 29201, 29273, 29261, 29237, 29248, 29230, 29213, 29275, 29278, 29221, 29194, 29338, 29232, 29227, 29251, 29202, 29175, 29369, 29661 and values within the ranges of these values ±5 are detected as a result of the mass spectrometry, a pathogenic strain having resistance to carbapenem antibiotics is present in the biological sample. More specifically, in this case, the pathogenic strain present in the sample is a GES protein-producing strain.

According to the present invention, the mass values listed above are the mass values of GES subtype proteins from which 18 amino acid residues at the N-terminus have been removed. It may be determined that, when one or more of these mass values are detected as a result of the mass spectrometry, the subject has been infected with a strain expressing one or more the GES subtype proteins.

According to a specific embodiment of the present invention, the mass value (m/z × z) further include a mass value that increased by 16 or 32 from each mass value. More specifically, the mass values (m/z × z) additionally include a mass value that decreased by 2 from each mass value.

As described below, the present inventors have found that one or two of six methionine residues (62, 95, 112, 143, 164 and 181) in the GES protein can exist in an oxidized state. Thus, it may be determined that, even when molecular weights that increased by one oxygen atom (+16) or two oxygen atoms (+32) from the above-listed mass values are measured, a strain expressing the GES protein is present in the sample. In addition, the present inventors also found that the GES protein can exist in a state in which there is a disulfide bond between Cys63 and Cys233. Thus, it may be determined that, even when molecular weights that decreased by the detachment of two hydrogen atoms (-2) from the above-listed mass values due to the disulfide bond are measured, a strain expressing the GES protein is present in the sample, whereby it is possible to more closely diagnose infection with an antibiotic-resistant strain.

### Advantageous Effects

The features and advantages of the present invention are summarized as follows:
(a) The present invention provides a method of detecting in a biological sample a pathogenic strain having resistance to carbapenem antibiotics.
(b) According to the present invention, it is possible to directly identify carbapenemase, specifically KPC, OXA, NDM, IMP, VIM and/or GES protein, by mass spectrometry, thereby making it possible to quickly determine not only whether a pathogenic strain has resistance to antibiotics, but also the type of protein involved in the resistance.
(c) According to the present invention, the physical and chemical properties of each carbapenemase *in vivo,* such as the unique N-terminal truncation length, methionine residue oxidation and disulfide bond formation in each type of carbapenemase, are identified and are reflected on reference mass values. Accordingly, it is possible to more closely detect the presence of an antibiotic-resistant strain with high reliability, and thus the present invention may be advantageously used to quickly establish an appropriate strategy for antibiotic administration at an early stage of infection.

### Brief Description of Drawings

FIG. 1 shows the results of SDS-PAGE analysis for the expression and sizes of KPC and OXA proteins derived from an antibiotic-resistant strain (FIG. 1a), and shows SDS-PAGE analysis results for the expression and sizes of three proteins (NDM, IMP and VIM) of the Metallo-β-Lactamase (hereinafter referred to as MBL) family, and the Class A carbapenemase GES protein (FIG. 1b). FIGS. 1c to 1h show the results of SDS-PAGE analysis for the expression and sizes of KPC, OXA, NDM, IMP, VIM and GES subtype proteins, respectively.
FIG. 2 shows the results of SDS-PAGE analysis for the expression and sizes of KPC and OXA proteins derived from a clinical strain (FIG. 2a), the MBL proteins NDM (FIG. 2b), IMP (FIG. 2c) and VIM (FIG. 2d), and GES-5 protein (FIG. 2e).
FIG. 3 shows the results of SDS-PAGE analysis for the expression and sizes of the MBL proteins depending on the concentration of zinc sulfate (ZnSO4).

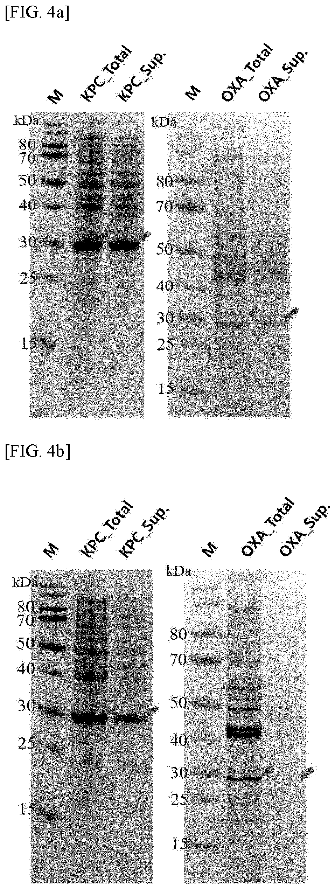

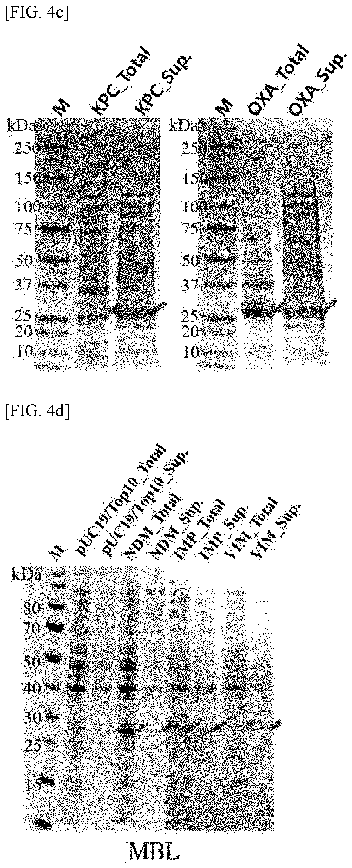

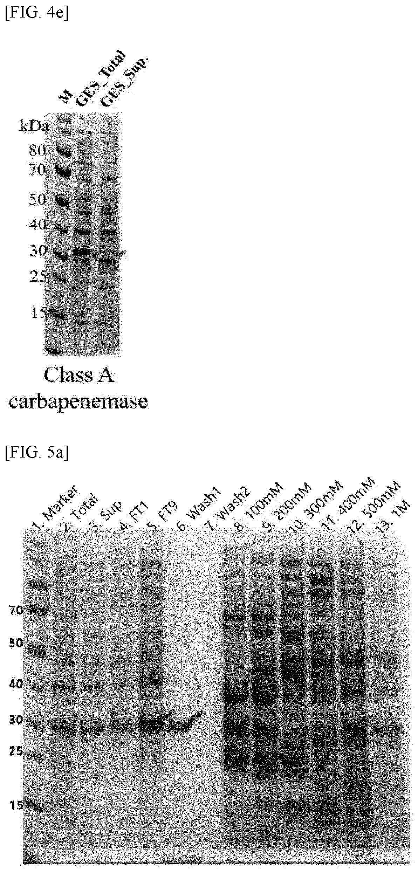

FIG. 4 shows the results of comparing the protein difference between a crude extract and a crude enzyme solution after sample pretreatment. For KPC and OXA proteins, cells were disrupted using each of a nonionic surfactant (FIG. 4a), an ionic surfactant (FIG. 4b) and a volatile buffer (FIG. 4c), the expression and solubility of each protein were analyzed by SDS-PAGE gel analysis. In addition, the expression and solubility of the MBL protein (FIG. 4d) in cells disrupted using a nonionic surfactant and the GES protein (FIG. 4e) in cells disrupted using with a volatile buffer were analyzed by SDS-PAGE gel analysis.

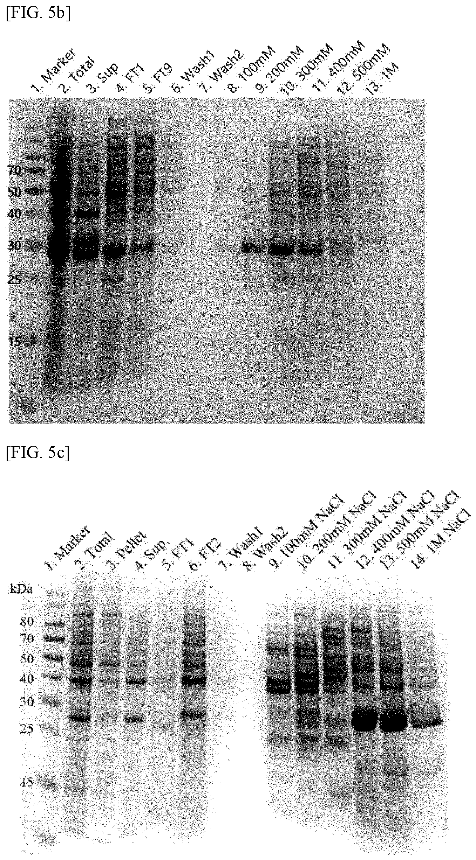

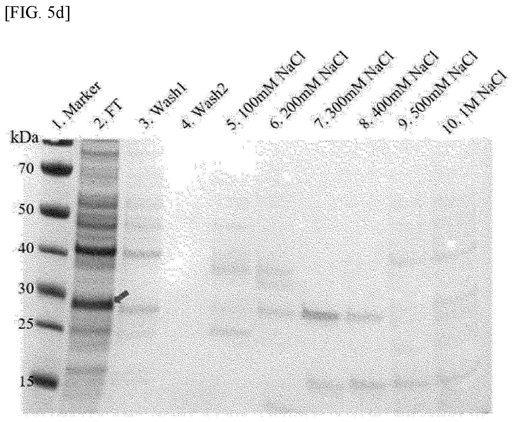

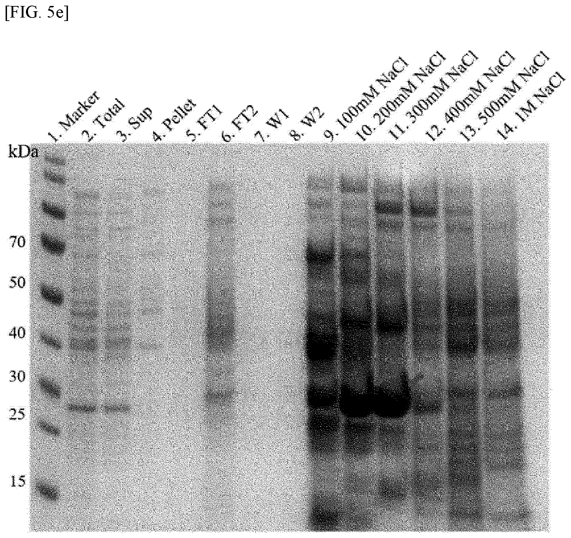

FIG. 5 shows the results of separating and purifying target proteins using ion chromatography. KPC (FIG. 5a), OXA (FIG. 5b), NDM (FIG. 5c), IMP (FIG. 5d), VIM (FIG. 5e) and GES-5 (FIG. 5f) were separated and purified using an anion exchange resin column.
FIG. 6 shows alignment results that comparatively show representative coverages (grey) for KPC protein, OXA protein, three MBL proteins, and GES protein, and oxidized methionine residues (bold and underlined) (FIGS. 6a to 6f).

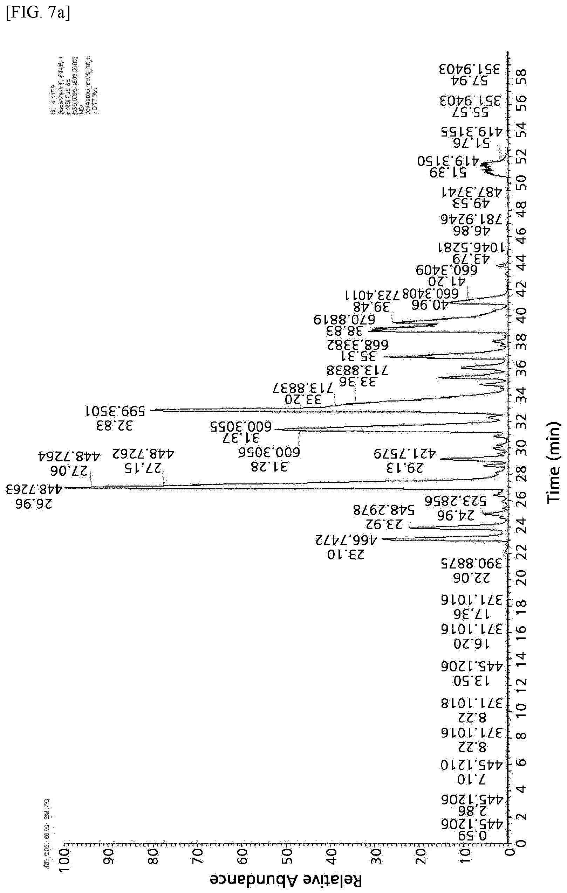

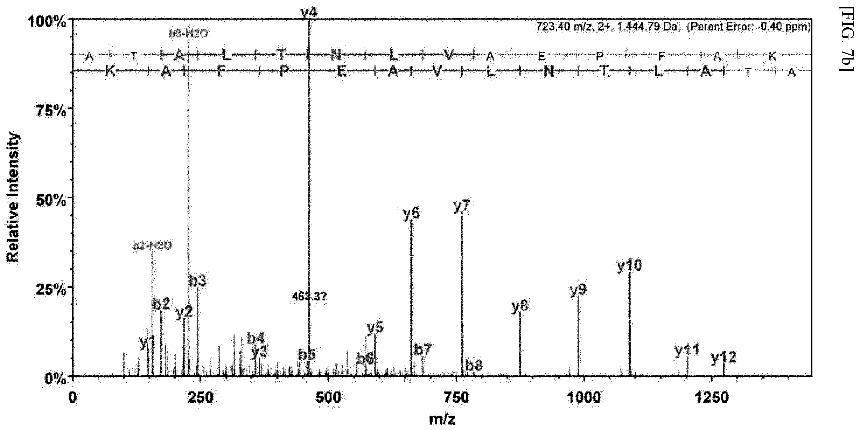

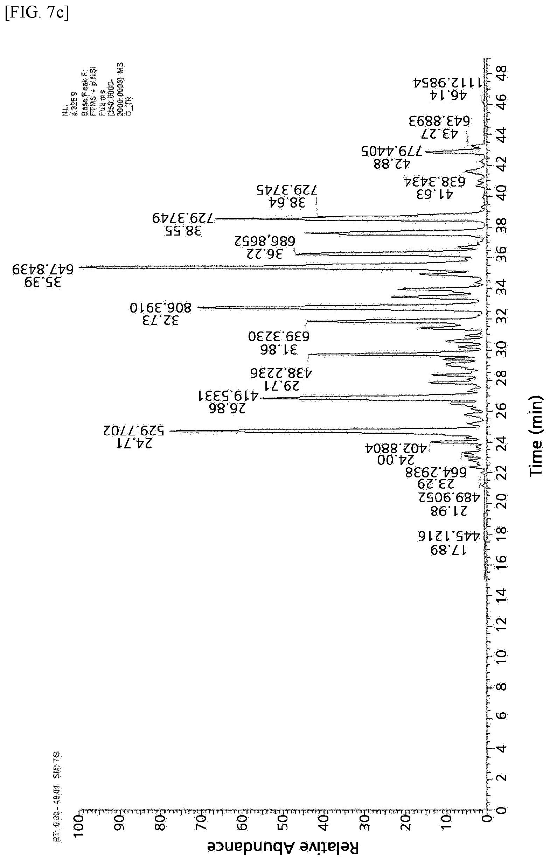

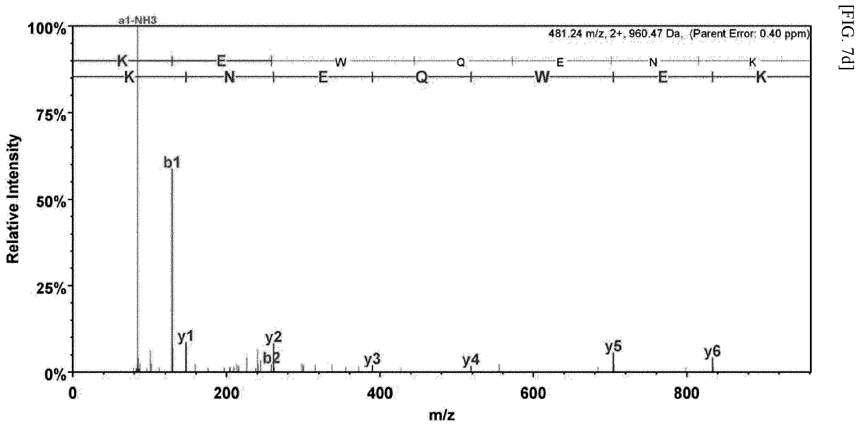

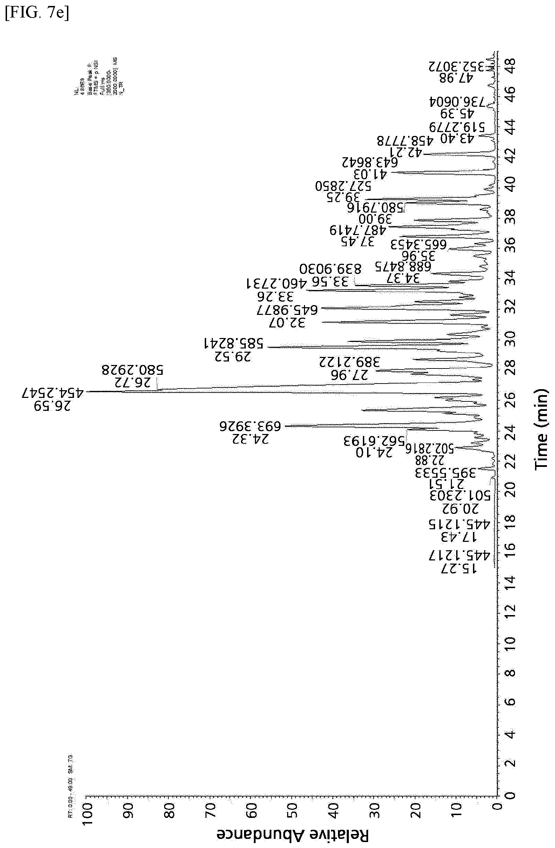

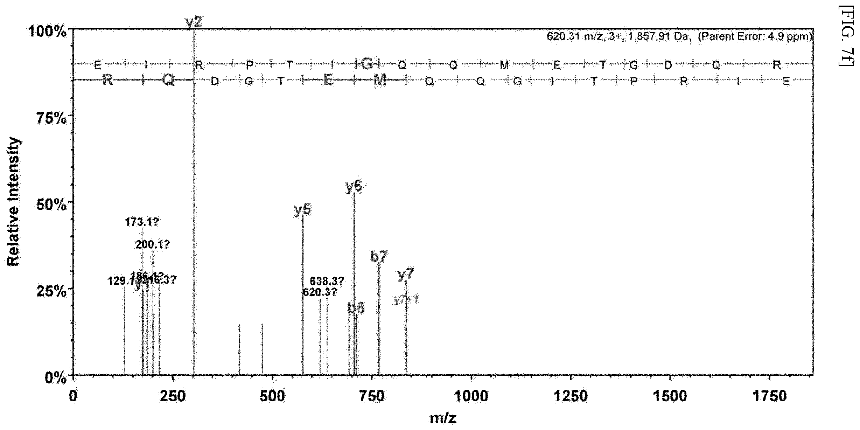

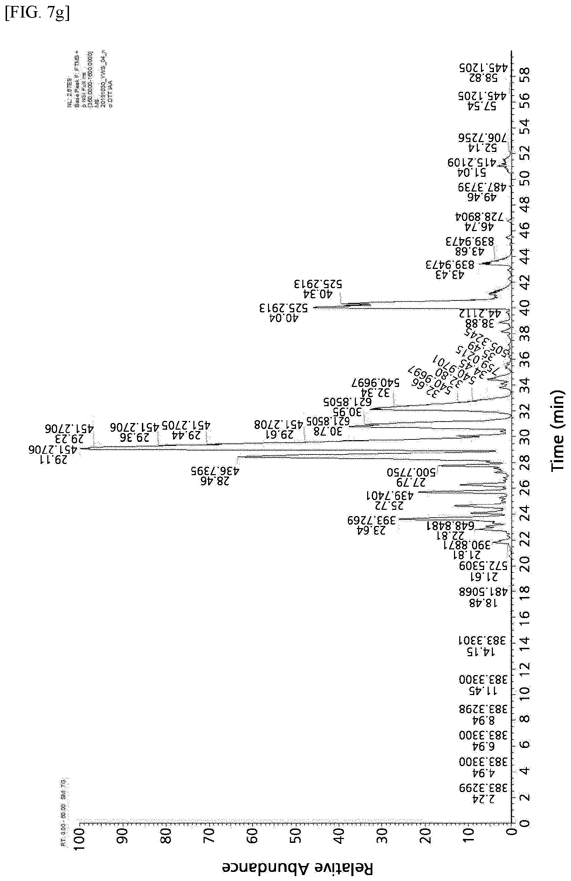

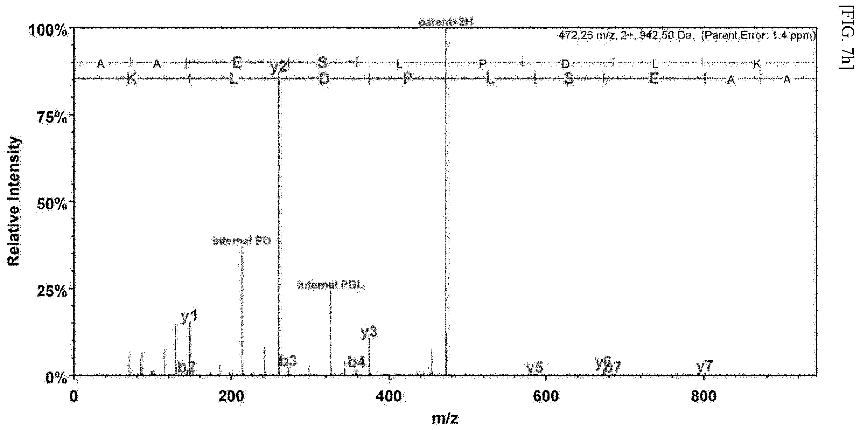

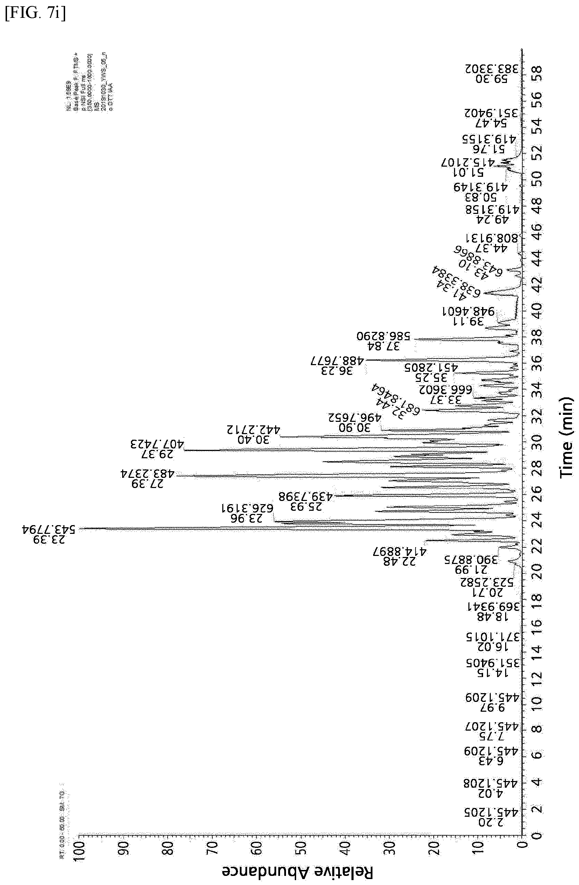

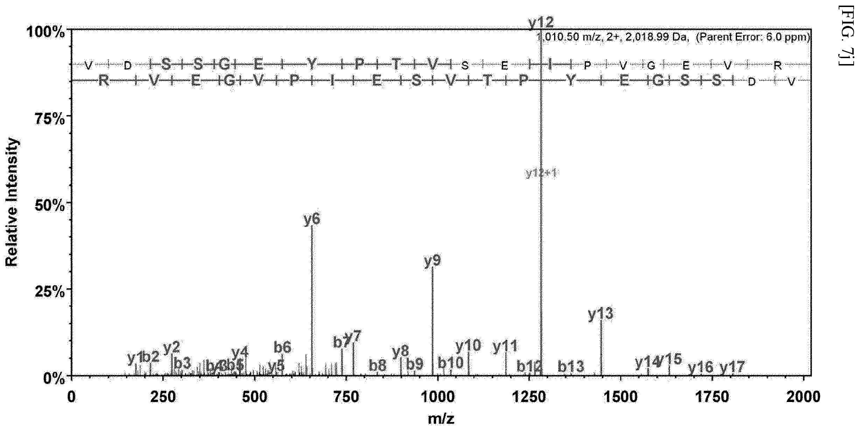

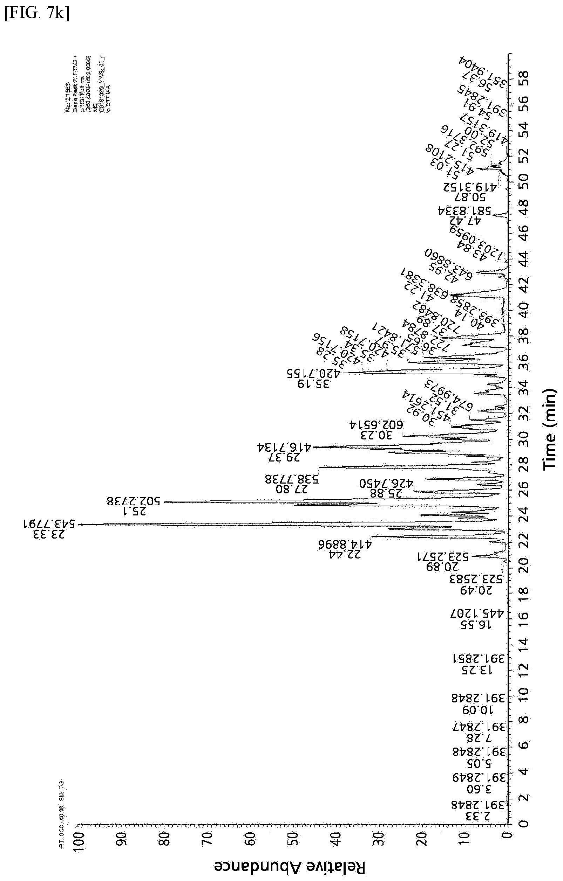

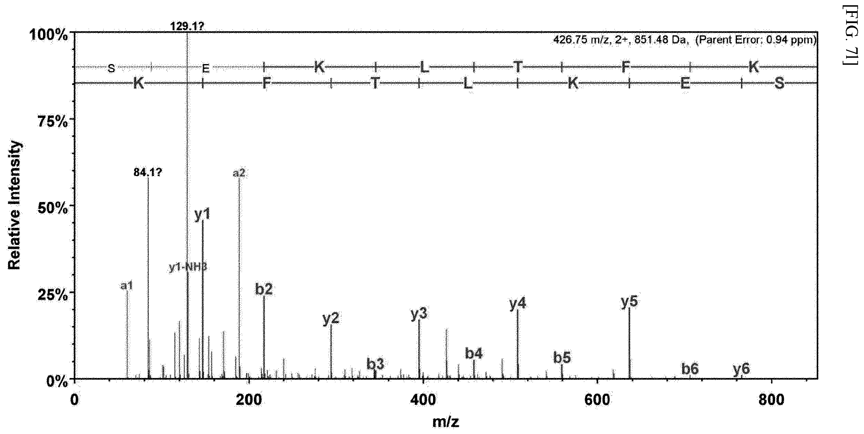

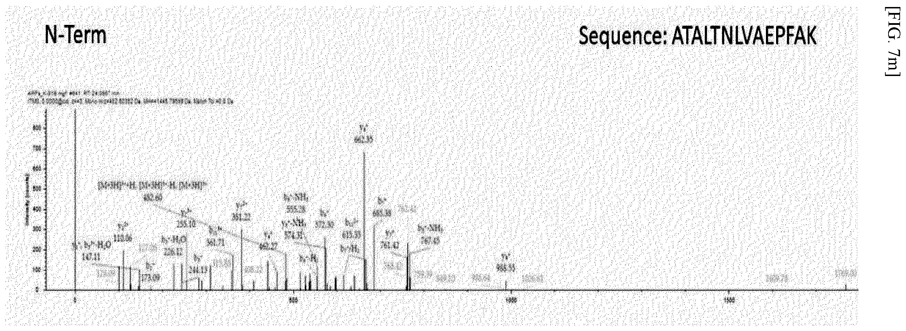

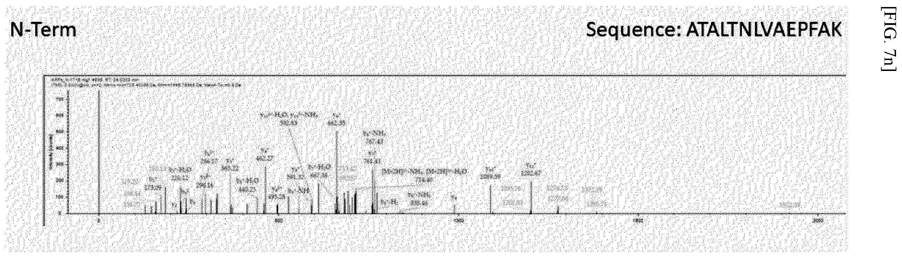

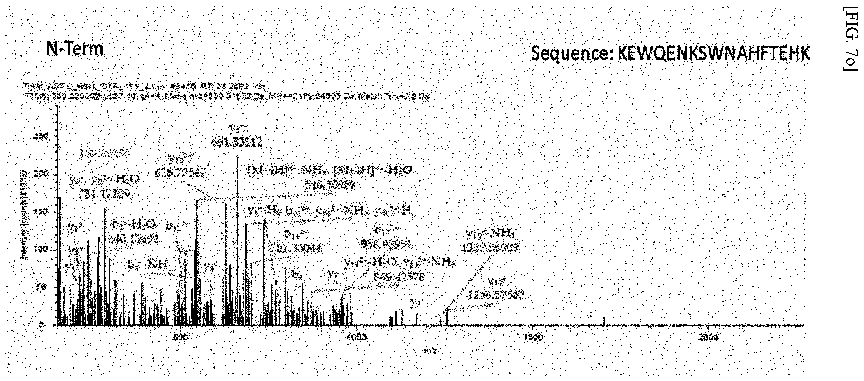

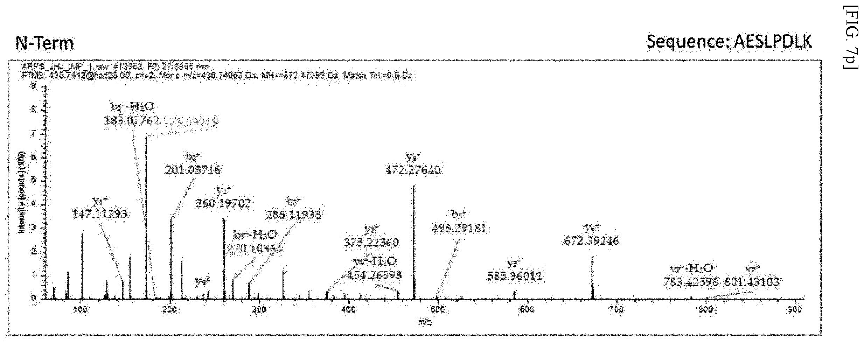

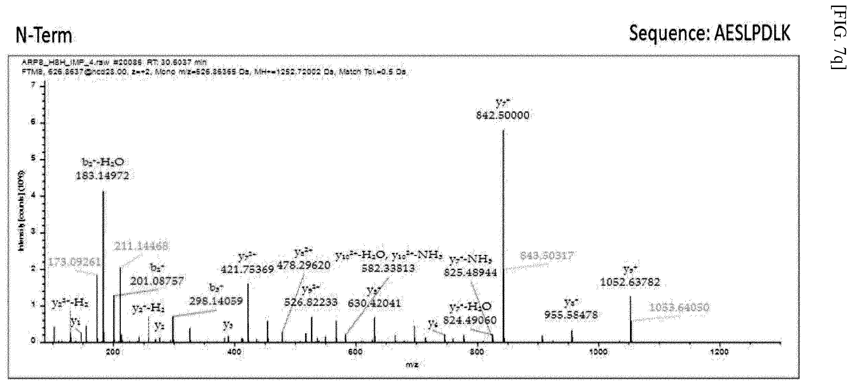

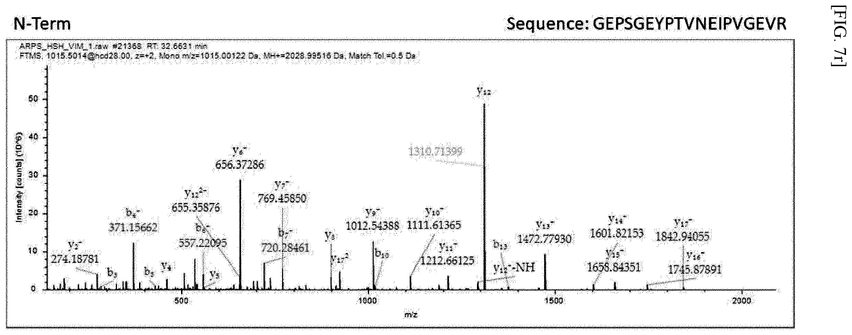

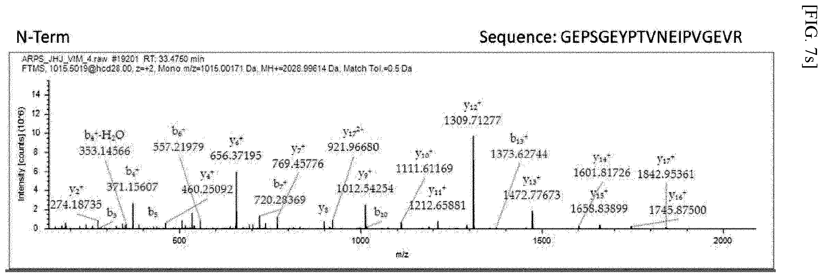

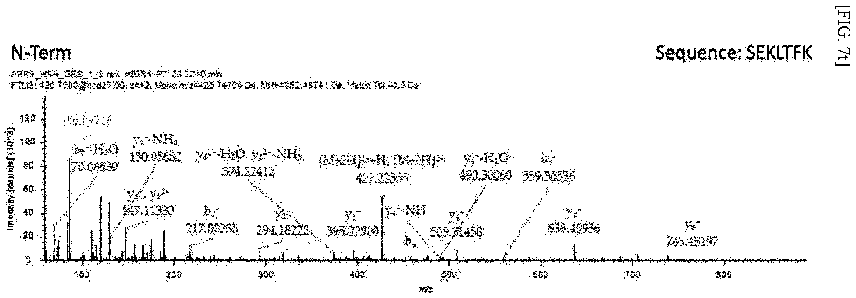

FIG. 7 shows tandem spectrum results for peptides identified as N-terminal peptides in each protein. FIG. 7a shows the separation chromatogram of the KPC peptide, and FIG. 7b shows the results of identification of the N-terminal peptide of KPC. FIG. 7c shows the separation chromatogram of the OXA peptide, and FIG. 7d shows the results of identification of the N-terminal peptide of OXA. FIG. 7e shows an example of the separation chromatogram of the NDM peptide, and FIG. 7f shows the results of identification of the N-terminal peptide of NDM. FIG. 7g shows an example of the separation chromatogram of the IMP peptide, and FIG. 7h shows the results of identification of the N-terminal peptide of IMP. FIG. 7i shows an example of the separation chromatogram of the VIM peptide, and FIG. 7j shows the results of identification of the N-terminal peptide of VIM. FIG. 7k shows an example of the separation chromatogram of the GES peptide, and FIG. 71 shows the results of identification of the N-terminal peptide of GES. FIGS. 7m to 7t show exemplary N-terminal identification results for representative subtypes of each peptide, and show the results of identification of the N-termini of KPC-3 (FIG. 7m), KPC-17 (FIG. 7n), OXA-181 (FIG. 7o), IMP-1 (FIG. 7p), IMP-4 (FIG. 7q), VIM-1 (FIG. 7r), VIM-4 (FIG. 7s), and GES-1 (FIG. 7t), respectively.
FIG. 8 shows examples of the results of multiple alignment analysis of the amino acid sequences of KPC (FIG. 8a), OXA (FIG. 8b), MBL (FIGS. 8c to 8e) proteins and GES protein (FIG. 8f), performed using the ClustalW program, the results of identification of the conservative amino acid sequences. Black boxes indicate N-terminal sequence regions.
FIG. 9 shows phylogenetic analysis results for KPC (FIG. 9a), OXA (FIG. 9b), NDM (FIG. 9c), IMP (FIG. 9d), VIM (FIG. 9e) and GES (FIG. 9f) proteins.

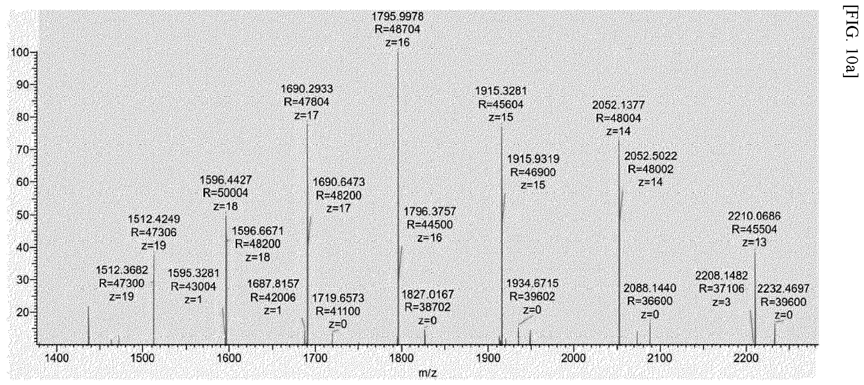

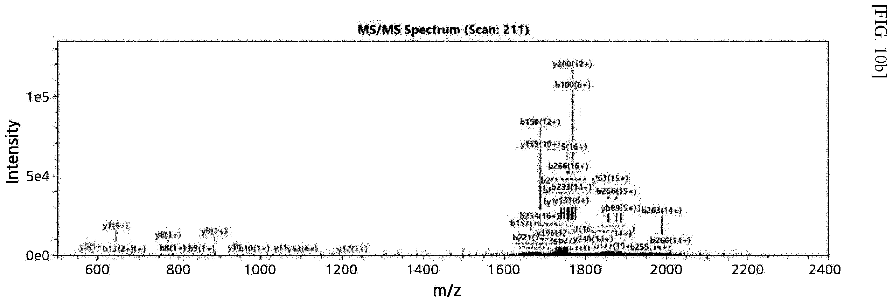

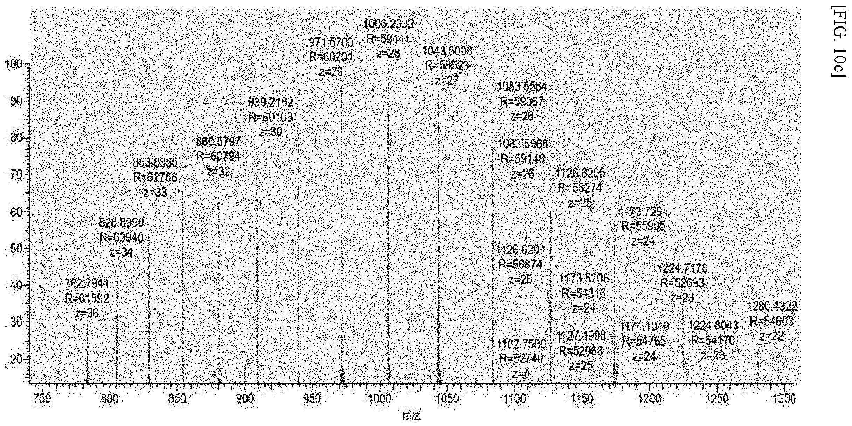

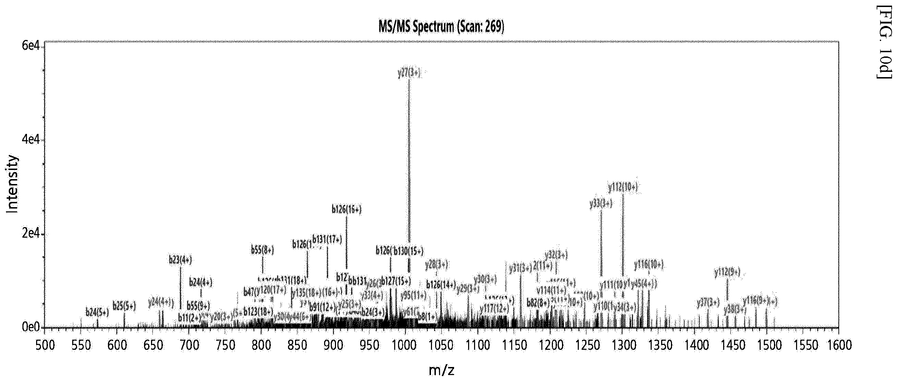

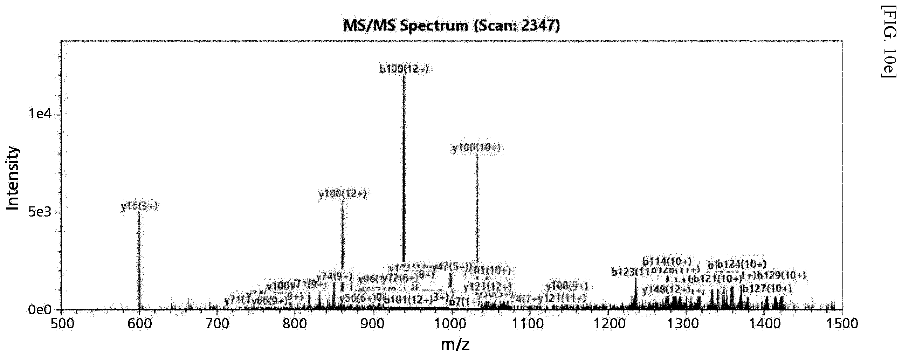

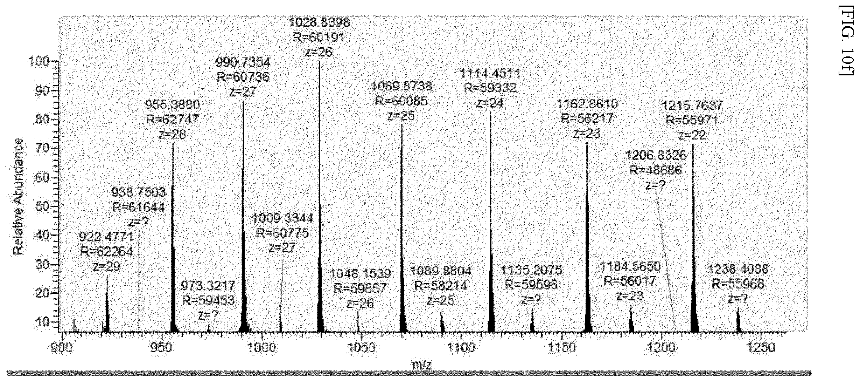

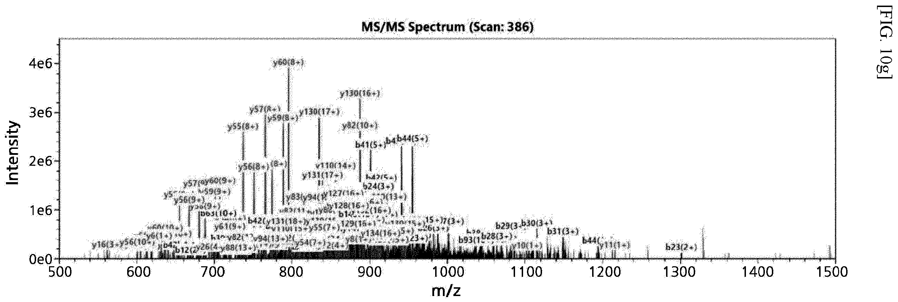

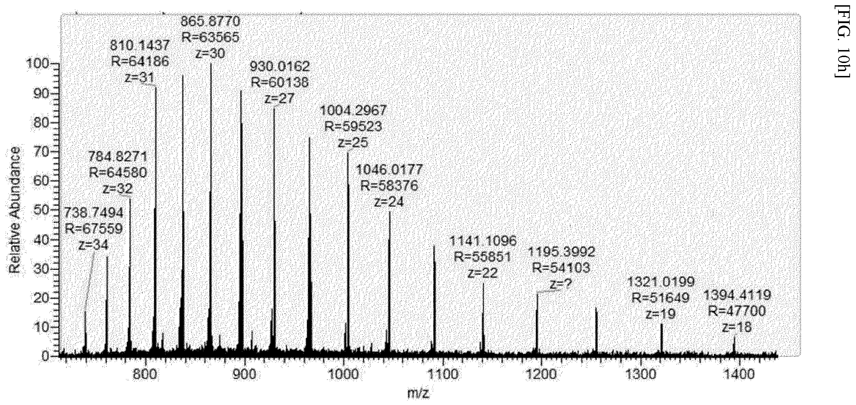

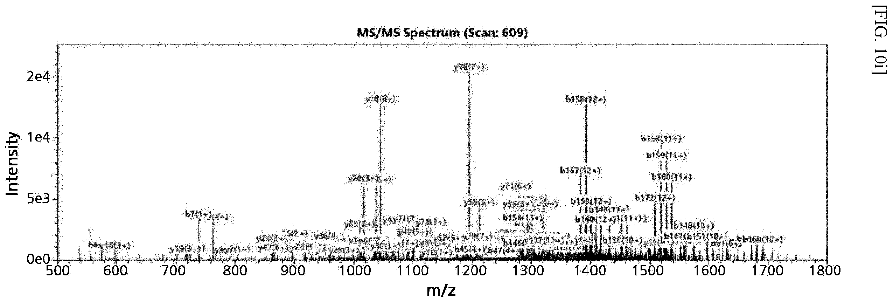

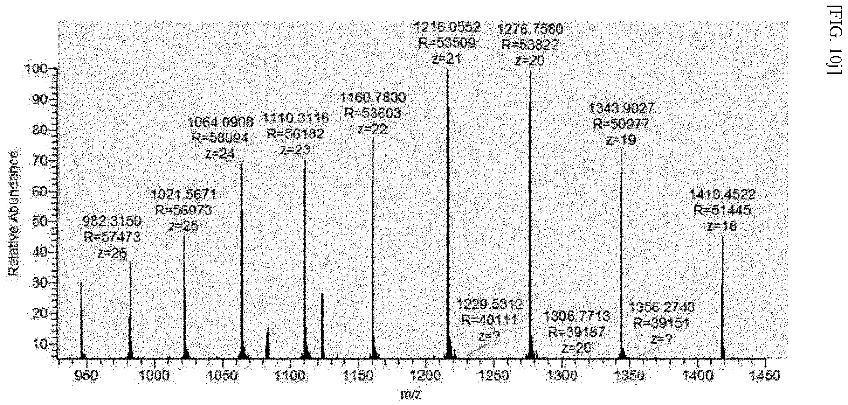

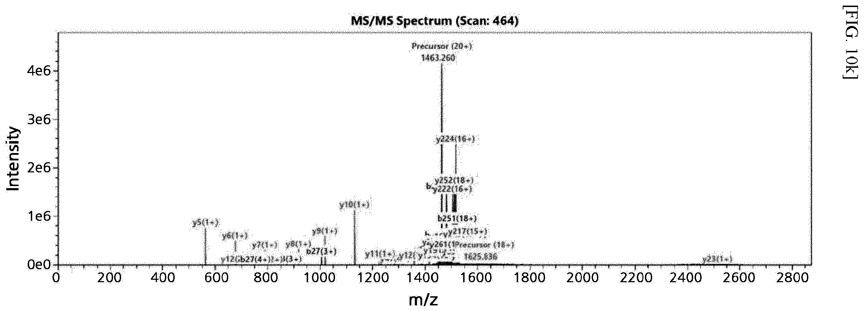

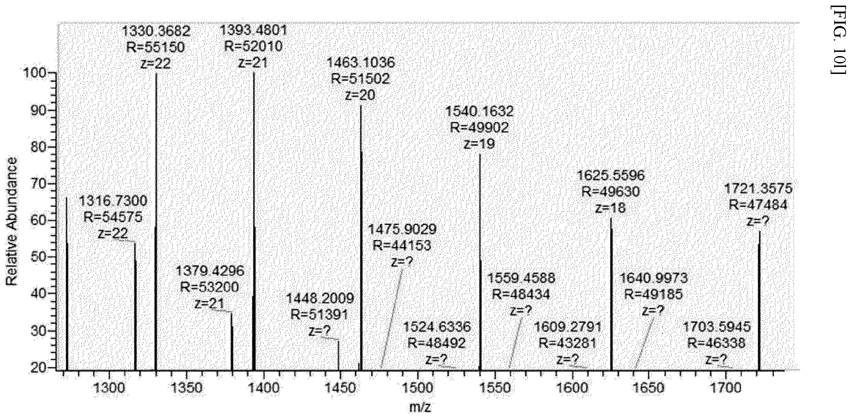

FIG. 10 shows the results of identification of KPC, OXA, NDM, IMP, VIM and GES proteins, performed using a high-resolution mass spectrometer. FIG. 10a shows the mass spectrum (monoisotopic mass: 28,700.69 m/z × z, average molecular weight: 28,718.13 m/z × z) of a multi-charged KPC protein, and FIG. 10b shows the tandem spectrum for KPC protein ions with a charge state of +17, and the identification result (E = 1.8E-9) for the 22-293 a.a. sequence. FIG. 10c shows the mass spectrum (monoisotopic mass: 28,129.28 m/z × z, average molecular weight: 28,146.69 m/z × z) of a multi-charged OXA protein, and FIG. 10d shows the tandem spectrum for OXA protein ions with a charge state of +27, and the identification result (E = 3.47E-42) for the 23-265 a.a. sequence. FIG. 10e shows the mass spectrum of a multi-charged NDM protein, and FIG. 10f shows the tandem spectrum for NDM protein ions with a charge state of the +25, and the identification result for the 20-270 a.a. sequence. FIG. 10g shows the mass spectrum of a multi-charged IMP protein, and FIG. 10h shows the tandem spectrum for IMP protein ions with a charge state of +30, and the identification result (E = 2.99E-76) for the 19-246 a.a. sequence. FIG. 10i shows the mass spectrum of a multi-charged VIM protein, and FIG. 10j shows the tandem spectrum for VIM protein ions with a charge state of +20, and the identification result (E = 4.76E-49) for the 27-266 a.a. sequence. FIG. 10k shows the mass spectrum of a multi-charged IMP protein, and FIG. 101 shows the tandem spectrum for GES protein ions with a charge state of +20, and the identification result (E = 7.12E-8) for the 19-287 a.a. sequence.
FIG. 11 shows the modified mass values of NDM protein, obtained by top-down mass spectrometry through high-resolution mass spectrometry. FIG. 11a shows the mass value of the palmitoylated protein type, and FIG. 11b shows the results for the methylated protein of the NDM protein and the resulting elution time difference.
FIG. 12 shows sequence coverages for KPC (FIG. 12a), OXA (FIG. 12b), NDM (FIG. 12c), IMP (FIG. 12d), VIM (FIG. 12e) and GES (FIG. 12f) proteins, obtained by top-down mass spectrometry through a high-resolution mass spectrometer.
FIG. 13 shows examples of mass spectrometry spectra of KPC protein (FIG. 13a), OXA protein (FIG. 13b), the MBL proteins NDM (FIG. 13c), IMP (FIG. 13d) and VIM (FIG. 13e), and GES protein (FIG. 13f), obtained using a low-resolution mass spectrometer (MALDI-TOF).

### Mode for Invention

Hereinafter, the present invention will be described in more detail with reference to examples. These examples serve merely to illustrate the present invention in more detail, and it will be apparent to those skilled in the art that the scope of the present invention according to the subject matter of the present invention is not limited by these examples.

### Examples

### Experimental Methods

### Example 1. Cloning of β-lactam antibiotic resistance gene and construction of antibiotic-resistant strain

Based on information about the gene sequences (European Molecular Biology Laboratory (EMBL) nucleotide sequence database accession numbers: KPC = CP026395.1, 882nt, OXA = AY236073.2, 798nt, NDM = CAZ39946.1, 813nt, VIM = AY884050.1, 801nt, IMP = AB616660.2, 741nt, GES = DQ236171.1, 864nt) obtained from the genes for carbapenemases, desired gene sequences were prepared by synthesis (Table 1). Using the synthetic genes, the following primers were prepared.
1) KPC
   Primer 1: 5'- AACTGCAGGATGTCACTGTATCGCCGTCTA-3' (30mer)
   Primer 2: 5'- GGAATTCTTACTGCCCGTTGACGCC-3' (25mer)
2) OXA
   Primer 1: 5'- AACTGCAGGATGCGTGTATTAGCCTTATCGG-3' (31mer)
   Primer 2: 5'- GGAATTCCTAGGGAATAATTTTTTCCTGTTTGA-3' (33mer)
3) NDM
   Primer 1: 5' - AAC TGC AGG ATG GAA TTG CCC AAT ATT ATG CA - 3' (32mer)
   Primer 2: 5' - GGA ATT CTC AGC GCA GCT TGT CGG - 3' (24mer)
4) IMP
   Primer 1: 5' - AAC TGC AGG ATG AGC AAG TTA TCT GTA TTC TTT ATA T - 3' (37mer)
   Primer 2: 5' - GGA ATT CTT AGT TGC TTG GTT TTG ATG GTT TTT - 3' (33mer)
5) VIM
   Primer 1: 5' - AAC TGC AGG ATG TTC AAA CTT TTG AGT AAG TTA TTG - 3' (36mer)
   Primer 2: 5' - GGA ATT CCT ACT CAA CGA CTG AGC GAT T - 3' (28mer)
6) GES
   Primer 1: 5' - AAC TGC AGG ATG CGC TTC ATT CAC GCA CTA T - 3' (31mer)
   Primer 2: 5' - CGG AAT TCC TAT TTG TCC GTG CTC AGG AT - 3' (29mer)

Restriction enzyme sites for cloning were added to the primers, and an open reading frame (ORF) was created to induce expression directly in a cloning vector.

Each target gene was amplified by PCR from three types of template DNA. For PCR, a total of 50 µl of a PCR reaction solution was prepared using 3 µl of template DNA, 1.25 µl of 5' primer, 1.25 µl of 3' primer, 1 µl of dNTPs, 10 µl of 5X buffer, and 5X GC enhance buffer, and then PCR was performed under the following conditions: 1) denaturation - at 98°C for 10 sec; 2) annealing- at 57°C for 30 sec; 3) extension - at 72°C for 30 sec. Cloning for construction of a recombinant plasmid containing each target gene was performed as follows: 1) For the insert gene and the vector, both ends of the DNA were cut into sticky ends using restriction enzymes, and 2) the insert gene was ligated into the vector using DNA ligase. 3) Thereafter, the vector was transformed into *E. coli* (*E. coli* Top10), and 4) recombinant *E. coli* was selected by the white/blue screening method. 5) A recombinant plasmid was extracted from the selected strain, and 6) the extracted plasmid was treated with restriction enzymes, and the DNA size was determined. 7) Finally, the inserted gene was confirmed through DNA sequencing.

**[Table 1]**

| Carbapenemases | |
|---|---|
| KPC | ATGTCACTGTATCGCCGTCTAGTTCTGCTGTCTTGTCTCTCATGGCCGCTGGCTGGCTTTTCT GCCACCGCGCTGACCAACCTCGTCGCGGAACCATTCGCTAAACTCGAACAGGACTTTGGC GGCTCCATCGGTGTGTACGCGATGGATACCGGCTCAGGCGCAACTGTAAGTTACCGCGCTG AGGAGCGCTTCCCACTGTGCAGCTCATTCAAGGGCTTTCTTGCTGCCGCTGTGCTGGCTCG CAGCCAGCAGCAGGCCGGCTTGCTGGACACACCCATCCGTTACGGCAAAAATGCGCTGGT TCCGTGGTCACCCATCTCGGAAAAATATCTGACAACAGGCATGACGGTGGCGGAGCTGTC CGCGGCCGCCGTGCAATACAGTGATAACGCCGCCGCCAATTTGTTGCTGAAGGAGTTGGG CGGCCCGGCCGGGCTGACGGCCTTCATGCGCTCTATCGGCGATACCACGTTCCGTCTGGAC CGCTGGGAGCTGGAGCTGAACTCCGCCATCCCAGGCGATGCGCGCGATACCTCATCGCCG CGCGCCGTGACGGAAAGCTTACAAAAACTGACACTGGGCTCTGCACTGGCTGCGCCGCAG CGGCAGCAGTTTGTTGATTGGCTAAAGGGAAACACGACCGGCAACCACCGCATCCGCGCG GCGGTGCCGGCAGACTGGGCAGTCGGAGACAAAACCGGAACCTGCGGAGTGTATGGCACG GCAAATGACTATGCCGTCGTCTGGCCCACTGGGCGCGCACCTATTGTGTTGGCCGTCTACA CCCGGGCGCCTAACAAGGATGACAAGCACAGCGAGGCCGTCATCGCCGCTGCGGCTAGAC TCGCGCTCGAGGGATTGGGCGTCAACGGGCAGTAA |
| OXA | ATGCGTGTATTAGCCTTATCGGCTGTGTTTTGGTGGCATCGATTATCGGAATGCCTGCGGT AGCAAAGGAATGGCAAGAAAACAAAAGTTGGAATGCTCACTTTACTGAACATAAATCACA |
| | GGGCGTAGTTGTGCTCTGGAATGAGAATAAGCAGCAAGGATTTACCAATAATCTTAAACG GGCGAACCAAGCATTTTTACCCGCATCTACCTTTAAAATTCCCAATAGCTTGATCGCCCTC GATTTGGGCGTGGTTAAGGATGAACACCAAGTCTTTAAGTGGGATGGACAGACGCGCGAT ATCGCCACTTGGAATCGCGATCATAATCTAATCACCGCGATGAAATATTCAGTTGTGCCTG TTTATCAAGAATTTGCCCGCCAAATTGGCGAGGCACGTATGAGCAAGATGCTACATGCTTT CGATTATGGTAATGAGGACATTTCGGGCAATGTAGACAGTTTCTGGCTCGACGGTGGTATT CGAATTTCGGCCACGGAGCAAATCAGCTTTTTAAGAAAGCTGTATCACAATAAGTTACACG TATCGGAGCGCAGCCAGCGTATTGTCAAACAAGCCATGCTGACCGAAGCCAATGGTGACT ATATTATTCGGGCTAAAACTGGATACTCGACTAGAATCGAACCTAAGATTGGCTGGTGGGT CGGTTGGGTTGAACTTGATGATAATGTGTGGTTTTTTGCGATGAATATGGATATGCCCACA TCGGATGGTTTAGGGCTGCGCCAAGCCATCACAAAAGAAGTGCTCAAACAGGAAAAAATT ATTCCCTAG |
| NDM | ATGGAATTGCCCAATATTATGCACCCGGTCGCGAAGCTGAGCACCGCATTAGCCGCTGCA TTGATGCTGAGCGGGTGCATGCCCGGTGAAATCCGCCCGACGATTGGCCAGCAAATGGAA ACTGGCGACCAACGGTTTGGCGATCTGGTTTTCCGCCAGCTCGCACCGAATGTCTGGCAG CACACTTCCTATCTCGACATGCCGGGTTTCGGGGCAGTCGCTTCCAACGGTTTGATCGTC AGGGATGGCGGCCGCGTGCTGGTGGTCGATACCGCCTGGACCGATGACCAGACCGCCCAG ATCCTCAACTGGATCAAGCAGGAGATCAACCTGCCGGTCGCGCTGGCGGTGGTGACTCAC GCGCATCAGGACAAGATGGGCGGTATGGACGCGCTGCATGCGGCGGGGATTGCGACTTAT GCCAATGCGTTGTCGAACCAGCTTGCCCCGCAAGAGGGGATGGTTGCGGCGCAACACAGC CTGACTTTCGCCGCCAATGGCTGGGTCGAACCAGCAACCGCGCCCAACTTTGGCCCGCTC AAGGTATTTTACCCCGGCCCCGGCCACACCAGTGACAATATCACCGTTGGGATCGACGGC ACCGACATCGCTTTTGGTGGCTGCCTGATCAAGGACAGCAAGGCCAAGTCGCTCGGCAAT CTCGGTGATGCCGACACTGAGCACTACGCCGCGTCAGCGCGCGCGTTTGGTGCGGCGTTC CCCAAGGCCAGCATGATCGTGATGAGCCATTCCGCCCCCGATAGCCGCGCCGCAATCACT |
| | CATACGGCCCGCATGGCCGACAAGCTGCGCTGA |
| IMP | ATGAGCAAGTTATCTGTATTCTTATATTTTGTTTGCAGCATTGCTACCGCAGCAGAG TCTTTGCCAGATTTAAAAATTGAAAAGCTTGATGAAGGCGTTTATGTTCATACTTCGTTT GAAGAAGTTAACGGGTGGGGCGTTGTTCCTAAACATGGTTTGGTGGTTCTTGTAAATGCT GAGGCTTACCTAATTGACACTCCATTTACGGCTAAAGATACTGAAAAGTTAGTCACTTGG TTTGTGGAGCGTGGCTATAAAATAAAAGGCAGCATTTCCTCTCATTTTCATAGCGACAGC ACGGGCGGAATAGAGTGGCTTAATTCTCGATCTATCCCCACGTATGCATCTGAATTAACA AATGAACTGCTTAAAAAAGACGGTAAGGTTCAAGCCACAAATTCATTTAGCGGAGTTAAC TATTGGCTAGTTAAAAATAAAATTGAAGTTTTTTATCCAGGCCCGGGACACACTCCAGAT AACGTAGTGGTTTGGTTGCCTGAAAGGAAAATATTATTCGGTGGTTGTTTTATTAAACCG TACGGTTTAGGCAATTTGGGTGACGCAAATATAGAAGCTTGGCCAAAGTCCGCCAAATTA TTAAAGTCCAAATATGGTAAGGCAAAACTGGTTGTTCCAGGTCACAGTGAAGTTGGAGAC GCATCACTCTTGAAACTTACATTAGAGCAGGCGGTTAAAGGGTTAAACGAAAGTAAAAAA CCATCAAAACCAAGCAACTAA |
| VIM | ATGTTCAAACTTTTGAGTAAGTTATTGGTCTATTTGACCGCGTCTATCATGGCTATTGCG AGTCCGCTCGCTTTTTCCGTAGATTCTAGCGGTGAGTATCCGACAGTCAGCGAAATTCCG GTCGGGGAGGTCCGGCTTTACCAGATTGCCGATGGTGTTTGGTCGCATATCGCAACGCAG TCGTTTGATGGCGCAGTCTACCCGTCCAATGGTCTCATTGTCCGTGATGGTGATGAGTTG CTTTTGATTGATACAGCGTGGGGTGCGAAAAACACAGCGGCACTTCTCGCGGAGATTGAG AAGCAAATTGGACTTCCTGTAACGCGTGCAGTCTCCACGCACTTTCATGACGACCGCGTC GGCGGCGTTGATGTCCTTCGGGCGGCTGGGGTGGCAACGTACGCATCACCGTCGACACGC CGGCTAGCCGAGGTAGAGGGGAACGAGATTCCCACGCACTCTCTAGAAGGACTCTCATCG AGCGGGGACGCAGTGCGCTTCGGTCCAGTAGAACTCTTCTATCCTGGTGCTGCGCATTCG ACCGACAACTTAGTTGTGTACGTCCCGTCTGCGAGTGTGCTCTATGGTGGTTGTGCGATT TATGAGTTGTCACGCACGTCTGCGGGGAACGTGGCCGATGCCGATCTGGCTGAATGGCCC |
| | ACCTCCATTGAGCGGATTCAACAACACTACCCGGAAGCACAGTTCGTCATTCCGGGGCAC GGCCTGCCGGGCGGTCTAGACTTGCTCAAGCACACAACGAATGTTGTAAAAGCGCACACA AATCGCTCAGTCGTTGAGTAG |
| GES | ATGCGCTTCATTCACGCACTATTACTGGCAGGGATCGCTCACTCTGCATATGCGTCGGAA AAATTAACCTTCAAGACCGATCTTGAGAAGCTAGAGCGCAAAAAGCAGCTCAGATCGGT GTTGCGATCGTCGATCCCCAAGGAGAGATCGTCGCGGCCACCGAATGGCGCAGCGTTTT GCAATGTGCTCAACGTTCAAGTTTCCGCTAGCCGCGCTGGTCTTTGAAAGAATTGACTCA GGCACCGAGCGGGGGGATCGAAAACTTTCATATGGGCCGGACATGATCGTCGAATGGTCT CCTGCCACGGAGCGGTTTCTAGCATCGGGACACATGACGGTTCTCGAGGCAGCGCAAGCT GCGGTGCAGCTTAGCGACAATGGGGCTACTAACCTCTTACTGAGAGAAATTGGCGGACCT GCTGCAATGACGCAGTATTTTCGTAAAATTGGCGACTCTGTGAGTCGGCTAGACCGGAAA GAGCCGGAGATGAGCGACAACACACCTGGCGACCTCAGAGATACAACTACGCCTATTGCT ATGGCACGTACTGTGGCTAAAGTCCTCTATGGCGGCGCACTGACGTCCACCTCGACCCAC ACCATTGAGAGGTGGCTGATCGGAAACCAAACGGGAGACGCGACACTACGAGCGGGTTTT CCTAAAGATTGGGTTGTTGGAGAGAAAACTGGTACCTGCGCCAACGGGGGCCGGAACGAC ATTGGTTTTTTTAAAGCCCAGGAGAGAGATTACGCTGTAGCGGTGTATACAACGGCCCCG AAACTATCGGCCGTAGAACGTGACGAATTAGTTGCCTCTGTCGGTCAAGTTATTACACAA CTCATCCTGAGCACGGACAAATAG |

### Example 2. Analysis of expression and size of each target protein

### (1) Target protein production and identification

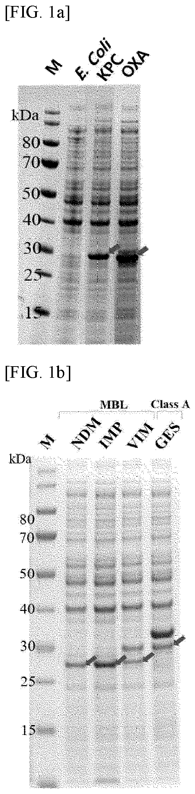

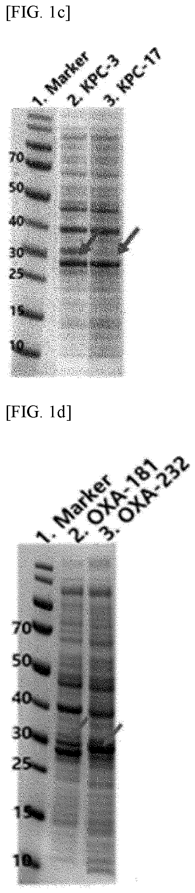

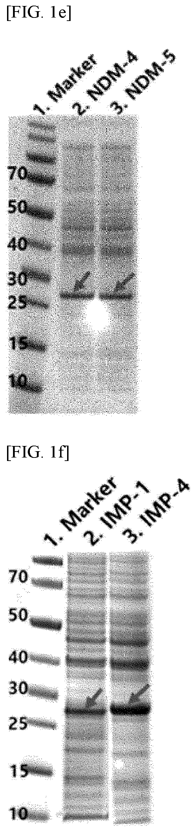

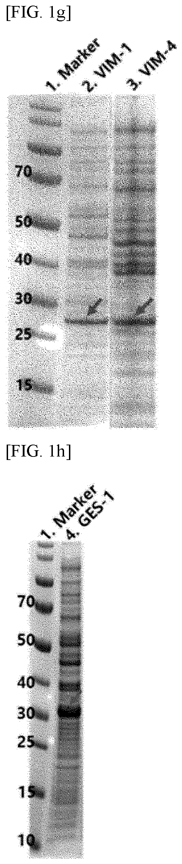

*E. coli* transformed with the plasmid containing each target gene was inoculated into Luria-bertani liquid medium containing 50 mg/L of ampicillin antibiotic, and cultured at 37°C for 16 hours or more. In order to analyze the expression and size of each target protein, the culture was centrifuged at 4,000 rpm for 15 minutes, and the cells were harvested by removing the supernatant. The harvested cells were added to SDS-sample buffer, heated at 95°C for 5 min, and centrifuged at 15,000 rpm for 5 min. Using the prepared samples, the expression and size of each target protein were analyzed through SDS-PAGE gel analysis (FIG. 1).

### (2) Confirmation of genotypes of KPC and OXA derived from clinical strain and confirmation of proteins

In order to confirm KPC and OXA derived from a clinical strain, a strain confirmed as positive for extended spectrum β-lactamase (ESBL) was collected. The collected strain was subjected to colony PCR using the primers used for KPC and OXA gene amplification. The resulting amplified PCR products were subjected to agar gel electrophoresis to confirm the sizes of the target genes. The PCR products whose sizes have been confirmed were subjected to DNA sequencing to confirm the exact genotypes of the KPC and OXA genes.

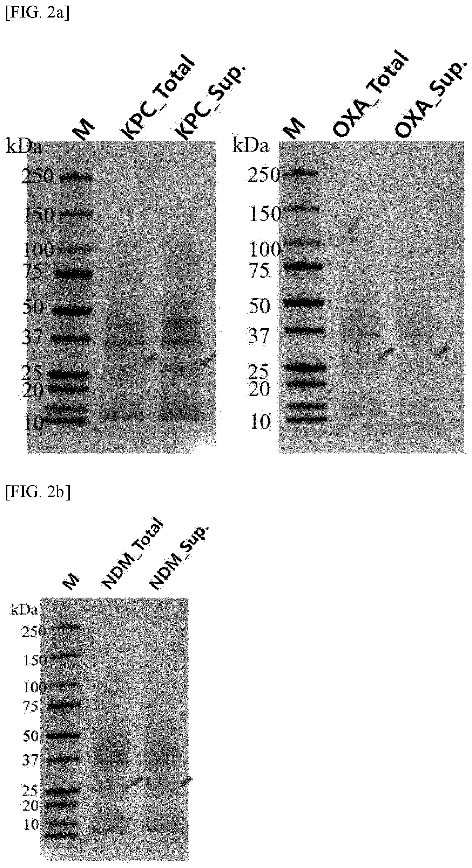

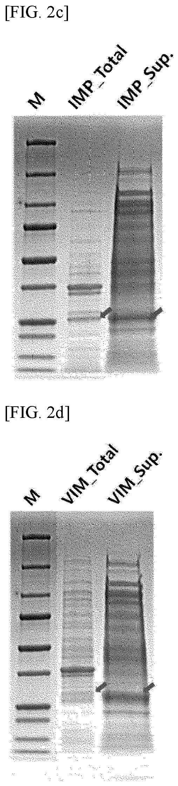

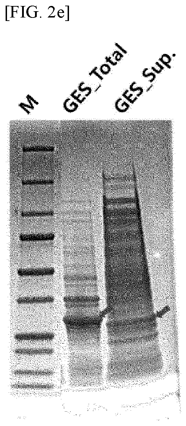

The strain whose genotypes have been confirmed was cultured in LB liquid medium, and SDS-PAGE gel analysis was performed to examine whether KPC and OXA proteins would be expressed. In addition, a recombinant strain containing the KPC and OXA genes derived from the clinical strain was constructed in the same manner as the existing recombinant strain comprising the vector containing the KPC and OXA genes, and the recombinant strain was also subjected to SDS-PAGE gel analysis in the same manner as the clinical strain to confirm the sizes of the actually expressed KPC and OXA proteins (FIG. 2).

Clinical strain-derived KPC and OXA proteins were each identified through the Q-TOF MS method. For KPC, it was confirmed that the sequence coverage for the identified peptides was 62.46% (183/293) in the full-length protein sequence and 67.28% (183/272) in the active form excluding the N-terminus. In addition, for OXA, it was confirmed that the sequence coverage for the identified peptides was 16.98% (45/265) in the full-length protein amino acid sequence and 18.52% (45/243) in the sequence of the active form.

### (3) MBL protein production and identification

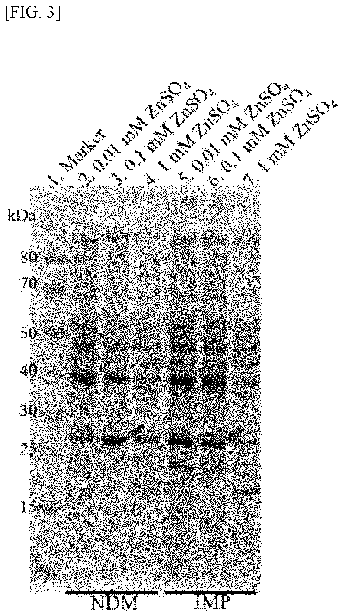

*E. coli* transformed with the plasmid containing the target MBL gene was inoculated into Luria-bertani liquid medium containing 50 mg/L of ampicillin antibiotic and zinc sulfate (0.01 mM to 1 mM ZnSO₄), and cultured at 37°C for 16 hours or more. In order to analyze the expression and size of the target protein, the culture was centrifuged at 4,000 rpm for 15 minutes, and the cells were harvested by removing the supernatant. The harvested cells were added to SDS-sample buffer, heated at 95°C for 5 min, and centrifuged at 15,000 rpm for 5 min. Using the prepared sample, the expression and size of the target protein were analyzed through SDS-PAGE gel analysis (FIG. 3).

### Example 3. Sample pretreatment method and identification of target protein from crude enzyme solution

### (1) Sample pretreatment with nonionic surfactant

For sample pretreatment, the culture was centrifuged at 4,000 rpm for 15 minutes, and the cells were harvested by removing the supernatant. To obtain a crude extract, the cells were treated with a buffer solution (0.25 mM Tris-HCl, 2% OG) and incubated at room temperature for 10 minutes. The prepared crude extract was separated into a supernatant (hereinafter referred to as crude enzyme solution) and a precipitate by centrifugation at 15,000 rpm at 4°C for 10 minutes. From the crude enzyme solution, the expression and size of the target protein were analyzed by SDS-PAGE analysis (FIG. 4).

### (2) Sample pretreatment with ionic surfactant

Sample pretreatment was performed by the following steps: 1) 100 ml of the expressed cell culture was centrifuged and to recover the cells, and 2) the supernatant was removed, and then a buffer solution (0.25 mM Tris-HCl, pH 8.0 and 2% DOC) containing 2% sodium deoxycholate (DOC) as a nonionic surfactant was added to the cells. 3) The suspension was incubated at room temperature for 10 minutes, and 4) centrifuged at 15,000 rpm at 4°C for 10 minutes to obtain a crude enzyme solution.

From the crude extract and the crude enzyme solution, obtained by treatment with the ionic surfactant (DOC), the expression and size of the protein were analyzed by SDS-PAGE gel analysis (FIG. 4b).

### (3) Sample pretreatment with volatile buffer

The sample pretreatment method is as follows: 1) 50 mM ammonium bicarbonate (hereinafter referred to as ABC) solution was added to the harvested cells, 2) the cells were resuspended by pipetting, and then 3) the suspension was centrifuged at 12,000 rpm and 4°C for 10 minutes to recover the supernatant. From the crude extract and crude enzyme solution obtained by treatment, the expression and size of the protein were analyzed by SDS-PAGE gel analysis (FIGS. 4c and 4e).

### (4) Sample pretreatment by sonication

The cells in the suspensions treated by the methods (1) to (3) above were disrupted by sonication using a Sonic bath (JAC 2010, Hansol Tech, Korea) at 40 Hz and 200 W for 5 to 10 minutes, and centrifuged at 15,000 rpm for 10 min, and the supernatant was recovered.

### Example 4. Separation/purification of target protein

Ion exchange chromatography was used to separate/purify each target protein. For anion exchange chromatography, a column containing Q-resin was used, and for cation exchange chromatography, a column containing SP-resin was used.

### (1) Anion exchange resin chromatography

The crude enzyme solution was loaded into a column containing Q-resin, and then the eluted solution was collected. The column was washed with 1 ml of 20 mM Tris-HCl (pH 8.0) buffer, and elution solutions containing 100 mM NaCl, 200 mM NaCl, 300 mM NaCl, 400 mM NaCl and 500 mM NaCl 1M, respectively, in buffer, were sequentially loaded into the column in an amount of 250 µl for each elution solution, and the eluate was collected for each zone.

### (2) Cation exchange resin chromatography

Using a column containing SP-resin, each target protein was separated/purified in the same manner as (1) anion exchange resin chromatography.

Finally, six desired target proteins were separated/purified through the above ion exchange chromatography method (FIG. 4). Finally, each high-purity protein was separated/purified from the cell lysate using the same method as described above.

### Example 5. Analysis of expression and size of each target protein

The proteins whose expression and size were confirmed on the SDS-PAGE gel were identified using the in-gel digestion method and the nano-LC-MS/MS method, thereby confirming the type of antibiotic resistance protein actually expressed in the strain (FIG. 7).

### (1) In-gel digestion

Only the band portion corresponding to the size of each target protein on the SDS-PAGE gel was obtained, and the stained gel was destained. The destained gel was subjected to a reduction/alkylation process, and then the protein was selectively digested using a trypsin enzyme. The digested peptides were recovered and desalted using DK-Tip (C18 Tip).

### (2) nano-LC-MS/MS

In order to confirm the sequence and coverage of the active protein expressed in the strain, nano liquid chromatography and high-resolution mass spectrometry were performed (Q-Exactive HF-X mass spectrometry system). The desalted peptide sample was dissolved with 0.1% formic acid solution and then loaded into a column. The peptide sample was separated using a C18 column (75 µm × 70 cm) and nanoflow liquid chromatography. Examples of the gradient conditions for sample loading and separation used in this case are as follows:
- buffer A: 0.1% formic acid in water/ buffer B: 0.1% formic acid in acetonitrile
- sample loading: from 0 to 5 min, 5% (B), 5 µL/min flow rate
- concentration gradient for separation:
   from 5 to 7 min, from 5% to 10% (B), 300 nL/min flow rate
   from 7 to 38 min, from 10% to 40% (B), 300 nL/min flow rate
   from 38 to 38.5 min, from 40% to 80% (B), 300 nL/min flow rate
   from 38.5 to 39.5 min, 80% (B), 300 nL/min flow rate
   from 39.5 to 40 min, from 80% to 5% (B), 300 nL/min flow rate
   from 40 to 60 min, 5% (B), 300 nL/min flow rate

Examples of the parameters of the mass spectrometer used in this case are as follows:
- resolution: Full MS 60,000, MS2 30,000
- Full MS: 350 to 2,000 m/z, 100 msec
- MS2: 50 msec, NCE 28, 1, ionized materials with a charge state of >6 were excluded from MS2 analysis

'Proteome Discoverer (v2.4)' software (Thermo scientific) was used to identify peptides and proteins based on bottom-up data, and protein/peptide identification was based on a FDR (false discovery rate) of 1%. Among proteins from *E. coli,* KPC, OXA, MBL proteins or GES protein was identified, 200 peptides (Table 2) for KPC protein, 177 peptides (Table 3) for OXA, 109 peptides (Table 4) for NDM protein, 146 peptides (Table 5) for IMP (Table 5), 159 peptides (Table 6) for VIM protein, and 111 peptides (Table 7) for GES were identified.

**[Table 2] Information on position and sequence of each peptide identified as KPC protein**

| **Start** | **Stop** | **Sequence** | **Oxidatio n site** | **Z** | **SEQUES T XCorr** | **SEQUE ST deltaCn** | **Observe d m/z** | **Actual mass (Da)** |
|---|---|---|---|---|---|---|---|---|
| 22 | 35 | (S)ATALTNLVAEPFAK(L) | | 2 | 4.48 | 0.67 | 723.40 | 1,444.79 |
| 23 | 35 | (A)TALTNLVAEPFAK(L) | | 2 | 4.56 | 0.67 | 687.88 | 1,373.75 |
| 24 | 35 | (T)ALTNLVAEPFAK(L) | | 2 | 3.17 | 0.53 | 637.36 | 1,272.71 |
| 25 | 35 | (A)LTNLVAEPFAK(L) | | 2 | 3.38 | 0.56 | 601.84 | 1,201.67 |
| 26 | 36 | (L)TNLVAEPFAKL(E) | | 2 | 2.05 | 0.27 | 601.84 | 1,201.67 |
| 26 | 60 | (L)TNLVAEPFAKLEQDFGGSIGVY AmDTGSGATVSYR(A) | M(49) | 3 | 2.99 | 0.50 | 1,223.26 | 3,666.76 |
| 27 | 35 | (T)NLVAEPFAK(L) | | 2 | 2.25 | 0.33 | 494.78 | 987.54 |
| 29 | 35 | (L)VAEPFAK(L) | | 1 | 2.65 | 0.43 | 761.41 | 760.41 |
| 34 | 47 | (F)AKLEQDFGGSIGVY(A) | | 2 | 3.15 | 0.52 | 742.38 | 1,482.74 |
| 34 | 60 | (F)AKLEQDFGGSIGVYAmDTGSGA TVSYR(A) | M(49) | 3 | 5.39 | 0.72 | 932.78 | 2,795.31 |
| 34 | 60 | (F)AKLEQDFGGSIGVYAMDTGSGA TVSYR(A) | | 3 | 3.97 | 0.62 | 927.45 | 2,779.32 |
| 36 | 60 | (K)LEQDFGGSIGVYAmDTGSGATV SYR(A) | M(49) | 2 | 6.81 | 0.78 | 1,299.10 | 2,596.18 |
| 36 | 60 | (K)LEQDFGGSIGVYAMDTGSGATV SYR(A) | | 2 | 6.83 | 0.78 | 1,291.10 | 2,580.18 |
| 42 | 60 | (G)GSIGVYAmDTGSGATVSYR(A) | M(49) | 2 | 2.01 | 0.25 | 954.44 | 1,906.87 |
| 45 | 60 | (I)GVYAMDTGSGATVSYR(A) | | 2 | 2.35 | 0.36 | 817.88 | 1,633.74 |
| 48 | 59 | (Y)AmDTGSGATVSY(R) | M(49) | 2 | 2.84 | 0.47 | 588.25 | 1,174.48 |
| 48 | 59 | (Y)AMDTGSGATVSY(R) | | 2 | 2.67 | 0.44 | 580.25 | 1,158.49 |
| 48 | 60 | (Y)AmDTGSGATVSYR(A) | M(49) | 2 | 3.30 | 0.55 | 666.30 | 1,330.58 |
| 48 | 60 | (Y)AMDTGSGATVSYR(A) | | 2 | 3.92 | 0.62 | 658.30 | 1,314.59 |
| 50 | 60 | (M)DTGSGATVSYR(A) | | 2 | 2.25 | 0.33 | 557.27 | 1,112.52 |
| 60 | 67 | (Y)RAEERFPL(C) | | 2 | 1.76 | 0.15 | 509.28 | 1,016.54 |
| 60 | 71 | (Y)RAEERFPLcSSF(K) | | 2 | 2.80 | 0.46 | 749.86 | 1,497.71 |
| 61 | 67 | (R)AEERFPL(C) | | 2 | 1.98 | 0.24 | 431.23 | 860.44 |
| 61 | 72 | (R)AEERFPLcSSFK(G) | | 2 | 4.26 | 0.65 | 735.86 | 1,469.70 |
| 62 | 72 | (A)EERFPLcSSFK(G) | | 2 | 3.39 | 0.56 | 700.34 | 1,398.67 |
| 63 | 72 | (E)ERFPLcSSFK(G) | | 2 | 3.86 | 0.61 | 635.82 | 1,269.62 |
| 65 | 72 | (R)FPLcSSFK(G) | | 2 | 2.35 | 0.36 | 493.25 | 984.48 |
| 68 | 74 | (L)cSSFKGF(L) | | 2 | 2.16 | 0.31 | 416.69 | 831.36 |
| 72 | 80 | (F)KGFLAAAVL(A) | | 2 | 2.57 | 0.42 | 445.28 | 888.54 |
| 72 | 82 | (F)KGFLAAAVLAR(S) | | 2 | 2.01 | 0.25 | 558.85 | 1,115.69 |
| 73 | 82 | (K)GFLAAAVLAR(S) | | 2 | 3.21 | 0.53 | 494.80 | 987.59 |
| 74 | 82 | (G)FLAAAVLAR(S) | | 2 | 1.97 | 0.24 | 466.29 | 930.56 |
| 75 | 82 | (F)LAAAVLAR(S) | | 2 | 2.96 | 0.49 | 392.76 | 783.50 |
| 76 | 82 | (L)AAAVLAR(S) | | 1 | 2.24 | 0.33 | 671.42 | 670.41 |
| 81 | 96 | (L)ARSQQQAGLLDTPIRY(G) | | 2 | 3.60 | 0.58 | 908.99 | 1,815.96 |
| 83 | 95 | (R)SQQQAGLLDTPIR(Y) | | 2 | 4.74 | 0.68 | 713.88 | 1,425.75 |
| 83 | 98 | (R)SQQQAGLLDTPIRYGK(N) | | 2 | 5.57 | 0.73 | 887.98 | 1,773.94 |
| 84 | 95 | (S)QQQAGLLDTPIR(Y) | | 2 | 2.45 | 0.39 | 670.37 | 1,338.73 |
| 85 | 95 | (Q)QQAGLLDTPIR(Y) | | 2 | 3.39 | 0.56 | 606.34 | 1,210.67 |
| 87 | 95 | (Q)AGLLDTPIR(Y) | | 2 | 2.37 | 0.37 | 478.28 | 954.55 |
| 90 | 96 | (L)LDTPIRY(G) | | 2 | 1.96 | 0.23 | 439.24 | 876.47 |
| 96 | 110 | (R)YGKNALVPWSPISEK(Y) | | 2 | 1.87 | 0.20 | 844.95 | 1,687.89 |
| 97 | 104 | (Y)GKNALVPW(S) | | 2 | 1.89 | 0.21 | 442.75 | 883.50 |
| 97 | 111 | (Y)GKNALVPWSPISEKY(L) | | 2 | 4.27 | 0.65 | 844.96 | 1,687.90 |
| 99 | 110 | (K)NALVPWSPISEK(Y) | | 2 | 3.22 | 0.53 | 670.86 | 1,339.71 |
| 100 | 110 | (N)ALVPWSPISEK(Y) | | 2 | 2.11 | 0.29 | 613.84 | 1,225.67 |
| 102 | 110 | (L)VPWSPISEK(Y) | | 1 | 2.69 | 0.44 | 1,042.55 | 1,041.54 |
| 103 | 110 | (V)PWSPISEK(Y) | | 2 | 2.19 | 0.31 | 472.25 | 942.48 |
| 105 | 111 | (W)SPISEKY(L) | | 2 | 1.90 | 0.21 | 412.22 | 822.42 |
| 105 | 112 | (W)SPISEKYL(T) | | 2 | 1.87 | 0.20 | 468.76 | 935.50 |
| 105 | 121 | (W)SPISEKYLTTGmTVAEL(S) | M(116) | 2 | 3.60 | 0.58 | 928.47 | 1,854.93 |
| 111 | 139 | (K)YLTTGmTVAELSAAAVQYSDN AAANLLLK(E) | M(116) | 2 | 8.51 | 0.82 | 1,508.27 | 3,014.53 |
| 111 | 139 | (K)YLTTGMTVAELSAAAVQYSDN AAANLLLK(E) | | 2 | 8.88 | 0.83 | 1,500.27 | 2,998.53 |
| 112 | 121 | (Y)LTTGmTVAEL(S) | M(116) | 2 | 1.90 | 0.21 | 526.27 | 1,050.53 |
| 113 | 139 | (L)TTGMTVAELSAAAVQYSDNAA ANLLLK(E) | | 2 | 3.10 | 0.52 | 1,362.20 | 2,722.39 |
| 117 | 139 | (M)TVAELSAAAVQYSDNAAANLL LK(E) | | 2 | 5.53 | 0.73 | 1,167.12 | 2,332.23 |
| 122 | 128 | (L) SAAAVQY(S) | | 1 | 1.62 | 0.07 | 709.35 | 708.35 |
| 122 | 139 | (L) SAAAVQYSDNAAANLLLK(E) | | 2 | 4.23 | 0.65 | 910.48 | 1,818.94 |
| 123 | 139 | (S)AAAVQYSDNAAANLLLK(E) | | 2 | 3.20 | 0.53 | 866.97 | 1,731.92 |
| 125 | 139 | (A)AVQYSDNAAANLLLK(E) | | 2 | 3.41 | 0.56 | 795.93 | 1,589.84 |
| 128 | 139 | (Q)YSDNAAANLLLK(E) | | 2 | 3.76 | 0.60 | 646.85 | 1,291.68 |
| 129 | 137 | (Y)SDNAAANLL(L) | | 1 | 2.19 | 0.32 | 888.45 | 887.44 |
| 129 | 139 | (Y)SDNAAANLLLK(E) | | 2 | 2.65 | 0.43 | 565.31 | 1,128.61 |
| 138 | 147 | (L)LKELGGPAGL(T) | | 2 | 3.57 | 0.58 | 477.79 | 953.56 |
| 139 | 150 | (L)KELGGPAGLTAF(M) | | 2 | 2.89 | 0.48 | 580.82 | 1,159.63 |
| 140 | 152 | (K)ELGGPAGLTAFmR(S) | M(151) | 2 | 3.56 | 0.58 | 668.34 | 1,334.67 |
| 140 | 152 | (K)ELGGPAGLTAFMR(S) | | 2 | 3.94 | 0.62 | 660.34 | 1,318.67 |
| 142 | 152 | (L)GGPAGLTAFmR(S) | M(151) | 2 | 2.11 | 0.29 | 547.28 | 1,092.54 |
| 143 | 152 | (G)GPAGLTAFmR(S) | M(151) | 2 | 2.63 | 0.43 | 518.77 | 1,035.52 |
| 144 | 152 | (G)PAGLTAFmR(S) | M(151) | 2 | 2.42 | 0.38 | 490.25 | 978.49 |
| 148 | 159 | (L)TAFmRSIGDTTF(R) | M(151) | 2 | 3.43 | 0.56 | 681.82 | 1,361.63 |
| 148 | 159 | (L)TAFMRSIGDTTF(R) | | 2 | 3.89 | 0.61 | 673.83 | 1,345.64 |
| 151 | 159 | (F)mRSIGDTTF(R) | M(151) | 2 | 2.57 | 0.42 | 522.25 | 1,042.48 |
| 151 | 159 | (F)MRSIGDTTF(R) | | 2 | 2.52 | 0.40 | 514.25 | 1,026.49 |
| 153 | 160 | (R)SIGDTTFR(L) | | 2 | 2.15 | 0.30 | 448.73 | 895.44 |
| 153 | 163 | (R)SIGDTTFRLDR(W) | | 2 | 2.55 | 0.41 | 640.83 | 1,279.65 |
| 153 | 177 | (R)SIGDTTFRLDRWELELNSAIPGD AR(D) | | 3 | 1.99 | 0.25 | 944.82 | 2,831.43 |
| 154 | 160 | (S)IGDTTFR(L) | | 2 | 2.08 | 0.28 | 405.21 | 808.41 |
| 154 | 163 | (S)IGDTTFRLDR(W) | | 2 | 1.88 | 0.20 | 597.32 | 1,192.62 |
| 155 | 163 | (I)GDTTFRLDR(W) | | 2 | 1.85 | 0.19 | 540.78 | 1,079.54 |
| 156 | 163 | (G)DTTFRLDR(W) | | 2 | 1.92 | 0.22 | 512.27 | 1,022.52 |
| 161 | 177 | (R)LDRWELELNSAIPGDAR(D) | | 2 | 3.95 | 0.62 | 978.00 | 1,953.99 |
| 161 | 183 | (R)LDRWELELNSAIPGDARDTSSPR (A) | | 3 | 2.59 | 0.42 | 866.77 | 2,597.29 |
| 164 | 177 | (R)WELELNSAIPGDAR(D) | | 2 | 4.22 | 0.64 | 785.90 | 1,569.78 |
| 164 | 183 | (R)WELELNSAIPGDARDTSSPR(A) | | 3 | 3.34 | 0.55 | 738.70 | 2,213.08 |
| 165 | 177 | (W)ELELNSAIPGDAR(D) | | 2 | 3.42 | 0.56 | 692.86 | 1,383.70 |
| 184 | 191 | (R)A VTESLQK(L) | | 2 | 2.27 | 0.34 | 438.24 | 874.47 |
| 184 | 203 | (R)AVTESLQKLTLGSALAAPQR(Q) | | 2 | 3.16 | 0.53 | 1,027.58 | 2,053.15 |
| 185 | 191 | (A)VTESLQK(L) | | 2 | 1.97 | 0.24 | 402.73 | 803.44 |
| 192 | 203 | (K)L TLGSALAAPQR(Q) | | 2 | 4.07 | 0.63 | 599.35 | 1,196.69 |
| 192 | 204 | (K)L TLGSALAAPQRQ(Q) | | 2 | 1.85 | 0.19 | 663.38 | 1,324.75 |
| 193 | 203 | (L)TLGSALAAPQR(Q) | | 2 | 2.24 | 0.33 | 542.81 | 1,083.61 |
| 193 | 206 | (L)TLGSALAAPQRQQF(V) | | 2 | 4.04 | 0.63 | 744.40 | 1,486.79 |
| 194 | 203 | (T)LGSALAAPQR(Q) | | 2 | 3.44 | 0.56 | 492.28 | 982.56 |
| 195 | 206 | (L)GSALAAPQRQQF(V) | | 2 | 2.10 | 0.29 | 637.34 | 1,272.66 |
| 199 | 209 | (L)AAPQRQQFVDW(L) | | 2 | 3.25 | 0.54 | 673.34 | 1,344.66 |
| 199 | 210 | (L)AAPQRQQFVDWL(K) | | 2 | 1.82 | 0.18 | 729.88 | 1,457.74 |
| 204 | 211 | (R)QQFVDWLK(G) | | 2 | 2.89 | 0.48 | 532.28 | 1,062.55 |
| 204 | 219 | (R)QQFVDWLKGNTTGNHR(I) | | 2 | 2.91 | 0.48 | 950.98 | 1,899.94 |
| 210 | 228 | (W)LKGNTTGNHRIRAAVPADW(A) | | 3 | 1.93 | 0.22 | 693.04 | 2,076.11 |
| 212 | 219 | (K)GNTTGNHR(I) | | 2 | 2.39 | 0.37 | 428.70 | 855.39 |
| 222 | 233 | (R)AAVPADWAVGDK(T) | | 2 | 2.66 | 0.44 | 600.31 | 1,198.60 |
| 222 | 235 | (R)AAVPADWAVGDKTG(T) | | 2 | 3.29 | 0.54 | 679.34 | 1,356.67 |
| 222 | 236 | (R)AAVPADWAVGDKTGT(C) | | 2 | 3.82 | 0.61 | 729.86 | 1,457.71 |
| 222 | 254 | (R)AAVPADWAVGDKTGTcGVYGT ANDYAVVWPTGR(A) | | 3 | 3.57 | 0.58 | 1,142.54 | 3,424.60 |
| 223 | 233 | (A)AVPADWAVGDK(T) | | 2 | 2.43 | 0.38 | 564.79 | 1,127.56 |
| 224 | 233 | (A)VPADWAVGDK(T) | | 2 | 2.89 | 0.48 | 529.27 | 1,056.52 |
| 225 | 233 | (V)PADWAVGDK(T) | | 2 | 3.15 | 0.52 | 479.73 | 957.46 |
| 226 | 233 | (P)ADWAVGDK(T) | | 1 | 2.59 | 0.42 | 861.41 | 860.40 |
| 229 | 240 | (W)AVGDKTGTcGVY(G) | | 2 | 3.20 | 0.53 | 614.29 | 1,226.56 |
| 229 | 246 | (W)AVGDKTGTcGVYGTANDY(A) | | 2 | 3.02 | 0.50 | 924.91 | 1,847.81 |
| 234 | 254 | (K)TGTcGVYGTANDYAVVWPTGR (A) | | 2 | 3.04 | 0.51 | 1,123.02 | 2,244.02 |
| 238 | 254 | (C)GVYGTANDYAVVWPTGR(A) | | 2 | 4.23 | 0.65 | 913.45 | 1,824.88 |
| 239 | 254 | (G)VYGTANDYAVVWPTGR(A) | | 2 | 4.11 | 0.64 | 884.94 | 1,767.86 |
| 240 | 254 | (V)YGTANDY A VVWPTGR(A) | | 2 | 3.37 | 0.55 | 835.40 | 1,668.79 |
| 241 | 254 | (Y)GTANDYAVVWPTGR(A) | | 2 | 1.98 | 0.24 | 753.87 | 1,505.73 |
| 245 | 254 | (N)DYAVVWPTGR(A) | | 2 | 2.69 | 0.44 | 582.29 | 1,162.58 |
| 247 | 259 | (Y)AVVWPTGRAPIVL(A) | | 2 | 2.21 | 0.32 | 689.92 | 1,377.82 |
| 251 | 262 | (W)PTGRAPIVLAVY(T) | | 2 | 2.40 | 0.38 | 628.87 | 1,255.73 |
| 255 | 264 | (R)APIVLAVYTR(A) | | 2 | 3.49 | 0.57 | 551.83 | 1,101.65 |
| 255 | 271 | (R)APIVLAVYTRAPNKDDK(H) | | 3 | 1.75 | 0.14 | 624.35 | 1,870.03 |
| 256 | 264 | (A)PIVLAVYTR(A) | | 2 | 2.99 | 0.50 | 516.32 | 1,030.62 |
| 257 | 264 | (P)IVLAVYTR(A) | | 2 | 1.99 | 0.25 | 467.79 | 933.56 |
| 260 | 283 | (L)AVYTRAPNKDDKHSEAVIAAA ARL(A) | | 4 | 3.51 | 0.57 | 642.60 | 2,566.37 |
| 263 | 283 | (Y)TRAPNKDDKHSEAVIAAAARL( A) | | 3 | 5.25 | 0.71 | 745.41 | 2,233.20 |
| 263 | 285 | (Y)TRAPNKDDKHSEAVIAAAARLA L(E) | | 3 | 3.67 | 0.59 | 806.78 | 2,417.33 |
| 265 | 282 | (R)APNKDDKHSEAVIAAAAR(L) | | 2 | 5.92 | 0.75 | 932.49 | 1,862.96 |
| 266 | 282 | (A)PNKDDKHSEAVIAAAAR(L) | | 3 | 5.88 | 0.74 | 598.31 | 1,791.92 |
| 267 | 282 | (P)NKDDKHSEAVIAAAAR(L) | | 2 | 4.96 | 0.70 | 848.44 | 1,694.87 |
| 268 | 282 | (N)KDDKHSEAVIAAAAR(L) | | 2 | 5.04 | 0.70 | 791.42 | 1,580.82 |
| 269 | 282 | (K)DDKHSEAVIAAAAR(L) | | 2 | 3.34 | 0.55 | 727.37 | 1,452.73 |
| 270 | 282 | (D)DKHSEAVIAAAAR(L) | | 3 | 2.12 | 0.29 | 446.91 | 1,337.71 |
| 271 | 282 | (D)KHSEAVIAAAAR(L) | | 2 | 3.35 | 0.55 | 612.35 | 1,222.68 |
| 272 | 282 | (K)HSEAVIAAAAR(L) | | 2 | 3.92 | 0.62 | 548.30 | 1,094.58 |
| 273 | 282 | (H)SEAVIAAAAR(L) | | 2 | 2.34 | 0.36 | 479.77 | 957.52 |
| 283 | 291 | (R)LALEGLGVN(G) | | 1 | 2.36 | 0.36 | 885.50 | 884.49 |
| 283 | 293 | (R)LALEGLGVNGQ(-) | | 2 | 3.66 | 0.59 | 535.80 | 1,069.58 |
| 284 | 293 | (L)ALEGLGVNGQ(-) | | 2 | 2.09 | 0.28 | 479.25 | 956.49 |

**[Table 3] Information on position and sequence of each peptide identified as OXA protein**

| **Start** | **Stop** | **Sequence** | **Oxidatio n site** | **Z** | **SEQUE ST XCorr** | **SEQUE ST deltaCn** | **Observ ed m/z** | **Actual mass (Da)** |
|---|---|---|---|---|---|---|---|---|
| 23 | 29 | (A)KEWQENK(S) | | 2 | 2.01 | 0.25 | 481.24 | 960.47 |
| 23 | 39 | (A)KEWQENKSWNAHFTEHK(S) | | 4 | 5.28 | 0.72 | 550.52 | 2198.03 |
| 28 | 39 | (E)NKSWNAHFTEHK(S) | | 2 | 3.06 | 0.51 | 749.87 | 1497.72 |
| 30 | 37 | (K)SWNAHFTE(H) | | 2 | 1.78 | 0.16 | 496.22 | 990.43 |
| 30 | 38 | (K)SWNAHFTEH(K) | | 2 | 3.14 | 0.52 | 564.75 | 1127.48 |
| 30 | 39 | (K)SWNAHFTEHK(S) | | 2 | 3.71 | 0.60 | 628.80 | 1255.58 |
| 31 | 39 | (S) WNAHFTEHK(S) | | 2 | 3.47 | 0.57 | 585.28 | 1168.54 |
| 32 | 39 | (W)NAHFTEHK(S) | | 2 | 3.09 | 0.51 | 492.24 | 982.46 |
| 32 | 39 | (W)NAHFTEHK(S) | | 3 | 2.80 | 0.46 | 328.50 | 982.47 |
| 33 | 39 | (N)AHFTEHK(S) | | 2 | 2.06 | 0.27 | 435.22 | 868.42 |
| 38 | 51 | (E)HKSQGVVVLWNENK(Q) | | 2 | 5.32 | 0.72 | 819.45 | 1636.88 |
| 40 | 49 | (K)SQGVVVL WNE(N) | | 2 | 2.56 | 0.41 | 565.80 | 1129.58 |
| 40 | 51 | (K) SQGVVVLWNENK(Q) | | 3 | 3.90 | 0.62 | 458.25 | 1371.71 |
| 40 | 60 | (K)SQGVVVL WNENKQQGFTNNLK(R) | | 2 | 4.90 | 0.69 | 1202.14 | 2402.26 |
| 40 | 61 | (K)SQGVVVL WNENKQQGFTNNLKR(A) | | 3 | 6.25 | 0.76 | 853.78 | 2558.33 |
| 42 | 51 | (Q)GVVVLWNENK(Q) | | 2 | 3.13 | 0.52 | 579.32 | 1156.63 |
| 49 | 60 | (N)ENKQQGFTNNLK(R) | | 2 | 2.79 | 0.46 | 710.87 | 1419.72 |
| 49 | 61 | (N)ENKQQGFTNNLKR(A) | | 3 | 4.16 | 0.64 | 526.28 | 1575.82 |
| 50 | 60 | (E)NKQQGFTNNLK(R) | | 2 | 3.50 | 0.57 | 646.34 | 1290.67 |
| 50 | 61 | (E)NKQQGFTNNLKR(A) | | 3 | 5.40 | 0.72 | 483.27 | 1446.77 |
| 51 | 60 | (N)KQQGFTNNLK(R) | | 2 | 3.73 | 0.60 | 589.32 | 1176.63 |
| 51 | 61 | (N)KQQGFTNNLKR(A) | | 2 | 2.07 | 0.28 | 667.37 | 1332.73 |
| 52 | 60 | (K)QQGFTNNLK(R) | | 2 | 3.28 | 0.54 | 525.27 | 1048.53 |
| 52 | 61 | (K)QQGFTNNLKR(A) | | 3 | 3.92 | 0.62 | 402.55 | 1204.64 |
| 53 | 61 | (Q)QGFTNNLKR(A) | | 2 | 2.93 | 0.49 | 539.29 | 1076.57 |
| 54 | 60 | (Q)GFTNNLK(R) | | 1 | 2.16 | 0.31 | 793.42 | 792.41 |
| 54 | 61 | (Q)GFTNNLKR(A) | | 2 | 2.44 | 0.38 | 475.26 | 948.51 |
| 55 | 61 | (G)FTNNLKR(A) | | 2 | 2.40 | 0.37 | 446.75 | 891.50 |
| 61 | 72 | (K)RANQAFLPASTF(K) | | 2 | 2.99 | 0.50 | 661.85 | 1321.68 |
| 61 | 73 | (K)RANQAFLPASTFK(I) | | 2 | 4.14 | 0.64 | 725.90 | 1449.78 |
| 62 | 72 | (R)ANQAFLP ASTF(K) | | 2 | 2.30 | 0.35 | 583.80 | 1165.58 |
| 62 | 73 | (R)ANQAFLP ASTFK(I) | | 2 | 3.95 | 0.62 | 647.85 | 1293.68 |
| 62 | 76 | (R)ANQAFLP ASTFKIPN(S) | | 2 | 2.10 | 0.29 | 809.94 | 1617.86 |
| 63 | 73 | (A)NQAFLPASTFK(I) | | 2 | 3.07 | 0.51 | 612.33 | 1222.64 |
| 64 | 73 | (N)QAFLPASTFK(I) | | 2 | 3.31 | 0.55 | 555.31 | 1108.60 |
| 65 | 73 | (Q)AFLPASTFK(I) | | 2 | 1.95 | 0.23 | 491.28 | 980.54 |
| 66 | 73 | (A)FLPASTFK(I) | | 1 | 2.53 | 0.41 | 910.51 | 909.50 |
| 67 | 73 | (F)LPASTFK(I) | | 1 | 3.04 | 0.51 | 763.43 | 762.43 |
| 74 | 87 | (K)IPNSLIALDLGVVK(D) | | 2 | 3.39 | 0.56 | 726.45 | 1450.88 |
| 74 | 94 | (K)IPNSLIALDLGVVKDEHQVFK(W) | | 3 | 8.46 | 0.82 | 779.11 | 2334.30 |
| 74 | 100 | (K)IPNSLIALDLGVVKDEHQVFKWDGQ TR(D) | | 5 | 2.45 | 0.39 | 616.53 | 3077.63 |
| 75 | 94 | (I)PNSLIALDLGVVKDEHQVFK(W) | | 4 | 5.10 | 0.71 | 556.31 | 2221.20 |
| 77 | 94 | (N)SLIALDLGVVKDEHQVFK(W) | | 2 | 4.75 | 0.68 | 1006.07 | 2010.12 |
| 80 | 94 | (I)ALDLGVVKDEHQVFK(W) | | 2 | 2.13 | 0.30 | 849.46 | 1696.91 |
| 81 | 94 | (A)LDLGVVKDEHQVFK(W) | | 2 | 1.82 | 0.18 | 813.95 | 1625.88 |
| 82 | 94 | (L)DLGVVKDEHQVFK(W) | | 2 | 3.04 | 0.51 | 757.40 | 1512.79 |
| 83 | 94 | (D)LGVVKDEHQVFK(W) | | 2 | 3.16 | 0.53 | 699.89 | 1397.77 |
| 83 | 100 | (D)LGVVKDEHQVFKWDGQTR(D) | | 3 | 3.36 | 0.55 | 714.71 | 2141.11 |
| 84 | 94 | (L)GVVKDEHQVFK(W) | | 2 | 3.26 | 0.54 | 643.35 | 1284.68 |
| 88 | 95 | (K)DEHQVFKW(D) | | 2 | 2.60 | 0.42 | 544.76 | 1087.51 |
| 88 | 100 | (K)DEHQVFKWDGQTR(D) | | 2 | 3.91 | 0.62 | 823.39 | 1644.77 |
| 89 | 100 | (D)EHQVFKWDGQTR(D) | | 3 | 1.82 | 0.18 | 510.92 | 1529.74 |
| 90 | 100 | (E)HQVFKWDGQTR(D) | | 3 | 3.49 | 0.57 | 467.91 | 1400.70 |
| 92 | 100 | (Q)VFKWDGQTR(D) | | 2 | 1.92 | 0.22 | 568.80 | 1135.58 |
| 96 | 107 | (W)DGQTRDIATWNR(D) | | 3 | 3.29 | 0.54 | 478.24 | 1431.69 |
| 101 | 107 | (R)DIATWNR(D) | | 2 | 2.13 | 0.30 | 438.22 | 874.43 |
| 101 | 110 | (R)DIATWNRDHN(L) | | 2 | 1.94 | 0.23 | 621.29 | 1240.56 |
| 101 | 116 | (R)DIATWNRDHNLITAmK(Y) | M(115) | 3 | 5.46 | 0.73 | 638.99 | 1913.94 |
| 101 | 116 | (R)DIATWNRDHNLITAMK(Y) | | 3 | 5.23 | 0.71 | 633.66 | 1897.95 |
| 103 | 116 | (I) A TWNRDHNLIT AmK(Y) | M(115) | 3 | 6.00 | 0.75 | 562.95 | 1685.83 |
| 103 | 116 | (I)ATWNRDHNLITAMK(Y) | | 3 | 3.52 | 0.57 | 557.62 | 1669.84 |
| 104 | 116 | (A)TWNRDHNLITAmK(Y) | M(115) | 3 | 2.18 | 0.31 | 539.27 | 1614.79 |
| 105 | 128 | (T)WNRDHNLITAMKYSVVPVYQEFAR( Q) | | 3 | 2.26 | 0.34 | 979.83 | 2936.46 |
| 107 | 116 | (N)RDHNLITAMK(Y) | | 2 | 2.89 | 0.48 | 599.82 | 1197.63 |
| 108 | 116 | (R)DHNLITAmK(Y) | M(115) | 2 | 3.80 | 0.61 | 529.77 | 1057.53 |
| 108 | 116 | (R)DHNLITAMK(Y) | | 2 | 3.45 | 0.56 | 521.77 | 1041.53 |
| 108 | 128 | (R)DHNLITAmKYSVVPVYQEFAR(Q) | M(115) | 4 | 2.38 | 0.37 | 625.07 | 2496.25 |
| 109 | 116 | (D)HNLITAMK(Y) | | 2 | 3.74 | 0.60 | 464.26 | 926.50 |
| 116 | 128 | (M)KYSVVPVYQEFAR(Q) | | 2 | 3.73 | 0.60 | 793.43 | 1584.84 |
| 117 | 125 | (K)YSVVPVYQE(F) | | 2 | 1.93 | 0.22 | 542.27 | 1082.53 |
| 117 | 126 | (K)YSVVPVYQEF(A) | | 2 | 2.25 | 0.33 | 615.81 | 1229.60 |
| 117 | 128 | (K)YSVVPVYQEFAR(Q) | | 2 | 3.11 | 0.52 | 729.38 | 1456.74 |
| 121 | 128 | (V)PVYQEFAR(Q) | | 2 | 1.86 | 0.19 | 505.26 | 1008.51 |
| 138 | 152 | (K)mLHAFDYGNEDISGN(V) | M(138) | 2 | 2.27 | 0.34 | 849.86 | 1697.71 |
| 138 | 155 | (K)MLHAFDYGNEDISGNVDS(F) | | 2 | 2.38 | 0.37 | 992.43 | 1982.84 |
| 138 | 163 | (K)mLHAFDYGNEDISGNVDSFWLDGGI R(I) | M(138) | 3 | 3.08 | 0.51 | 982.11 | 2943.31 |
| 138 | 163 | (K)MLHAFDYGNEDISGNVDSFWLDGGI R(I) | | 2 | 3.14 | 0.52 | 1464.67 | 2927.32 |
| 143 | 153 | (F)DYGNEDISGNV(D) | | 2 | 2.50 | 0.40 | 591.75 | 1181.49 |
| 147 | 163 | (N)EDISGNVDSFWLDGGIR(I) | | 2 | 2.56 | 0.41 | 940.44 | 1878.87 |
| 153 | 163 | (N) VDSFWLDGGIR(I) | | 2 | 2.90 | 0.48 | 632.82 | 1263.63 |
| 164 | 174 | (R)ISATEQISFLR(K) | | 2 | 4.43 | 0.66 | 632.85 | 1263.68 |
| 164 | 175 | (R)ISATEQISFLRK(L) | | 2 | 4.21 | 0.64 | 696.90 | 1391.79 |
| 165 | 174 | (I)SATEQISFLR(K) | | 2 | 2.42 | 0.38 | 576.31 | 1150.60 |
| 165 | 175 | (I)SATEQISFLRK(L) | | 2 | 3.50 | 0.57 | 640.36 | 1278.70 |
| 166 | 174 | (S)ATEQISFLR(K) | | 2 | 2.46 | 0.39 | 532.79 | 1063.56 |
| 166 | 175 | (S)ATEQISFLRK(L) | | 2 | 3.28 | 0.54 | 596.84 | 1191.67 |
| 167 | 174 | (A)TEQISFLR(K) | | 2 | 2.28 | 0.34 | 497.27 | 992.53 |
| 167 | 175 | (A)TEQISFLRK(L) | | 2 | 2.74 | 0.45 | 561.32 | 1120.63 |
| 169 | 175 | (E)QISFLRK(L) | | 3 | 2.05 | 0.27 | 297.85 | 890.54 |
| 175 | 186 | (R)KLYHNKLHVSER(S) | | 3 | 2.58 | 0.42 | 508.62 | 1522.84 |
| 176 | 186 | (K)L YHNKLHVSER(S) | | 3 | 2.90 | 0.48 | 465.92 | 1394.75 |
| 190 | 206 | (R)IVKQAmL TEANGDYIIR(A) | M(195) | 2 | 3.21 | 0.53 | 976.03 | 1950.04 |
| 193 | 200 | (K)QAMLTEAN(G) | | 1 | 2.61 | 0.42 | 877.41 | 876.40 |
| 193 | 206 | (K)QAmL TEANGDYIIR(A) | M(195) | 2 | 3.19 | 0.53 | 805.89 | 1609.77 |
| 193 | 206 | (K)QAMLTEANGDYIIR(A) | | 2 | 4.43 | 0.66 | 797.90 | 1593.79 |
| 194 | 206 | (Q)AmLTEANGDYIIR(A) | M(195) | 2 | 3.76 | 0.60 | 741.87 | 1481.73 |
| 196 | 206 | (M)LTEANGDYIIR(A) | | 2 | 2.90 | 0.48 | 632.83 | 1263.65 |
| 207 | 218 | (R)AKTGYSTRIEPK(I) | | 3 | 2.31 | 0.35 | 450.92 | 1349.74 |
| 209 | 218 | (K)TGYSTRIEPK(I) | | 2 | 2.21 | 0.32 | 576.31 | 1150.60 |
| 210 | 218 | (T)GYSTRIEPK(I) | | 2 | 1.81 | 0.17 | 525.79 | 1049.56 |
| 219 | 250 | (K)IGWWVGWVELDDNVWFF AmNmDm PTSDGLGLR(Q) | M(237, 239,241) | 4 | 1.86 | 0.19 | 952.18 | 3804.69 |
| 235 | 250 | (F)FAMNMDMPTSDGLGLR(Q) | | 2 | 3.17 | 0.53 | 878.40 | 1754.78 |
| 236 | 250 | (F)AMNmDmPTSDGLGLR(Q) | M(239,2 41) | 2 | 3.15 | 0.52 | 820.86 | 1639.71 |
| 236 | 250 | (F)AMNMDMPTSDGLGLR(Q) | | 2 | 2.98 | 0.50 | 804.86 | 1607.71 |
| 238 | 250 | (M)NmDMPTSDGLGLR(Q) | M(239) | 2 | 3.11 | 0.52 | 711.82 | 1421.63 |
| 238 | 250 | (M)NMDMPTSDGLGLR(Q) | | 2 | 2.95 | 0.49 | 703.82 | 1405.63 |
| 239 | 250 | (N)mDmPTSDGLGLR(Q) | M(239, 241) | 2 | 2.39 | 0.37 | 662.80 | 1323.58 |
| 245 | 265 | (S)DGLGLRQAITKEVLKQEKIIP(-) | | 3 | 2.55 | 0.41 | 783.80 | 2348.39 |
| 251 | 262 | (R)QAITKEVLKQEK(I) | | 2 | 4.24 | 0.65 | 707.92 | 1413.82 |
| 256 | 262 | (K)EVLKQEK(I) | | 2 | 2.55 | 0.41 | 437.26 | 872.50 |
| 256 | 265 | (K)EVLKQEKIIP(-) | | 2 | 2.11 | 0.29 | 598.87 | 1195.72 |

**[Table 4] Information on position and sequence of each peptide identified as**

| NDM protein | | | | | | | | |
|---|---|---|---|---|---|---|---|---|
| **Start** | **Stop** | **Sequence** | **Oxidatio n site** | **Char ge (z)** | **SEQU EST XCorr** | **SEQ UEST delta Cn** | **Observe d m/z** | **Actual mass value (Da)** |
| 28 | 45 | (M)PGEIRPTIGQQmETGDQR(F) | M (39) | 3 | 3.61 | 0.58 | 677.00 | 2027.98 |
| 28 | 45 | (M)PGEIRPTIGQQMETGDQR(F) | | 3 | 2.30 | 0.35 | 671.67 | 2011.99 |
| 30 | 45 | (G)EIRPTIGQQMETGDQR(F) | | 3 | 1.64 | 0.09 | 620.31 | 1857.91 |
| 33 | 45 | (R)PTIGQQmETGDQR(F) | M (39) | 2 | 2.33 | 0.36 | 738.84 | 1475.67 |
| 33 | 45 | (R)PTIGQQMETGDQR(F) | | 2 | 2.31 | 0.35 | 730.84 | 1459.68 |
| 35 | 45 | (T)IGQQmETGDQR(F) | M (39) | 2 | 2.27 | 0.34 | 639.79 | 1277.57 |
| 45 | 52 | (Q)RFGDL VFR(Q) | | 2 | 1.39 | 0.00 | 505.29 | 1008.56 |
| 46 | 52 | (R)FGDLVFR(Q) | | 2 | 2.72 | 0.45 | 427.23 | 852.45 |
| 47 | 52 | (F)GDLVFR(Q) | | 1 | 1.19 | 0.00 | 706.39 | 705.38 |
| 53 | 61 | (R)QLAPNVWQH(T) | | 2 | 1.33 | 0.00 | 546.79 | 1091.56 |
| 53 | 76 | (R)QLAPNVWQHTSYLDMPGFGAVASN( G) | | 2 | 1.97 | 0.24 | 1302.13 | 2602.25 |
| 53 | 81 | (R)QLAPNVWQHTSYLDmPGFGAVASN GLIVR(D) | M (67) | 3 | 3.91 | 0.62 | 1053.20 | 3156.58 |
| 53 | 81 | (R)QLAPNVWQHTSYLDMPGFGAVASN GLIVR(D) | | 3 | 5.86 | 0.74 | 1047.87 | 3140.60 |
| 53 | 82 | (R)QLAPNVWQHTSYLDMPGFGAVASN GLIVRD(G) | | 4 | 1.61 | 0.07 | 814.92 | 3255.64 |
| 53 | 85 | (R)QLAPNVWQHTSYLDmPGFGAVASN GLIVRDGGR(V) | M (67) | 4 | 2.41 | 0.38 | 886.45 | 3541.77 |
| 58 | 81 | (N)VWQHTSYLDMPGFGAVASNGLIVR( D) | | 3 | 2.26 | 0.34 | 873.45 | 2617.34 |
| 62 | 81 | (H)TSYLDmPGFGAVASNGLIVR(D) | M (67) | 2 | 3.46 | 0.57 | 1042.53 | 2083.05 |
| 68 | 81 | (M)PGFGAVASNGLIVR(D) | | 2 | 2.92 | 0.49 | 679.39 | 1356.76 |
| 71 | 81 | (F)GAVASNGLIVR(D) | | 2 | 1.41 | 0.00 | 528.82 | 1055.62 |
| 86 | 92 | (R)VLVVDTA(W) | | 1 | 0.79 | 0.00 | 716.42 | 715.41 |
| 86 | 102 | (R)VLVVDTAWTDDQTAQIL(N) | | 2 | 2.06 | 0.27 | 944.49 | 1886.97 |
| 86 | 106 | (R)VLVVDTAWTDDQTAQILNWIK(Q) | | 2 | 4.86 | 0.69 | 1215.15 | 2428.28 |
| 91 | 106 | (D)TAWTDDQTAQILNWIK(Q) | | 2 | 1.98 | 0.24 | 952.49 | 1902.96 |
| 94 | 106 | (W)TDDQTAQILNWIK(Q) | | 2 | 2.84 | 0.47 | 773.40 | 1544.79 |
| 96 | 106 | (D)DQTAQILNWIK(Q) | | 2 | 1.71 | 0.12 | 665.37 | 1328.72 |
| 104 | 125 | (N)WIKQEINLPVALAVVTHAHQDK(M) | | 3 | 1.84 | 0.19 | 837.47 | 2509.40 |
| 107 | 120 | (K)QEINLPV ALA VVTH(A) | | 2 | 2.24 | 0.33 | 752.43 | 1502.85 |
| 107 | 121 | (K)QEINLPV ALA VVTHA(H) | | 2 | 2.26 | 0.34 | 787.95 | 1573.89 |
| 107 | 124 | (K)QEINLPV ALA VVTHAHQD(K) | | 3 | 3.06 | 0.51 | 652.35 | 1954.03 |
| 107 | 125 | (K)QEINLPV ALA VVTHAHQDK(M) | | 3 | 7.39 | 0.80 | 695.05 | 2082.13 |
| 110 | 125 | (I)NLPVALAVVTHAHQDK(M) | | 3 | 2.55 | 0.41 | 571.66 | 1711.95 |
| 111 | 125 | (N)LPVALAVVTHAHQDK(M) | | 2 | 2.58 | 0.42 | 799.96 | 1597.90 |
| 112 | 125 | (L)PVALAVVTHAHQDK(M) | | 3 | 5.46 | 0.73 | 495.95 | 1484.82 |
| 114 | 125 | (V)ALAVVTHAHQDK(M) | | 2 | 3.84 | 0.61 | 645.35 | 1288.69 |
| 115 | 125 | (A)LAVVTHAHQDK(M) | | 2 | 3.89 | 0.61 | 609.84 | 1217.66 |
| 116 | 125 | (L)AVVTHAHQDK(M) | | 2 | 3.30 | 0.55 | 553.29 | 1104.57 |
| 117 | 125 | (A) VVTHAHQDK(M) | | 2 | 2.14 | 0.30 | 517.78 | 1033.54 |
| 118 | 125 | (V)VTHAHQDK(M) | | 1 | 1.59 | 0.06 | 935.47 | 934.46 |
| 119 | 125 | (V)THAHQDK(M) | | 1 | 0.55 | 0.03 | 836.40 | 835.39 |
| 126 | 135 | (K)mGGmDALHAA(G) | M (126), M (129) | 2 | 1.65 | 0.09 | 503.21 | 1004.41 |
| 126 | 140 | (K)mGGmDALHAAGIATY(A) | M (126), M (129) | 2 | 1.40 | 0.00 | 755.84 | 1509.67 |
| 126 | 140 | (K)MGGMDALHAAGIATY(A) | | 2 | 1.63 | 0.08 | 739.85 | 1477.68 |
| 126 | 142 | (K)MGGmDALHAAGIATYAN(A) | M (129) | 2 | 1.93 | 0.00 | 840.38 | 1678.75 |
| 126 | 142 | (K)mGGmDALHAAGIATYAN(A) | M (126), M (129) | 2 | 2.78 | 0.46 | 848.38 | 1694.75 |
| 126 | 142 | (K)MGGMDALHAAGIATYAN(A) | | 2 | 2.25 | 0.33 | 832.39 | 1662.76 |
| 126 | 144 | (K)MGGMDALHAAGIATYANAL(S) | | 2 | 2.24 | 0.33 | 924.45 | 1846.88 |
| 126 | 146 | (K)mGGMDALHAAGIATYANALSN(Q) | M (126) | 2 | 2.18 | 0.04 | 1032.98 | 2063.95 |
| 126 | 146 | (K)MGGMDALHAAGIATYANALSN(Q) | | 2 | 2.10 | 0.29 | 1024.99 | 2047.96 |
| 126 | 159 | (K)mGGmDALHAAGIATY ANALSNQLA PQEGMV AAQH(S) | M (126), M (129) | 3 | 2.59 | 0.42 | 1147.88 | 3440.61 |
| 126 | 159 | (K)MGGMDALHAAGIATY ANALSNQLA PQEGMV AAQH(S) | | 3 | 3.73 | 0.60 | 1137.22 | 3408.63 |
| 126 | 161 | (K)MGGMDALHAAGIATY ANALSNQLA PQEGMV AAQHSL(T) | | 3 | 3.83 | 0.61 | 1203.92 | 3608.74 |
| 126 | 163 | (K)MGGMDALHAAGIATYANALSNQLA PQEGMVAAQHSLTF(A) | | 3 | 3.62 | 0.59 | 1286.63 | 3856.85 |
| 126 | 166 | (K)MGGMDALHAAGIATYANALSNQLA PQEGMVAAQHSLTFAAN(G) | | 3 | 2.81 | 0.47 | 1372.00 | 4112.98 |
| 143 | 181 | (N)ALSNQLAPQEGmVAAQHSLTFAANG WVEPATAPNFGPLK(V) | M (153) | 4 | 2.20 | 0.32 | 1013.25 | 4048.97 |
| 143 | 181 | (N)ALSNQLAPQEGMVAAQHSLTFAAN GWVEP AT APNFGPLK(V) | | 3 | 4.58 | 0.67 | 1345.36 | 4033.05 |
| 167 | 181 | (N)GWVEPATAPNFGPLK(V) | | 2 | 1.99 | 0.25 | 792.41 | 1582.81 |
| 168 | 181 | (G)WVEPATAPNFGPLK(V) | | 2 | 2.36 | 0.36 | 763.91 | 1525.80 |
| 169 | 181 | (W)VEPATAPNFGPLK(V) | | 2 | 2.04 | 0.26 | 670.87 | 1339.72 |
| 171 | 181 | (E)PATAPNFGPLK(V) | | 2 | 2.24 | 0.33 | 556.81 | 1111.61 |
| 173 | 181 | (A)TAPNFGPLK(V) | | 2 | 1.59 | 0.05 | 472.77 | 943.52 |
| 175 | 181 | (A)PNFGPLK(V) | | 2 | 2.07 | 0.28 | 386.72 | 771.43 |
| 182 | 202 | (K)VFYPGPGHTSDNITVGIDGTD(I) | | 2 | 2.26 | 0.34 | 1081.51 | 2161.01 |
| 182 | 207 | (K)VFYPGPGHTSDNITVGIDGTDIAFGG( C) | | 2 | 1.78 | 0.16 | 1304.13 | 2606.24 |
| 182 | 208 | (K)VFYPGPGHTSDNITVGIDGTDIAFGGc | | 2 | 2.85 | 0.47 | 1384.14 | 2766.26 |
| | | (L) | | | | | | |
| 182 | 211 | (K)VFYPGPGHTSDNITVGIDGTDIAFGGc LIK(D) | | 3 | 6.14 | 0.76 | 1041.19 | 3120.54 |
| 182 | 214 | (K)VFYPGPGHTSDNITVGIDGTDIAFGGc LIKDSK(A) | | 3 | 8.25 | 0.82 | 1151.24 | 3450.71 |
| 182 | 216 | (K)VFYPGPGHTSDNITVGIDGTDIAFGGc LIKDSKAK(S) | | 4 | 3.32 | 0.55 | 913.47 | 3649.84 |
| 185 | 216 | (Y)PGPGHTSDNITVGIDGTDIAFGGcLIK DSKAK(S) | | 3 | 1.88 | 0.20 | 1081.20 | 3240.59 |
| 190 | 214 | (H)TSDNITVGIDGTDIAFGGcLIKDSK(A) | | 3 | 3.11 | 0.52 | 866.43 | 2596.28 |
| 193 | 211 | (D)NITV GIDGTD IAFGGcLIK(D) | | 2 | 4.00 | 0.63 | 982.52 | 1963.03 |
| 194 | 211 | (N)ITV GIDGTD IAFGGcLIK(D) | | 2 | 2.98 | 0.50 | 925.50 | 1848.98 |
| 194 | 214 | (N)ITVGIDGTDIAFGGcLIKDSK(A) | | 3 | 1.93 | 0.22 | 727.38 | 2179.12 |
| 200 | 211 | (D)GTDIAFGGcLIK(D) | | 2 | 1.56 | 0.04 | 626.33 | 1250.64 |
| 200 | 214 | (D)GTDIAFGGcLIKDSK(A) | | 2 | 2.57 | 0.42 | 791.41 | 1580.80 |
| 203 | 214 | (D)IAFGGcLIKDSK(A) | | 2 | 1.63 | 0.08 | 654.86 | 1307.70 |
| 215 | 234 | (K)AKSLGNLGDADTEHYAASAR(A) | | 2 | 4.25 | 0.65 | 1024.00 | 2045.99 |
| 217 | 223 | (K)SLGNLGD(A) | | 1 | 0.69 | 0.00 | 675.33 | 674.32 |
| 217 | 229 | (K)SLGNLGDADTEHY(A) | | 2 | 1.87 | 0.20 | 696.31 | 1390.61 |
| 217 | 231 | (K)SLGNLGDADTEHYAA(S) | | 2 | 1.75 | 0.14 | 767.35 | 1532.69 |
| 217 | 232 | (K)SLGNLGDADTEHYAAS(A) | | 2 | 2.34 | 0.36 | 810.87 | 1619.72 |
| 217 | 234 | (K)SLGNLGDADTEHYAASAR(A) | | 2 | 5.99 | 0.75 | 924.43 | 1846.85 |
| 217 | 242 | (K)SLGNLGDADTEHYAASARAFGAAFP K(A) | | 3 | 4.83 | 0.69 | 879.77 | 2636.28 |
| 218 | 234 | (S)LGNLGDADTEHYAASAR(A) | | 3 | 2.68 | 0.44 | 587.61 | 1759.81 |
| 219 | 234 | (L)GNLGDADTEHYAASAR(A) | | 2 | 4.78 | 0.69 | 824.37 | 1646.73 |
| 220 | 234 | (G)NLGDADTEHYAASAR(A) | | 2 | 2.16 | 0.31 | 795.86 | 1589.70 |
| 221 | 234 | (N)LGDADTEHYAASAR(A) | | 2 | 4.99 | 0.70 | 738.85 | 1475.68 |
| 222 | 234 | (L)GDADTEHYAASAR(A) | | 2 | 4.57 | 0.67 | 682.30 | 1362.58 |
| 225 | 234 | (A)DTEHYAASAR(A) | | 2 | 1.83 | 0.18 | 560.75 | 1119.49 |
| 235 | 242 | (R)AFGAAFPK(A) | | 2 | 2.26 | 0.34 | 404.72 | 807.43 |
| 235 | 256 | (R)AFGAAFPKASmIVMSHSAPDSR(A) | M (245) | 2 | 2.65 | 0.00 | 1147.56 | 2293.11 |
| 235 | 256 | (R)AFGAAFPKASmIVmSHSAPDSR(A) | M (245), M (248) | 3 | 1.86 | 0.19 | 770.71 | 2309.11 |
| 235 | 256 | (R)AFGAAFPKASMIVMSHSAPDSR(A) | | 2 | 3.89 | 0.61 | 1139.56 | 2277.11 |
| 236 | 242 | (A)FGAAFPK(A) | | 2 | 1.97 | 0.24 | 369.20 | 736.39 |
| 237 | 242 | (F)GAAFPK(A) | | 1 | 1.22 | 0.00 | 590.33 | 589.32 |
| 243 | 254 | (K)ASMIVMSHSAPD(S) | | 2 | 1.67 | 0.10 | 623.29 | 1244.56 |
| 243 | 256 | (K)ASmIVMSHSAPDSR(A) | M (245) | 3 | 5.03 | 0.70 | 502.24 | 1503.69 |
| 243 | 256 | (K)ASmIVmSHSAPDSR(A) | M (245), M (248) | 3 | 4.91 | 0.69 | 507.57 | 1519.69 |
| 243 | 256 | (K)ASMIVMSHSAPD SR(A) | | 3 | 5.74 | 0.74 | 496.91 | 1487.69 |
| 244 | 256 | (A)SmIVmSHSAPDSR(A) | M (248) | 2 | 1.67 | 0.10 | 725.33 | 1448.64 |
| 244 | 256 | (A)SMIVMSHSAPDSR(A) | | 2 | 4.54 | 0.67 | 709.33 | 1416.65 |
| 245 | 256 | (S)MIVmSHSAPDSR(A) | M (248) | 2 | 2.85 | 0.47 | 673.82 | 1345.62 |
| 245 | 256 | (S)MIVMSHSAPDSR(A) | | 2 | 3.54 | 0.58 | 665.82 | 1329.62 |
| 246 | 256 | (M)IVmSHSAPDSR(A) | M (248) | 2 | 3.10 | 0.52 | 608.29 | 1214.57 |
| 247 | 256 | (I)VMSHSAPDSR(A) | | 2 | 3.06 | 0.51 | 543.76 | 1085.50 |
| 251 | 256 | (H)SAPDSR(A) | | 1 | 0.54 | 0.00 | 632.30 | 631.29 |
| 257 | 264 | (R)AAITHTAR(M) | | 2 | 2.74 | 0.45 | 420.74 | 839.46 |
| 258 | 264 | (A)AITHTAR(M) | | 2 | 1.81 | 0.17 | 385.22 | 768.42 |
| 259 | 264 | (A)ITHTAR(M) | | 1 | 1.38 | 0.00 | 698.39 | 697.39 |
| 265 | 270 | (R)mADKLR(-) | M (265) | 2 | 2.08 | 0.28 | 375.20 | 748.39 |

**[Table 5] Peptide information on position and sequence of each peptide identified as IPM protein**

| **Start** | **Stop** | **Sequence** | **Oxidat ion site** | **Charge (z)** | **SEQUEST XCorr** | **SEQUES T deltaCn** | **Observ ed m/z** | **Actual mass value (Da)** |
|---|---|---|---|---|---|---|---|---|
| 19 | 26 | (A)AESLPDLK(I) | | 2 | 2.48 | 0.40 | 436.74 | 871.47 |
| 19 | 29 | (A)AESLPDLKIEK(L) | | 2 | 3.47 | 0.57 | 621.85 | 1241.69 |
| 20 | 26 | (A)ESLPDLK(I) | | 2 | 2.06 | 0.27 | 401.22 | 800.43 |
| 20 | 29 | (A)ESLPDLKIEK(L) | | 2 | 3.20 | 0.53 | 586.33 | 1170.65 |
| 21 | 29 | (E)SLPDLKIEK(L) | | 3 | 2.53 | 0.41 | 348.21 | 1041.61 |
| 22 | 29 | (S)LPDLKIEK(L) | | 2 | 2.99 | 0.50 | 478.29 | 954.57 |
| 23 | 29 | (L)PDLKIEK(L) | | 2 | 3.04 | 0.51 | 421.75 | 841.49 |
| 27 | 37 | (K)IEKLDEGVYVH(T) | | 3 | 2.29 | 0.34 | 434.56 | 1300.67 |
| 27 | 51 | (K)IEKLDEGVYVHTSFEEVNG WGVVPK(H) | | 3 | 5.36 | 0.72 | 944.49 | 2830.44 |
| 30 | 37 | (K)LDEGVYVH(T) | | 2 | 2.43 | 0.38 | 466.23 | 930.45 |
| 30 | 51 | (K)LDEGVYVHTSFEEVNGWG VVPK(H) | | 2 | 6.47 | 0.77 | 1231.11 | 2460.20 |
| 38 | 51 | (H)TSFEEVNGWGVVPK(H) | | 2 | 2.67 | 0.44 | 774.89 | 1547.76 |
| 52 | 59 | (K)HGL VVLVN(A) | | 2 | 1.88 | 0.20 | 425.76 | 849.51 |
| 52 | 72 | (K)HGL VVLVNAEAYLIDTPFT AK(D) | | 2 | 6.65 | 0.77 | 1136.13 | 2270.25 |
| 52 | 76 | (K)HGL VVLVNAEAYLIDTPFT AKDTEK(L) | | 2 | 5.68 | 0.74 | 1372.74 | 2743.46 |
| 58 | 72 | (L)VNAEAYLIDTPFTAK(D) | | 2 | 3.34 | 0.55 | 826.93 | 1651.85 |
| 60 | 72 | (N)AEAYLIDTPFTAK(D) | | 2 | 3.60 | 0.58 | 720.38 | 1438.74 |
| 60 | 76 | (N)AEAYLIDTPFTAKDTEK(L) | | 2 | 4.60 | 0.67 | 956.98 | 1911.95 |
| 61 | 72 | (A)EAYLIDTPFTAK(D) | | 2 | 1.97 | 0.24 | 684.86 | 1367.70 |
| 64 | 72 | (Y)LIDTPFTAK(D) | | 2 | 1.84 | 0.19 | 503.29 | 1004.56 |
| 64 | 76 | (Y)LIDTPFTAKDTEK(L) | | 2 | 3.50 | 0.57 | 739.90 | 1477.78 |
| 73 | 84 | (K)DTEKLVTWFVER(G) | | 2 | 3.91 | 0.62 | 761.90 | 1521.79 |
| 73 | 87 | (K)DTEKLVTWFVERGYK(I) | | 3 | 3.36 | 0.55 | 624.33 | 1869.97 |
| 75 | 84 | (T)EKL VTWFVER(G) | | 2 | 2.62 | 0.43 | 653.86 | 1305.72 |
| 76 | 84 | (E)KLVTWFVER(G) | | 2 | 2.32 | 0.35 | 589.34 | 1176.67 |
| 77 | 84 | (K)L VTWFVER(G) | | 2 | 2.62 | 0.43 | 525.29 | 1048.57 |
| 77 | 85 | (K)L VTWFVERG(Y) | | 2 | 2.43 | 0.38 | 553.81 | 1105.60 |
| 77 | 87 | (K)L VTWFVERGYK(I) | | 2 | 2.49 | 0.40 | 699.39 | 1396.76 |
| 78 | 84 | (L) VTWFVER(G) | | 2 | 1.85 | 0.19 | 468.75 | 935.49 |
| 88 | 99 | (K)IKGSISSHFHSD(S) | | 3 | 3.27 | 0.54 | 438.89 | 1313.64 |
| 88 | 100 | (K)IKGSISSHFHSDS(T) | | 3 | 2.29 | 0.35 | 467.90 | 1400.67 |
| 88 | 102 | (K)IKGSISSHFHSDSTG(G) | | 3 | 2.29 | 0.34 | 520.59 | 1558.74 |
| 88 | 105 | (K)IKGSISSHFHSDSTGGIE(W) | | 2 | 5.14 | 0.71 | 929.96 | 1857.90 |
| 88 | 110 | (K)IKGSISSHFHSDSTGGIEWLNSR(S) | | 3 | 8.19 | 0.82 | 839.08 | 2514.23 |
| 90 | 98 | (K)GSISSHFHS(D) | | 2 | 1.88 | 0.20 | 479.73 | 957.44 |
| 90 | 99 | (K)GSISSHFHSD(S) | | 3 | 1.85 | 0.19 | 358.49 | 1072.46 |
| 90 | 101 | (K)GSISSHFHSDST(G) | | 2 | 2.83 | 0.47 | 631.28 | 1260.54 |
| 90 | 102 | (K)GSISSHFHSDSTG(G) | | 2 | 3.51 | 0.57 | 659.79 | 1317.56 |
| 90 | 105 | (K)GSISSHFHSDSTGGIE(W) | | 2 | 3.89 | 0.61 | 809.36 | 1616.71 |
| 90 | 110 | (K)GSISSHFHSDSTGGIEWLNS R(S) | | 3 | 8.69 | 0.83 | 758.69 | 2273.05 |
| 90 | 111 | (K)GSISSHFHSDSTGGIEWLNS RS(I) | | 3 | 2.00 | 0.25 | 787.70 | 2360.09 |
| 97 | 110 | (F)HSDSTGGIEWLNSR(S) | | 2 | 4.66 | 0.68 | 779.87 | 1557.72 |
| 98 | 110 | (H)SDSTGGIEWLNSR(S) | | 2 | 3.76 | 0.60 | 711.34 | 1420.67 |
| 99 | 110 | (S)DSTGGIEWLNSR(S) | | 2 | 2.13 | 0.30 | 667.82 | 1333.63 |
| 100 | 110 | (D)STGGIEWLNSR(S) | | 2 | 2.80 | 0.46 | 610.31 | 1218.61 |
| 103 | 110 | (G)GIEWLNSR(S) | | 2 | 2.33 | 0.36 | 487.76 | 973.50 |
| 104 | 110 | (G)IEWLNSR(S) | | 2 | 2.09 | 0.28 | 459.25 | 916.48 |
| 111 | 125 | (R)SIPTYASELTNELLK(K) | | 2 | 4.29 | 0.65 | 839.95 | 1677.88 |
| 111 | 126 | (R)SIPTYASELTNELLKK(D) | | 3 | 4.86 | 0.69 | 603.00 | 1805.98 |
| 111 | 127 | (R)SIPTYASELTNELLKKD(G) | | 2 | 3.35 | 0.55 | 961.51 | 1921.02 |
| 111 | 129 | (R)SIPTYASELTNELLKKDGK( V) | | 3 | 4.29 | 0.65 | 703.05 | 2106.13 |
| 113 | 125 | (I)PTYASELTNELLK(K) | | 2 | 4.75 | 0.68 | 739.89 | 1477.76 |
| 113 | 126 | (I)PTYASELTNELLKK(D) | | 3 | 7.19 | 0.79 | 536.30 | 1605.87 |
| 113 | 129 | (I)PTYASELTNELLKKDGK(V) | | 3 | 3.48 | 0.57 | 636.34 | 1906.00 |
| 114 | 126 | (P)TYASELTNELLKK(D) | | 2 | 3.89 | 0.61 | 755.42 | 1508.82 |
| 115 | 126 | (T)YASELTNELLKK(D) | | 3 | 1.96 | 0.24 | 470.26 | 1407.77 |
| 116 | 125 | (Y)ASEL TNELLK(K) | | 2 | 1.77 | 0.15 | 559.31 | 1116.61 |
| 116 | 126 | (Y)ASEL TNELLKK(D) | | 2 | 2.90 | 0.48 | 623.36 | 1244.70 |
| 116 | 129 | (Y)ASEL TNELLKKDGK(V) | | 2 | 3.63 | 0.59 | 773.43 | 1544.84 |
| 118 | 126 | (S)ELTNELLKK(D) | | 3 | 1.83 | 0.18 | 363.22 | 1086.63 |
| 126 | 136 | (K)KDGKVQA TNSF(S) | | 2 | 1.99 | 0.25 | 597.81 | 1193.61 |
| 126 | 145 | (K)KDGKVQA TNSFSGVNYWL VK(N) | | 2 | 5.39 | 0.72 | 1121.09 | 2240.16 |
| 127 | 136 | (K)DGKVQATNSF(S) | | 2 | 2.04 | 0.26 | 533.76 | 1065.51 |
| 127 | 140 | (K)DGKVQATNSFSGVN(Y) | | 2 | 2.68 | 0.44 | 712.35 | 1422.68 |
| 127 | 141 | (K)DGKVQATNSFSGVNY(W) | | 2 | 3.35 | 0.55 | 793.88 | 1585.74 |
| 127 | 145 | (K)DGKVQATNSFSGVNYWLV K(N) | | 2 | 6.57 | 0.77 | 1057.04 | 2112.07 |
| 129 | 145 | (G)KVQATNSFSGVNYWLVK(N ) | | 2 | 6.17 | 0.76 | 971.02 | 1940.03 |
| 130 | 136 | (K)VQATNSF(S) | | 1 | 2.42 | 0.38 | 766.38 | 765.37 |
| 130 | 141 | (K) VQA TNSFSGVNY (W) | | 2 | 2.28 | 0.34 | 643.81 | 1285.60 |
| 130 | 145 | (K)VQATNSFSGVNYWLVK(N) | | 2 | 5.47 | 0.73 | 906.97 | 1811.92 |
| 132 | 145 | (Q)ATNSFSGVNYWLVK(N) | | 2 | 4.10 | 0.63 | 793.41 | 1584.80 |
| 133 | 145 | (A)TNSFSGVNYWLVK(N) | | 2 | 3.14 | 0.52 | 757.89 | 1513.76 |
| 134 | 145 | (T)NSFSGVNYWLVK(N) | | 2 | 2.64 | 0.43 | 707.36 | 1412.71 |
| 135 | 145 | (N)SFSGVNYWLVK(N) | | 2 | 3.07 | 0.51 | 650.34 | 1298.67 |
| 137 | 145 | (F)SGVNYWLVK(N) | | 1 | 2.26 | 0.34 | 1065.58 | 1064.57 |
| 146 | 157 | (K)NKIEVFYPGPGH(T) | | 2 | 3.85 | 0.61 | 679.35 | 1356.68 |
| 146 | 158 | (K)NKIEVFYPGPGHT(P) | | 2 | 2.75 | 0.45 | 729.88 | 1457.74 |
| 146 | 160 | (K)NKIEVFYPGPGHTPD(N) | | 2 | 4.74 | 0.68 | 835.92 | 1669.82 |
| 146 | 161 | (K)NKIEVFYPGPGHTPDN(V) | | 2 | 4.79 | 0.69 | 892.94 | 1783.86 |
| 146 | 169 | (K)NKIEVFYPGPGHTPDNVVV WLPER(K) | | 3 | 7.92 | 0.81 | 921.81 | 2762.41 |
| 146 | 170 | (K)NKIEVFYPGPGHTPDNVVV WLPERK(I) | | 3 | 7.59 | 0.80 | 964.51 | 2890.52 |
| 147 | 170 | (N)KIEVFYPGPGHTPDNVVVW LPERK(I) | | 3 | 1.79 | 0.16 | 926.50 | 2776.48 |
| 148 | 160 | (K)IEVFYPGPGHTPD(N) | | 2 | 2.83 | 0.47 | 714.85 | 1427.68 |
| 148 | 161 | (K)IEVFYPGPGHTPDN(V) | | 2 | 2.54 | 0.41 | 771.87 | 1541.72 |
| 148 | 169 | (K)IEVFYPGPGHTPDNVVVWL PER(K) | | 2 | 4.76 | 0.69 | 1261.15 | 2520.28 |
| 148 | 170 | (K)IEVFYPGPGHTPDNVVVWL PERK(I) | | 3 | 5.72 | 0.74 | 883.80 | 2648.37 |
| 152 | 170 | (F) YPGPGHTPDNVVVWLPERK (I) | | 3 | 1.99 | 0.25 | 721.04 | 2160.11 |
| 153 | 170 | (Y)PGPGHTPDNVVVWLPERK(I ) | | 3 | 2.90 | 0.48 | 666.69 | 1997.05 |
| 155 | 169 | (G)PGHTPDNVVVWLPER(K) | | 3 | 3.66 | 0.59 | 572.64 | 1714.89 |
| 157 | 169 | (G)HTPDNVVVWLPER(K) | | 3 | 3.39 | 0.56 | 521.28 | 1560.81 |
| 158 | 169 | (H)TPDNVVVWLPER(K) | | 2 | 4.30 | 0.65 | 712.88 | 1423.75 |
| 158 | 170 | (H)TPDNVVVWLPERK(I) | | 2 | 2.16 | 0.31 | 776.93 | 1551.85 |
| 161 | 170 | (D)NVVVWLPERK(I) | | 2 | 2.01 | 0.25 | 620.37 | 1238.72 |
| 162 | 169 | (N) VVVWLPER(K) | | 2 | 2.01 | 0.25 | 499.30 | 996.58 |
| 162 | 170 | (N) VVVWLPERK(I) | | 2 | 2.25 | 0.33 | 563.35 | 1124.68 |
| 170 | 176 | (R)KILFGGc(F) | | 2 | 1.99 | 0.25 | 397.72 | 793.42 |
| 170 | 177 | (R)KILFGGcF(I) | | 2 | 1.76 | 0.15 | 471.25 | 940.49 |
| 170 | 179 | (R)KILFGGcFIK(P) | | 2 | 3.85 | 0.61 | 591.84 | 1181.67 |
| 170 | 181 | (R)KILFGGcFIKPY (G) | | 2 | 3.83 | 0.61 | 721.90 | 1441.79 |
| 170 | 185 | (R)KILFGGcFIKPYGLGN(L) | | 2 | 4.54 | 0.67 | 892.49 | 1782.96 |
| 170 | 196 | (R)KILFGGcFIKPYGLGNLGDA NIEAWPK(S) | | 3 | 3.74 | 0.60 | 993.53 | 2977.58 |
| 171 | 181 | (K)ILFGGcFIKPY (G) | | 2 | 2.71 | 0.45 | 657.85 | 1313.69 |
| 171 | 182 | (K)ILFGGcFIKPY G(L) | | 2 | 1.99 | 0.25 | 686.36 | 1370.71 |
| 171 | 196 | (K)ILFGGcFIKPYGLGNLGDANI EAWPK(S) | | 2 | 3.43 | 0.56 | 1425.75 | 2849.48 |
| 177 | 196 | (C)FIKPYGLGNLGDANIEAWPK (S) | | 3 | 2.71 | 0.45 | 735.06 | 2202.16 |
| 178 | 196 | (F)IKPYGLGNLGDANIEAWPK( S) | | 2 | 3.24 | 0.54 | 1028.55 | 2055.08 |
| 180 | 196 | (K)PYGLGNLGDANIEAWPK(S) | | 2 | 2.53 | 0.41 | 907.96 | 1813.91 |
| 182 | 196 | (Y)GLGNLGDANIEA WPK(S) | | 2 | 4.68 | 0.68 | 777.90 | 1553.79 |
| 182 | 199 | (Y)GLGNLGDANIEA WPKSAK( L) | | 2 | 2.71 | 0.45 | 920.99 | 1839.96 |
| 184 | 196 | (L)GNLGDANIEAWPK(S) | | 2 | 3.70 | 0.59 | 692.85 | 1383.69 |
| 186 | 196 | (N)LGDANIEAWPK(S) | | 2 | 3.55 | 0.58 | 607.32 | 1212.62 |
| 205 | 225 | (K)YGKAKLVVPGHSEVGDASL LK(L) | | 3 | 5.90 | 0.75 | 723.41 | 2167.21 |
| 206 | 225 | (Y)GKAKLVVPGHSEVGDASLL K(L) | | 3 | 3.58 | 0.58 | 669.06 | 2004.15 |
| 208 | 220 | (K)AKL VVPGHSEVGD(A) | | 2 | 2.98 | 0.50 | 654.35 | 1306.69 |
| 208 | 224 | (K)AKL VVPGHSEVGDASLL(K) | | 2 | 3.35 | 0.55 | 846.48 | 1690.94 |
| 208 | 225 | (K)AKL VVPGHSEVGDASLLK(L ) | | 2 | 5.43 | 0.72 | 910.52 | 1819.03 |
| 208 | 233 | (K)AKL VVPGHSEVGDASLLKL TLEQA VK(G) | | 3 | 2.13 | 0.30 | 901.52 | 2701.54 |
| 209 | 225 | (A)KLVVPGHSEVGDASLLK(L) | | 3 | 5.50 | 0.73 | 583.67 | 1747.99 |
| 210 | 219 | (K)L VVPGHSEVG(D) | | 2 | 1.83 | 0.18 | 497.27 | 992.53 |
| 210 | 220 | (K)L VVPGHSEVGD(A) | | 2 | 2.52 | 0.41 | 554.79 | 1107.56 |
| 210 | 221 | (K)L VVPGHSEVGDA(S) | | 2 | 1.89 | 0.21 | 590.31 | 1178.60 |
| 210 | 224 | (K)L VVPGHSEVGDASLL(K) | | 2 | 2.71 | 0.45 | 746.90 | 1491.79 |
| 210 | 225 | (K)L VVPGHSEVGDASLLK(L) | | 2 | 4.77 | 0.69 | 810.96 | 1619.90 |
| 210 | 226 | (K)L VVPGHSEVGDASLLKL(T) | | 2 | 1.99 | 0.25 | 867.50 | 1732.98 |
| 210 | 233 | (K)L VVPGHSEVGDASLLKL TL EQAVK(G) | | 3 | 3.22 | 0.53 | 835.14 | 2502.41 |
| 211 | 225 | (L)VVPGHSEVGDASLLK(L) | | 2 | 3.87 | 0.61 | 754.41 | 1506.81 |
| 212 | 225 | (V) VPGHSEVGDASLLK(L) | | 2 | 5.14 | 0.71 | 704.88 | 1407.74 |
| 213 | 225 | (V)PGHSEVGDASLLK(L) | | 2 | 5.48 | 0.73 | 655.34 | 1308.67 |
| 214 | 225 | (P)GHSEVGDASLLK(L) | | 2 | 4.50 | 0.67 | 606.81 | 1211.61 |
| 215 | 225 | (G)HSEVGDASLLK(L) | | 2 | 4.18 | 0.64 | 578.30 | 1154.59 |
| 216 | 225 | (H)SEVGDASLLK(L) | | 2 | 2.52 | 0.41 | 509.78 | 1017.54 |
| 219 | 225 | (V)GDASLLK(L) | | 2 | 2.38 | 0.37 | 352.20 | 702.39 |
| 225 | 233 | (L)KLTLEQAVK(G) | | 2 | 3.47 | 0.57 | 515.32 | 1028.62 |
| 225 | 239 | (L)KLTLEQAVKGLNESK(K) | | 2 | 4.55 | 0.67 | 829.48 | 1656.95 |
| 226 | 233 | (K)L TLEQAVK(G) | | 2 | 2.44 | 0.38 | 451.27 | 900.53 |
| 226 | 239 | (K)L TLEQA VKGLNESK(K) | | 2 | 4.86 | 0.69 | 765.43 | 1528.85 |
| 226 | 246 | (K)L TLEQA VKGLNESKKPSKPS N(-) | | 4 | 6.74 | 0.78 | 567.82 | 2267.25 |
| 227 | 233 | (L)TLEQA VK(G) | | 2 | 2.31 | 0.35 | 394.73 | 787.45 |
| 227 | 246 | (L)TLEQA VKGLNESKKPSKPSN (-) | | 3 | 8.03 | 0.81 | 719.06 | 2154.17 |
| 228 | 246 | (T)LEQAVKGLNESKKPSKPSN(-) | | 3 | 5.99 | 0.75 | 685.38 | 2053.11 |
| 229 | 246 | (L)EQAVKGLNESKKPSKPSN(-) | | 3 | 6.06 | 0.75 | 647.69 | 1940.03 |
| 230 | 246 | (E)QAVKGLNESKKPSKPSN(-) | | 3 | 5.71 | 0.74 | 604.67 | 1810.98 |
| 231 | 246 | (Q)AVKGLNESKKPSKPSN(-) | | 3 | 4.71 | 0.68 | 561.99 | 1682.94 |
| 232 | 246 | (A)VKGLNESKKPSKPSN(-) | | 3 | 3.90 | 0.62 | 538.30 | 1611.89 |
| 234 | 246 | (K)GLNESKKPSKPSN(-) | | 2 | 4.18 | 0.64 | 693.37 | 1384.73 |
| 238 | 246 | (E)SKKPSKPSN(-) | | 2 | 2.49 | 0.40 | 486.78 | 971.54 |

**[Table 6] Information on position and sequence of each peptide identified as VIM protein**

| **Start** | **Stop** | **Sequence** | **Oxidati on site** | **Char ge (z)** | **SEQUE ST XCorr** | **SEQUE ST deltaCn** | **Observe d m/z** | **Actual mass value (Da)** |
|---|---|---|---|---|---|---|---|---|
| 27 | 45 | (S)VDSSGEYPTVSEIPVGEVR(L) | | 2 | 5.08 | 0.70 | 1010.50 | 2018.99 |
| 28 | 45 | (V)DSSGEYPTVSEIPVGEVR(L) | | 2 | 3.43 | 0.56 | 960.97 | 1919.92 |
| 29 | 45 | (D)SSGEYPTVSEIPVGEVR(L) | | 2 | 4.55 | 0.67 | 903.45 | 1804.89 |
| 31 | 45 | (S)GEYPTVSEIPVGEVR(L) | | 2 | 3.85 | 0.61 | 816.42 | 1630.83 |
| 33 | 45 | (E) YPTVSEIPVGEVR(L) | | 2 | 2.45 | 0.39 | 723.39 | 1444.76 |
| 34 | 45 | (Y)PTVSEIPVGEVR(L) | | 2 | 4.25 | 0.65 | 641.86 | 1281.70 |
| 34 | 46 | (Y)PTVSEIPVGEVRL(Y) | | 2 | 2.80 | 0.47 | 698.40 | 1394.78 |
| 34 | 47 | (Y)PTVSEIPVGEVRLY(Q) | | 2 | 4.39 | 0.66 | 779.93 | 1557.85 |
| 35 | 45 | (P)TVSEIPVGEVR(L) | | 2 | 1.97 | 0.24 | 593.33 | 1184.65 |
| 36 | 45 | (T)VSEIPVGEVR(L) | | 2 | 2.91 | 0.48 | 542.81 | 1083.60 |
| 37 | 45 | (V)SEIPVGEVR(L) | | 2 | 1.74 | 0.14 | 493.27 | 984.53 |
| 46 | 56 | (R)LYQIADGVWSH(I) | | 2 | 2.76 | 0.46 | 644.82 | 1287.63 |
| 46 | 60 | (R)LYQIADGVWSHIATQ(S) | | 2 | 2.67 | 0.44 | 851.44 | 1700.86 |
| 55 | 62 | (W)SHIATQSF(D) | | 2 | 2.15 | 0.30 | 445.72 | 889.43 |
| 55 | 67 | (W)SHIATQSFDGAVY(P) | | 2 | 3.51 | 0.57 | 698.33 | 1394.65 |
| 55 | 72 | (W)SHIATQSFDGAVYPSNGL(I) | | 2 | 4.66 | 0.68 | 932.45 | 1862.89 |
| 55 | 75 | (W)SHIATQSFDGAVYPSNGLIVR(D) | | 2 | 4.76 | 0.68 | 1116.58 | 2231.14 |
| 57 | 75 | (H)IATQSFDGAVYPSNGLIVR(D) | | 2 | 3.76 | 0.60 | 1004.53 | 2007.05 |
| 59 | 75 | (A)TQSFDGAVYPSNGLIVR(D) | | 2 | 3.29 | 0.54 | 912.47 | 1822.92 |
| 60 | 75 | (T)QSFDGAVYPSNGLIVR(D) | | 2 | 2.97 | 0.49 | 861.95 | 1721.88 |
| 63 | 75 | (F)DGAVYPSNGLIVR(D) | | 2 | 3.00 | 0.50 | 680.87 | 1359.72 |
| 68 | 80 | (Y)PSNGLIVRDGDEL(L) | | 2 | 3.43 | 0.56 | 692.86 | 1383.71 |
| 68 | 81 | (Y)PSNGLIVRDGDELL(L) | | 2 | 5.05 | 0.70 | 749.40 | 1496.79 |
| 73 | 81 | (L)IVRDGDELL(L) | | 2 | 2.27 | 0.34 | 515.28 | 1028.55 |
| 76 | 90 | (R)DGDELLLIDTAWGAK(N) | | 2 | 3.70 | 0.59 | 808.92 | 1615.82 |
| 77 | 90 | (D)GDELLLIDTAWGAK(N) | | 2 | 2.13 | 0.30 | 751.40 | 1500.79 |
| 81 | 87 | (L)LLIDTAW(G) | | 1 | 1.63 | 0.08 | 831.46 | 830.46 |
| 81 | 90 | (L)LLIDTAWGAK(N) | | 2 | 1.77 | 0.15 | 544.31 | 1086.61 |
| 88 | 96 | (W)GAKNTAALL(A) | | 2 | 1.85 | 0.19 | 429.76 | 857.50 |
| 88 | 109 | (W)GAKNTAALLAEIEKQIGLPVTR(A) | | 3 | 6.16 | 0.76 | 765.12 | 2292.33 |
| 89 | 101 | (G)AKNTAALLAEIEK(Q) | | 3 | 2.13 | 0.29 | 457.94 | 1370.78 |
| 90 | 101 | (A)KNTAALLAEIEK(Q) | | 2 | 4.46 | 0.66 | 650.88 | 1299.74 |
| 91 | 101 | (K)NTAALLAEIEK(Q) | | 2 | 3.92 | 0.62 | 586.83 | 1171.65 |
| 91 | 109 | (K)NTAALLAEIEKQIGLPVTR(A) | | 2 | 5.72 | 0.74 | 1019.10 | 2036.18 |
| 92 | 101 | (N)TAALLAEIEK(Q) | | 2 | 3.40 | 0.56 | 529.81 | 1057.61 |
| 92 | 109 | (N)TAALLAEIEKQIGLPVTR(A) | | 3 | 4.81 | 0.69 | 641.72 | 1922.13 |
| 93 | 101 | (T)AALLAEIEK(Q) | | 1 | 2.41 | 0.38 | 957.56 | 956.56 |
| 95 | 101 | (A)LLAEIEK(Q) | | 1 | 2.47 | 0.00 | 815.49 | 814.48 |
| 96 | 109 | (L)LAEIEKQIGLPVTR(A) | | 2 | 3.17 | 0.53 | 783.97 | 1565.92 |
| 96 | 115 | (L)LAEIEKQIGLPVTRA VSTHF (H) | | 2 | 5.38 | 0.72 | 1105.13 | 2208.25 |
| 97 | 109 | (L)AEIEKQIGLPVTR(A) | | 3 | 2.86 | 0.47 | 485.29 | 1452.83 |
| 97 | 115 | (L)AEIEKQIGLPVTRAVSTHF(H) | | 2 | 4.43 | 0.66 | 1048.58 | 2095.15 |
| 97 | 126 | (L)AEIEKQIGLPVTRAVSTHFHDDRV GGVDVL(R) | | 3 | 4.13 | 0.64 | 1086.92 | 3257.74 |
| 100 | 109 | (I)EKQIGLPVTR(A) | | 2 | 2.16 | 0.30 | 570.84 | 1139.67 |
| 102 | 109 | (K)QIGLPVTR(A) | | 2 | 2.81 | 0.47 | 442.27 | 882.53 |
| 102 | 119 | (K)QIGLPVTRAVSTHFHDDR(V) | | 4 | 2.34 | 0.36 | 513.02 | 2048.07 |
| 103 | 109 | (Q)IGLPVTR(A) | | 2 | 1.84 | 0.19 | 378.24 | 754.47 |
| 109 | 127 | (T)RAVSTHFHDDRVGGVDVLR(A) | | 4 | 4.65 | 0.68 | 534.78 | 2135.11 |
| 110 | 117 | (R)A VSTHFHD(D) | | 2 | 1.97 | 0.24 | 457.21 | 912.41 |
| 110 | 119 | (R)A VSTHFHDDR(V) | | 2 | 3.67 | 0.59 | 592.78 | 1183.54 |
| 110 | 121 | (R)A VSTHFHDDRVG(G) | | 2 | 1.88 | 0.20 | 670.82 | 1339.63 |
| 110 | 122 | (R)AVSTHFHDDRVGG(V) | | 3 | 1.99 | 0.24 | 466.56 | 1396.66 |
| 110 | 124 | (R)A VSTHFHDDRVGGVD(V) | | 2 | 2.94 | 0.49 | 806.38 | 1610.75 |
| 110 | 127 | (R)A VSTHFHDDRVGGVDVLR(A) | | 2 | 3.95 | 0.62 | 990.51 | 1979.00 |
| 110 | 140 | (R)AVSTHFHDDRVGGVDVLRAAGVA TY ASPSTR(R) | | 4 | 1.86 | 0.19 | 803.91 | 3211.63 |
| 111 | 119 | (A)VSTHFHDDR(V) | | 2 | 2.62 | 0.43 | 557.26 | 1112.50 |
| 111 | 127 | (A)VSTHFHDDRVGGVDVLR(A) | | 3 | 4.76 | 0.68 | 637.00 | 1907.97 |
| 112 | 119 | (V)STHFHDDR(V) | | 2 | 2.67 | 0.44 | 507.72 | 1013.43 |
| 112 | 127 | (V)STHFHDDRVGGVDVLR(A) | | 3 | 4.15 | 0.64 | 603.97 | 1808.90 |
| 113 | 127 | (S)THFHDDRVGGVDVLR(A) | | 3 | 1.76 | 0.15 | 574.96 | 1721.87 |
| 114 | 127 | (T)HFHDDRVGGVDVLR(A) | | 3 | 3.69 | 0.59 | 541.28 | 1620.82 |
| 115 | 127 | (H)FHDDRVGGVDVLR(A) | | 2 | 2.80 | 0.46 | 742.88 | 1483.76 |
| 116 | 126 | (F)HDDRVGGVDVL(R) | | 2 | 2.89 | 0.48 | 591.30 | 1180.59 |
| 116 | 127 | (F)HDDRVGGVDVLR(A) | | 2 | 2.93 | 0.49 | 669.35 | 1336.69 |
| 116 | 134 | (F)HDDRVGGVDVLRAAGVATY(A) | | 3 | 4.41 | 0.66 | 657.68 | 1970.00 |
| 116 | 140 | (F)HDDRVGGVDVLRAAGVATYASPS TR(R) | | 3 | 2.16 | 0.31 | 857.44 | 2569.29 |
| 117 | 127 | (H)DDRVGGVDVLR(A) | | 2 | 2.87 | 0.48 | 600.82 | 1199.62 |
| 118 | 127 | (D)DRVGGVDVLR(A) | | 3 | 2.35 | 0.36 | 362.54 | 1084.60 |
| 120 | 127 | (R)VGGVDVLR(A) | | 2 | 2.83 | 0.47 | 407.74 | 813.47 |
| 120 | 140 | (R) VGGVD VLRAAGVATY ASPSTR(R) | | 3 | 4.02 | 0.63 | 683.04 | 2046.09 |
| 121 | 127 | (V)GGVDVLR(A) | | 1 | 1.28 | 0.00 | 715.41 | 714.40 |
| 125 | 140 | (D)VLRAAGVATYASPSTR(R) | | 3 | 2.14 | 0.30 | 540.64 | 1618.89 |
| 127 | 134 | (L)RAAGVATY(A) | | 2 | 1.82 | 0.18 | 404.72 | 807.43 |
| 128 | 140 | (R)AAGVATYASPSTR(R) | | 2 | 3.99 | 0.62 | 626.32 | 1250.63 |
| 128 | 141 | (R)AAGVATYASPSTRR(L) | | 2 | 3.09 | 0.51 | 704.37 | 1406.73 |
| 129 | 140 | (A)AGVATYASPSTR(R) | | 2 | 3.22 | 0.53 | 590.80 | 1179.59 |
| 129 | 141 | (A)AGVATYASPSTRR(L) | | 2 | 1.87 | 0.20 | 668.85 | 1335.69 |
| 130 | 140 | (A)GVATYASPSTR(R) | | 2 | 2.80 | 0.46 | 555.29 | 1108.56 |
| 130 | 141 | (A)GVATYASPSTRR(L) | | 2 | 3.03 | 0.51 | 633.34 | 1264.66 |
| 131 | 140 | (G)VATYASPSTR(R) | | 2 | 2.63 | 0.43 | 526.77 | 1051.53 |
| 132 | 141 | (V)ATYASPSTRR(L) | | 2 | 2.27 | 0.34 | 555.29 | 1108.57 |
| 135 | 155 | (Y)ASPSTRRLAEVEGNEIPTHSL(E) | | 4 | 2.34 | 0.36 | 566.80 | 2263.17 |
| 135 | 158 | (Y)ASPSTRRLAEVEGNEIPTHSLEGL(S ) | | 4 | 4.13 | 0.64 | 641.58 | 2562.31 |
| 141 | 153 | (R)RLAEVEGNEIPTH(S) | | 3 | 2.33 | 0.36 | 488.92 | 1463.74 |
| 141 | 155 | (R)RLAEVEGNEIPTHSL(E) | | 3 | 2.98 | 0.50 | 555.63 | 1663.86 |
| 141 | 156 | (R)RLAEVEGNEIPTHSLE(G) | | 2 | 2.51 | 0.40 | 897.46 | 1792.90 |
| 141 | 157 | (R)RLAEVEGNEIPTHSLEG(L) | | 3 | 3.43 | 0.56 | 617.65 | 1849.92 |
| 141 | 158 | (R)RLAEVEGNEIPTHSLEGL(S) | | 3 | 4.87 | 0.69 | 655.34 | 1963.01 |
| 141 | 163 | (R)RLAEVEGNEIPTHSLEGLSSSGD(A) | | 3 | 3.95 | 0.62 | 799.73 | 2396.17 |
| 141 | 166 | (R)RLAEVEGNEIPTHSLEGLSSSGDAV R(F) | | 4 | 8.05 | 0.81 | 681.60 | 2722.38 |
| 142 | 155 | (R)LAEVEGNEIPTHSL(E) | | 2 | 3.01 | 0.50 | 754.89 | 1507.76 |
| 142 | 158 | (R)LAEVEGNEIPTHSLEGL(S) | | 2 | 2.70 | 0.44 | 904.47 | 1806.93 |
| 142 | 163 | (R)LAEVEGNEIPTHSLEGLSSSGD(A) | | 2 | 3.54 | 0.58 | 1121.03 | 2240.05 |
| 142 | 166 | (R)LAEVEGNEIPTHSLEGLSSSGDAVR (F) | | 2 | 7.36 | 0.80 | 1284.13 | 2566.25 |
| 143 | 155 | (L)AEVEGNEIPTHSL(E) | | 2 | 2.41 | 0.38 | 698.34 | 1394.67 |
| 143 | 158 | (L)AEVEGNEIPTHSLEGL(S) | | 2 | 3.41 | 0.56 | 847.92 | 1693.82 |
| 143 | 166 | (L)AEVEGNEIPTHSLEGLSSSGDAVR( F) | | 2 | 4.68 | 0.68 | 1227.60 | 2453.19 |
| 143 | 167 | (L)AEVEGNEIPTHSLEGLSSSGDAVRF (G) | | 3 | 6.07 | 0.75 | 867.76 | 2600.25 |
| 144 | 166 | (A)EVEGNEIPTHSLEGLSSSGDAVR(F) | | 3 | 5.10 | 0.71 | 795.05 | 2382.14 |
| 145 | 166 | (E)VEGNEIPTHSLEGLSSSGDAVR(F) | | 3 | 5.67 | 0.74 | 752.04 | 2253.09 |
| 146 | 166 | (V)EGNEIPTHSLEGLSSSGDAVR(F) | | 2 | 5.27 | 0.72 | 1078.02 | 2154.02 |
| 147 | 166 | (E)GNEIPTHSLEGLSSSGDAVR(F) | | 3 | 5.07 | 0.70 | 676.00 | 2024.98 |
| 148 | 166 | (G)NEIPTHSLEGLSSSGDAVR(F) | | 3 | 4.97 | 0.70 | 656.99 | 1967.96 |
| 149 | 166 | (N)EIPTHSLEGLSSSGDAVR(F) | | 3 | 4.99 | 0.70 | 618.98 | 1853.91 |
| 151 | 166 | (I)PTHSLEGLSSSGDAVR(F) | | 2 | 5.07 | 0.70 | 806.90 | 1611.79 |
| 153 | 166 | (T)HSLEGLSSSGDAVR(F) | | 2 | 3.24 | 0.54 | 707.85 | 1413.69 |
| 154 | 166 | (H)SLEGLSSSGDAVR(F) | | 2 | 3.10 | 0.52 | 639.32 | 1276.63 |
| 156 | 166 | (L)EGLSSSGDAVR(F) | | 2 | 2.69 | 0.44 | 539.26 | 1076.51 |
| 156 | 167 | (L)EGLSSSGDAVRF(G) | | 2 | 2.59 | 0.42 | 612.79 | 1223.57 |
| 157 | 166 | (E)GLSSSGDAVR(F) | | 2 | 2.61 | 0.42 | 474.74 | 947.47 |
| 159 | 167 | (L)SSSGDAVRF(G) | | 2 | 2.27 | 0.34 | 463.22 | 924.43 |
| 159 | 172 | (L)SSSGDAVRFGPVEL(F) | | 2 | 2.68 | 0.44 | 710.86 | 1419.71 |
| 167 | 179 | (R)FGPVELFYPGAAH(S) | | 2 | 3.73 | 0.60 | 702.85 | 1403.69 |
| 167 | 180 | (R)FGPVELFYPGAAHS(T) | | 2 | 2.07 | 0.28 | 746.37 | 1490.73 |
| 167 | 182 | (R)FGPVELFYPGAAHSTD(N) | | 2 | 2.59 | 0.42 | 854.41 | 1706.80 |
| 174 | 187 | (F)YPGAAHSTDNLVVY(V) | | 2 | 2.45 | 0.39 | 753.87 | 1505.72 |
| 188 | 194 | (Y)VPSASVL(Y) | | 1 | 2.07 | 0.28 | 672.39 | 671.39 |
| 188 | 201 | (Y)VPSASVLYGGcAIY(E) | | 2 | 2.36 | 0.37 | 728.86 | 1455.71 |
| 191 | 205 | (S)ASVLYGGcAIYELSR(T) | | 2 | 1.88 | 0.20 | 829.92 | 1657.82 |
| 192 | 205 | (A)SVLYGGcAIYELSR(T) | | 2 | 2.70 | 0.44 | 794.40 | 1586.78 |
| 193 | 205 | (S)VLYGGcAIYELSR(T) | | 2 | 3.12 | 0.52 | 750.88 | 1499.75 |
| 195 | 205 | (L)Y GGcAIYELSR(T) | | 2 | 3.62 | 0.59 | 644.81 | 1287.60 |
| 196 | 205 | (Y)GGcAIYELSR(T) | | 2 | 2.09 | 0.28 | 563.27 | 1124.53 |
| 197 | 205 | (G)GcAIYELSR(T) | | 2 | 2.29 | 0.35 | 534.76 | 1067.51 |
| 197 | 225 | (G)GcAIYELSRTSAGNVADADLAEWP TSIER(I) | | 3 | 3.45 | 0.56 | 1051.50 | 3151.48 |
| 199 | 205 | (C)AIYELSR(T) | | 2 | 2.15 | 0.30 | 426.24 | 850.46 |
| 202 | 216 | (Y)ELSRTSAGNVADADL(A) | | 2 | 3.79 | 0.60 | 759.88 | 1517.74 |
| 206 | 215 | (R)TSAGNVADAD(L) | | 1 | 1.87 | 0.20 | 920.40 | 919.39 |
| 206 | 218 | (R)TSAGNV ADADLAE(W) | | 2 | 2.97 | 0.50 | 617.29 | 1232.56 |
| 206 | 225 | (R)TSAGNV ADADLAEWPTSIER(I) | | 2 | 6.34 | 0.76 | 1052.00 | 2101.99 |
| 208 | 225 | (S)AGNVADADLAEWPTSIER(I) | | 2 | 2.78 | 0.46 | 957.97 | 1913.92 |
| 211 | 225 | (N)VADADLAEWPTSIER(I) | | 2 | 4.37 | 0.66 | 836.92 | 1671.82 |
| 214 | 225 | (D)ADLAEWPTSIER(I) | | 2 | 2.27 | 0.34 | 694.35 | 1386.68 |
| 216 | 225 | (D)LAEWPTSIER(I) | | 2 | 3.20 | 0.53 | 601.32 | 1200.62 |
| 220 | 235 | (W)PTSIERIQQHYPEAQF(V) | | 2 | 2.77 | 0.46 | 972.49 | 1942.96 |
| 226 | 234 | (R)IQQHYPEAQ(F) | | 2 | 2.41 | 0.38 | 557.27 | 1112.53 |
| 226 | 239 | (R)IQQHYPEAQFVIPG(H) | | 2 | 2.40 | 0.37 | 813.92 | 1625.84 |
| 226 | 240 | (R)IQQHYPEAQFVIPGH(G) | | 3 | 4.16 | 0.64 | 588.64 | 1762.89 |
| 226 | 242 | (R)IQQHYPEAQFVIPGHGL(P) | | 3 | 1.97 | 0.24 | 645.34 | 1932.99 |
| 226 | 244 | (R)IQQHYPEAQFVIPGHGLPG(G) | | 3 | 2.41 | 0.38 | 696.70 | 2087.07 |
| 226 | 245 | (R)IQQHYPEAQFVIPGHGLPGG(L) | | 3 | 2.70 | 0.45 | 715.71 | 2144.09 |
| 226 | 246 | (R)IQQHYPEAQFVIPGHGLPGGL(D) | | 3 | 3.95 | 0.62 | 753.40 | 2257.18 |
| 226 | 247 | (R)IQQHYPEAQFVIPGHGLPGGLD(L) | | 3 | 4.63 | 0.68 | 791.74 | 2372.20 |
| 226 | 248 | (R)IQQHYPEAQFVIPGHGLPGGLDL(L) | | 3 | 2.67 | 0.44 | 829.44 | 2485.28 |
| 226 | 249 | (R)IQQHYPEAQFVIPGHGLPGGLDLL( K) | | 3 | 2.70 | 0.44 | 867.13 | 2598.37 |
| 226 | 250 | (R)IQQHYPEAQFVIPGHGLPGGLDLL K(H) | | 3 | 7.74 | 0.81 | 909.83 | 2726.47 |
| 226 | 254 | (R)IQQHYPEAQFVIPGHGLPGGLDLL KHTTN(V) | | 4 | 2.63 | 0.43 | 795.93 | 3179.69 |
| 226 | 257 | (R)IQQHYPEAQFVIPGHGLPGGLDLL KHTTNVVK(A) | | 5 | 3.45 | 0.57 | 702.19 | 3505.89 |
| 235 | 250 | (Q)FVIPGHGLPGGLDLLK(H) | | 3 | 2.23 | 0.33 | 544.99 | 1631.95 |
| 236 | 246 | (F)VIPGHGLPGGL(D) | | 2 | 1.98 | 0.24 | 508.80 | 1015.59 |
| 236 | 248 | (F)VIPGHGLPGGLDL(L) | | 2 | 2.70 | 0.44 | 622.86 | 1243.70 |
| 236 | 250 | (F) VIPGHGLPGGLDLLK(H) | | 2 | 3.04 | 0.51 | 743.45 | 1484.89 |
| 237 | 250 | (V)IPGHGLPGGLDLLK(H) | | 2 | 3.93 | 0.62 | 693.91 | 1385.80 |
| 238 | 250 | (I)PGHGLPGGLDLLK(H) | | 2 | 4.71 | 0.68 | 637.37 | 1272.72 |
| 241 | 250 | (H)GLPGGLDLLK(H) | | 2 | 1.80 | 0.17 | 491.80 | 981.59 |
| 243 | 250 | (L)PGGLDLLK(H) | | 2 | 2.19 | 0.32 | 406.75 | 811.48 |
| 251 | 257 | (K)HTTNVVK(A) | | 2 | 2.44 | 0.38 | 399.73 | 797.44 |
| 251 | 262 | (K)HTTNVVKAHTNR(S) | | 3 | 2.09 | 0.28 | 459.92 | 1376.74 |
| 251 | 266 | (K)HTTNVVKAHTNRSVVE(-) | | 3 | 2.25 | 0.33 | 597.99 | 1790.95 |

**[Table 7] Information on position and sequence of each peptide identified as GES protein**

| **Start** | **Stop** | **Sequence** | **Oxidati on site** | **Charg e (z)** | **SEQUE ST XCorr** | **SEQUE ST deltaCn** | **Observe d m/z** | **Actual mass value (Da)** |
|---|---|---|---|---|---|---|---|---|
| 19 | 25 | (A)SEKLTFK(T) | | 2 | 2.70 | 0.45 | 426.75 | 851.48 |
| 22 | 30 | (K)L TFKTDLEK(L) | | 3 | 2.62 | 0.43 | 365.54 | 1093.61 |
| 22 | 33 | (K)L TFKTDLEKLER(E) | | 3 | 4.22 | 0.64 | 498.29 | 1491.84 |
| 26 | 33 | (K)TDLEKLER(E) | | 2 | 3.65 | 0.59 | 502.28 | 1002.54 |
| 26 | 35 | (K)TDLEKLEREK(A) | | 3 | 2.55 | 0.41 | 420.90 | 1259.68 |
| 27 | 33 | (T)DLEKLER(E) | | 2 | 2.31 | 0.35 | 451.75 | 901.49 |
| 34 | 55 | (R)EKAAQIGVAIVDPQGEIVAGHR( M) | | 4 | 7.82 | 0.81 | 565.31 | 2257.21 |
| 36 | 55 | (K)AAQIGVAIVDPQGEIVAGHR(M) | | 2 | 6.48 | 0.77 | 1001.05 | 2000.08 |
| 38 | 55 | (A)QIGVAIVDPQGEIVAGHR(M) | | 3 | 3.42 | 0.56 | 620.34 | 1858.01 |
| 39 | 55 | (Q)IGVAIVDPQGEIVAGHR(M) | | 2 | 3.98 | 0.62 | 865.98 | 1729.94 |
| 40 | 55 | (I)GVAIVDPQGEIVAGHR(M) | | 2 | 2.90 | 0.48 | 809.44 | 1616.86 |
| 41 | 55 | (G)VAIVDPQGEIVAGHR(M) | | 3 | 4.05 | 0.63 | 520.95 | 1559.84 |
| 42 | 55 | (V)AIVDPQGEIVAGHR(M) | | 2 | 4.01 | 0.63 | 731.40 | 1460.78 |
| 43 | 55 | (A)IVDPQGEIVAGHR(M) | | 3 | 3.70 | 0.59 | 464.25 | 1389.73 |
| 44 | 55 | (I) VDPQGEIVAGHR(M) | | 2 | 2.44 | 0.38 | 639.33 | 1276.65 |
| 45 | 55 | (V)DPQGEIVAGHR(M) | | 2 | 2.91 | 0.48 | 589.80 | 1177.58 |
| 46 | 55 | (D)PQGEIVAGHR(M) | | 2 | 3.02 | 0.50 | 532.28 | 1062.56 |
| 60 | 67 | (R)FAmcSTFK(F) | M (62) | 2 | 2.60 | 0.42 | 504.22 | 1006.43 |
| 60 | 67 | (R)FAMcSTFK(F) | | 2 | 2.15 | 0.30 | 496.22 | 990.44 |
| 60 | 77 | (R)FAmcSTFKFPLAALVFER(I) | M (62) | 3 | 2.17 | 0.31 | 717.70 | 2150.08 |
| 61 | 67 | (F)AMcSTFK(F) | | 1 | 1.54 | 0.03 | 844.37 | 843.36 |
| 68 | 77 | (K)FPLAAL VFER(I) | | 2 | 3.43 | 0.56 | 581.84 | 1161.66 |
| 70 | 77 | (P)LAALVFER(I) | | 2 | 1.94 | 0.23 | 459.78 | 917.54 |
| 71 | 77 | (L)AALVFER(I) | | 2 | 2.40 | 0.37 | 403.23 | 804.44 |
| 78 | 84 | (R)IDSGTER(G) | | 2 | 2.17 | 0.31 | 389.19 | 776.37 |
| 78 | 87 | (R)IDSGTERGDR(K) | | 3 | 2.03 | 0.26 | 369.18 | 1104.52 |
| 88 | 105 | (R)KLSYGPDmIVEWSPATER(F) | M (95) | 3 | 4.89 | 0.69 | 699.01 | 2094.01 |
| 88 | 105 | (R)KLSYGPDMIVEWSPATER(F) | | 3 | 3.35 | 0.55 | 693.68 | 2078.01 |
| 89 | 105 | (K)LSYGPDmIVEWSPATER(F) | M (95) | 2 | 4.26 | 0.65 | 983.97 | 1965.92 |
| 89 | 105 | (K)LSYGPDMIVEWSPATER(F) | | 3 | 2.26 | 0.34 | 650.98 | 1949.91 |
| 106 | 118 | (R)FLASGHmTVLEAA(Q) | M (112) | 2 | 2.65 | 0.43 | 681.84 | 1361.66 |
| 106 | 118 | (R)FLASGHMTVLEAA(Q) | | 2 | 2.68 | 0.44 | 673.84 | 1345.67 |
| 106 | 120 | (R)FLASGHmTVLEAAQA(A) | M (112) | 3 | 3.34 | 0.55 | 521.26 | 1560.76 |
| 106 | 120 | (R)FLASGHMTVLEAAQA(A) | | 3 | 3.39 | 0.56 | 515.93 | 1544.76 |
| 106 | 121 | (R)FLASGHmTVLEAAQAA(V) | M (112) | 3 | 4.55 | 0.67 | 544.94 | 1631.80 |
| 106 | 121 | (R)FLASGHMTVLEAAQAA(V) | | 3 | 2.84 | 0.47 | 539.61 | 1615.80 |
| 106 | 123 | (R)FLASGHmTVLEAAQAAVQ(L) | M (112) | 3 | 3.89 | 0.61 | 620.65 | 1858.93 |
| 106 | 123 | (R)FLASGHMTVLEAAQAAVQ(L) | | 3 | 2.07 | 0.28 | 615.32 | 1842.93 |
| 106 | 135 | (R)FLASGHmTVLEAAQAAVQLSDN GATNLLLR(E) | M (112) | 3 | 3.22 | 0.53 | 1043.21 | 3126.59 |
| 106 | 135 | (R)FLASGHMTVLEAAQAAVQLSDN GATNLLLR(E) | | 3 | 2.64 | 0.43 | 1037.87 | 3110.60 |
| 110 | 135 | (S)GHmTVLEAAQAAVQLSDNGATN LLLR(E) | M (112) | 3 | 3.49 | 0.57 | 903.80 | 2708.39 |
| 110 | 135 | (S)GHMTVLEAAQAAVQLSDNGATN LLLR(E) | | 3 | 4.30 | 0.65 | 898.47 | 2692.39 |
| 116 | 135 | (L)EAAQAAVQLSDNGATNLLLR(E) | | 3 | 2.95 | 0.49 | 685.70 | 2054.07 |
| 119 | 135 | (A)QAAVQLSDNGATNLLLR(E) | | 3 | 4.24 | 0.65 | 595.33 | 1782.96 |
| 121 | 135 | (A)AVQLSDNGATNLLLR(E) | | 3 | 3.96 | 0.62 | 528.96 | 1583.86 |
| 122 | 135 | (A)VQLSDNGATNLLLR(E) | | 3 | 3.38 | 0.56 | 505.28 | 1512.82 |
| 124 | 135 | (Q)LSDNGATNLLLR(E) | | 3 | 2.56 | 0.42 | 429.57 | 1285.70 |
| 125 | 135 | (L) SDNGATNLLLR(E) | | 2 | 3.40 | 0.56 | 587.32 | 1172.62 |
| 128 | 135 | (N)GATNLLLR(E) | | 2 | 2.09 | 0.28 | 429.26 | 856.51 |
| 136 | 148 | (R)EIGGP AAmTQYFR(K) | M (143) | 2 | 3.76 | 0.60 | 728.85 | 1455.68 |
| 136 | 148 | (R)EIGGP AAMTQYFR(K) | | 2 | 3.68 | 0.59 | 720.85 | 1439.69 |
| 136 | 149 | (R)EIGGP AAmTQYFRK(I) | M (143) | 2 | 3.26 | 0.54 | 792.90 | 1583.78 |
| 138 | 148 | (I)GGPAAmTQYFR(K) | M (143) | 2 | 2.28 | 0.34 | 607.78 | 1213.55 |
| 149 | 156 | (R)KIGDSVSR(L) | | 2 | 2.47 | 0.39 | 431.24 | 860.48 |
| 149 | 159 | (R)KIGDSVSRLDR(K) | | 3 | 2.38 | 0.37 | 415.90 | 1244.69 |
| 150 | 159 | (K)IGDSVSRLDR(K) | | 2 | 1.90 | 0.21 | 559.30 | 1116.59 |
| 157 | 173 | (R)LDRKEPEmSDNTPGDLR(D) | M (164) | 3 | 5.69 | 0.74 | 663.65 | 1987.93 |
| 157 | 173 | (R)LDRKEPEMSDNTPGDLR(D) | | 3 | 6.08 | 0.75 | 658.32 | 1971.93 |
| 160 | 173 | (R)KEPEmSDNTPGDLR(D) | M (164) | 3 | 4.63 | 0.68 | 535.58 | 1603.73 |
| 160 | 173 | (R)KEPEMSDNTPGDLR(D) | | 2 | 3.84 | 0.61 | 794.87 | 1587.72 |
| 160 | 181 | (R)KEPEmSDNTPGDLRDTTTPIAM(A ) | M (164) | 3 | 2.39 | 0.37 | 812.37 | 2434.09 |
| 160 | 181 | (R)KEPEmSDNTPGDLRDTTTPIAm(A ) | M (164), M (181) | 3 | 1.82 | 0.18 | 817.70 | 2450.09 |
| 160 | 183 | (R)KEPEmSDNTPGDLRDTTTPIAMA R(T) | M (164) | 4 | 5.52 | 0.73 | 666.32 | 2661.25 |
| 160 | 183 | (R)KEPEmSDNTPGDLRDTTTPIAmA R(T) | M (164), M (181) | 4 | 5.33 | 0.72 | 670.32 | 2677.24 |
| 160 | 183 | (R)KEPEMSDNTPGDLRDTTTPIAMA R(T) | | 3 | 4.74 | 0.68 | 882.76 | 2645.24 |
| 161 | 173 | (K)EPEmSDNTPGDLR(D) | M (164) | 2 | 3.35 | 0.55 | 738.82 | 1475.63 |
| 161 | 173 | (K)EPEMSDNTPGDLR(D) | | 2 | 2.84 | 0.47 | 730.82 | 1459.62 |
| 161 | 181 | (K)EPEmSDNTPGDLRDTTTPIAM(A) | M (164) | 3 | 1.99 | 0.24 | 769.67 | 2306.00 |
| 161 | 183 | (K)EPEMSDNTPGDLRDTTTPIAmAR( T) | M (181) | 3 | 4.07 | 0.63 | 845.39 | 2533.14 |
| 161 | 183 | (K)EPEmSDNTPGDLRDTTTPIAmAR( T) | M (164), M (181) | 4 | 3.75 | 0.60 | 638.29 | 2549.14 |
| 161 | 183 | (K)EPEMSDNTPGDLRDTTTPIAMAR( | | 3 | 3.93 | 0.62 | 840.06 | 2517.15 |
| | | T) | | | | | | |
| 174 | 183 | (R)DTTTPIAmAR(T) | M (181) | 2 | 2.49 | 0.40 | 546.77 | 1091.52 |
| 174 | 183 | (R)DTTTPIAMAR(T) | | 2 | 2.35 | 0.36 | 538.78 | 1075.54 |
| 184 | 204 | (R)TVAKVLYGGALTSTSTHTIER(W) | | 3 | 3.36 | 0.55 | 735.73 | 2204.18 |
| 188 | 196 | (K)VLYGGALTS(T) | | 1 | 2.08 | 0.28 | 880.48 | 879.47 |
| 188 | 197 | (K)VLYGGALTST(S) | | 1 | 2.23 | 0.33 | 981.52 | 980.52 |
| 188 | 200 | (K)VLYGGALTSTSTH(T) | | 2 | 2.92 | 0.49 | 653.83 | 1305.65 |
| 188 | 204 | (K)VLYGGALTSTSTHTIER(W) | | 3 | 5.96 | 0.75 | 602.66 | 1804.95 |
| 188 | 217 | (K)VLYGGALTSTSTHTIERWLIGNQ TGDATLR(A) | | 4 | 2.09 | 0.28 | 808.67 | 3230.66 |
| 189 | 204 | (V)LYGGALTSTSTHTIER(W) | | 2 | 3.77 | 0.60 | 853.94 | 1705.86 |
| 190 | 204 | (L)YGGALTSTSTHTIER(W) | | 3 | 4.90 | 0.69 | 531.93 | 1592.77 |
| 191 | 204 | (Y)GGALTSTSTHTIER(W) | | 2 | 4.04 | 0.63 | 715.87 | 1429.73 |
| 192 | 204 | (G)GALTSTSTHTIER(W) | | 2 | 4.06 | 0.63 | 687.35 | 1372.68 |
| 193 | 204 | (G)ALTSTSTHTIER(W) | | 3 | 1.81 | 0.17 | 439.56 | 1315.67 |
| 195 | 204 | (L)TSTSTHTIER(W) | | 2 | 2.43 | 0.38 | 566.78 | 1131.55 |
| 205 | 217 | (R)WLIGNQTGDATLR(A) | | 3 | 3.79 | 0.60 | 482.26 | 1443.74 |
| 206 | 217 | (W)LIGNQTGDATLR(A) | | 2 | 2.56 | 0.41 | 629.84 | 1257.67 |
| 218 | 229 | (R)AGFPKDWVVGEK(T) | | 3 | 3.90 | 0.62 | 444.90 | 1331.69 |
| 219 | 229 | (A)GFPKDWVVGEK(T) | | 2 | 2.45 | 0.39 | 631.33 | 1260.65 |
| 220 | 229 | (G)FPKDWVVGEK(T) | | 2 | 2.70 | 0.44 | 602.82 | 1203.63 |
| 221 | 229 | (F)PKDWVVGEK(T) | | 2 | 3.42 | 0.56 | 529.29 | 1056.56 |
| 223 | 229 | (K)DWVVGEK(T) | | 2 | 1.96 | 0.23 | 416.72 | 831.42 |
| 230 | 245 | (K)TGTcANGGRNDIGFFK(A) | | 3 | 2.49 | 0.40 | 572.28 | 1713.81 |
| 239 | 245 | (R)NDIGFFK(A) | | 2 | 2.68 | 0.44 | 420.72 | 839.42 |
| 239 | 249 | (R)NDIGFFKAQER(D) | | 2 | 2.34 | 0.36 | 662.84 | 1323.67 |
| 246 | 256 | (K)AQERDYAVAVY(T) | | 2 | 1.83 | 0.18 | 642.81 | 1283.61 |
| 246 | 261 | (K)AQERDYAVAVYTTAPK(L) | | 3 | 4.80 | 0.69 | 594.98 | 1781.91 |
| 250 | 261 | (R)DYAVAVYTTAPK(L) | | 2 | 3.62 | 0.59 | 649.84 | 1297.66 |
| 262 | 272 | (K)LSA VERDEL VA(S) | | 2 | 2.48 | 0.39 | 601.32 | 1200.63 |
| 262 | 275 | (K)LSAVERDELVASVG(Q) | | 2 | 2.56 | 0.41 | 722.88 | 1443.75 |
| 262 | 276 | (K)LSAVERDELVASVGQ(V) | | 3 | 1.99 | 0.25 | 524.94 | 1571.81 |
| 262 | 279 | (K)LSAVERDELVASVGQVIT(Q) | | 3 | 1.78 | 0.16 | 629.34 | 1885.01 |
| 262 | 280 | (K)LSAVERDELVASVGQVITQ(L) | | 3 | 3.75 | 0.60 | 672.03 | 2013.07 |
| 262 | 281 | (K)LSAVERDELVASVGQVITQL(I) | | 3 | 1.76 | 0.15 | 709.73 | 2126.16 |
| 262 | 287 | (K)LSAVERDELVASVGQVITQLILST DK(-) | | 4 | 4.56 | 0.67 | 696.89 | 2783.53 |
| 273 | 287 | (A)SVGQVITQLILSTDK(-) | | 2 | 2.98 | 0.50 | 801.46 | 1600.90 |
| 274 | 287 | (S)VGQVITQLILSTDK(-) | | 3 | 2.82 | 0.47 | 505.63 | 1513.87 |
| 276 | 287 | (G)QVITQLILSTDK(-) | | 2 | 2.76 | 0.46 | 679.90 | 1357.78 |
| 277 | 287 | (Q)VITQLILSTDK(-) | | 2 | 3.08 | 0.51 | 615.87 | 1229.72 |
| 280 | 287 | (T)QLILSTDK(-) | | 2 | 1.80 | 0.17 | 459.27 | 916.52 |
| 281 | 287 | (Q)LILSTDK(-) | | 1 | 2.28 | 0.34 | 789.47 | 788.46 |

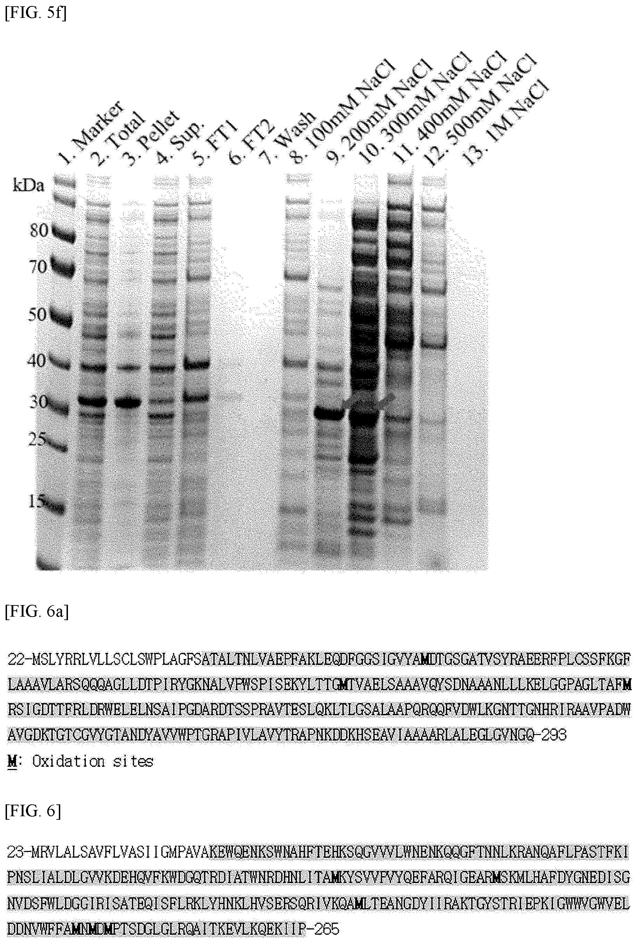

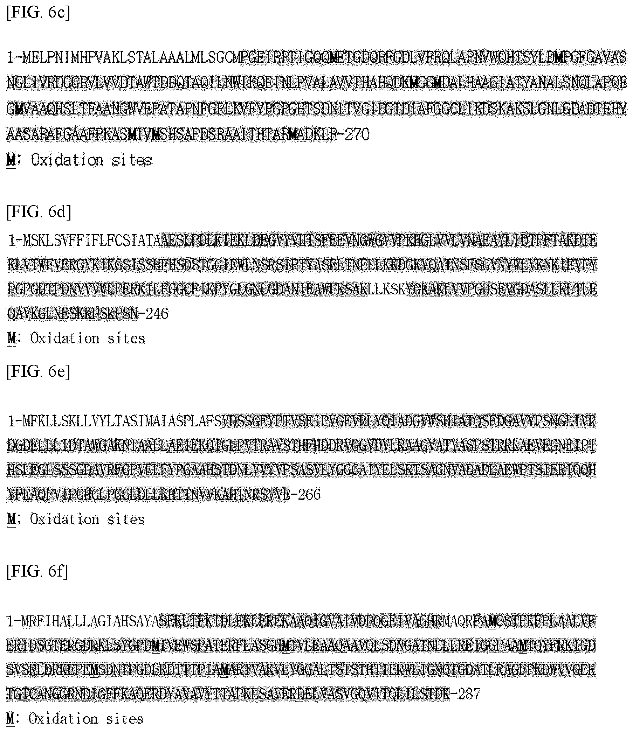

For the KPC protein, a peptide in which one methionine among three methionine residues (49, 116 and 151) was in an oxidized form was identified (FIG. 6a). For OXA, a peptide in which one methionine or two or three methionine residues among six methionine residues (115, 138, 195, 237, 239 and 241) were in an oxidized form was identified (FIG. 6b).

Specifically, for the KPC protein, an N-terminal sequence peptide consisting of residues 1-21 was not detected, and for the OXA protein, an N-terminal sequence peptide consisting of residues 1-22 was not detected. For this KPC, when the sequence except residues 1-21 was considered, peptides covering the entire sequence were identified, and the sequence coverage increased from 92.8% (272/293) to 100% (272/272). For OXA, when the sequence except residues 1-22 was considered, the sequence coverage increased from 91.7% (243/265) to 100% (243/243).

For the NDM protein, a peptide in which one methionine among seven methionine residues (39, 67, 126, 129, 245, 248 and 265) was in an oxidized form was identified (FIG. 6e). For GES, a peptide in which one methionine or two methionine residues among six methionine residues (62, 95, 112, 143, 164 and 181) were in an oxidized form was identified (FIG. 6f). For IMP and VIM, a peptide in which methionine was in an oxidized form was not identified (FIGS. 6d and 6e).

For the IMP and VIM proteins, N-terminal sequence peptides consisting of residues 1-18 and residues 1-26, respectively, were not detected, and for GES, an N-terminal sequence peptide consisting of residues 1-18 was not detected. When the target protein sequences excluding the N-terminus were considered, the identified sequence coverages were 97.2 (243/250) for NDM, 97.8% (223/228) for IMP, 100% (240/240) for VIM, and 98.5% (265/269) for GES.

(3)Identification of N-Terminal sequences (FIG. 7)

### KPC

- N-terminal sequence: (S)/ATALTNLVAEPFAK(L) (semi-tryptic)

### KPC-3

- N-terminal sequence: (S)/ATALTNLVAEPFAK(L) (semi-tryptic)

### KPC-17

- N-terminal sequence: (S)/ATALTNLVAEPFAK(L) (semi-tryptic)

### OXA

- N-terminal sequence: (A)/KEWQENK(S) (semi-tryptic)

### OXA-181

- N-terminal sequence: (A)/KEWQENKSWNAHFTEHK(S) (semi-tryptic)

### NDM

- N-terminal sequence: (M)PGEIRPTIGQQMETGDQR(F) (semi-tryptic)

### IMP

- N-terminal sequence: (A)AESLPDLK(I) (semi-tryptic)

### IMP-1

- N-terminal sequence: (A)/AESLPDLK(I) (semi-tryptic)

### IMP-4

- N-terminal sequence: (A)/AESLPDLK(I) (semi-tryptic)

### VIM

- N-terminal sequence: (S)/VDSSGEYPTVSEIPVGEVR(L) (semi-tryptic)

### VIM-1

- N-terminal sequence: (S)/GEPSGEYPTVNEIPVGEVR(L) (semi-tryptic)

### VIM-4

- N-terminal sequence: (S)/GEPSGEYPTVNEIPVGEVR(L) (semi-tryptic)

### GES

- N-terminal sequence: (A)SEKLTFK(T) (semi-tryptic)

### GES-1

- N-terminal sequence: (A)/SEKLTFK(T) (semi-tryptic)

### Example 6: Amino acid sequencing and characterization of each protein

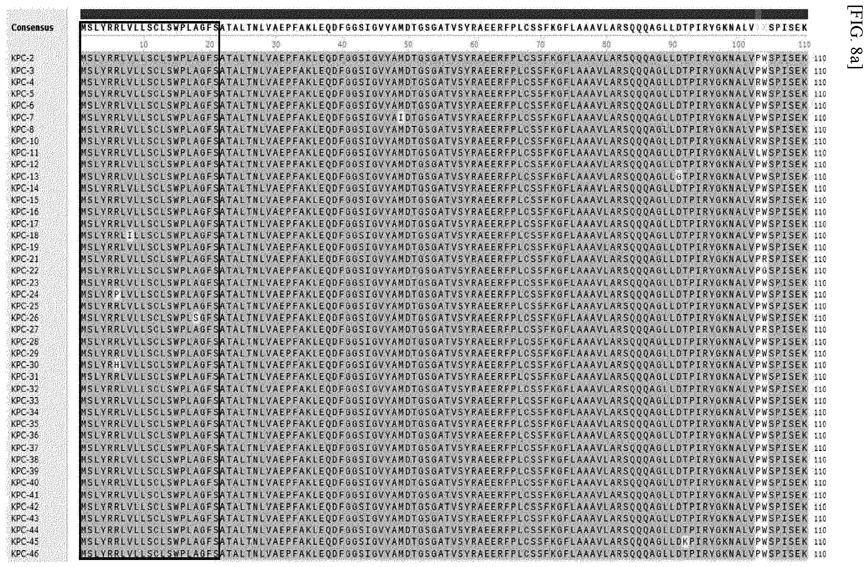

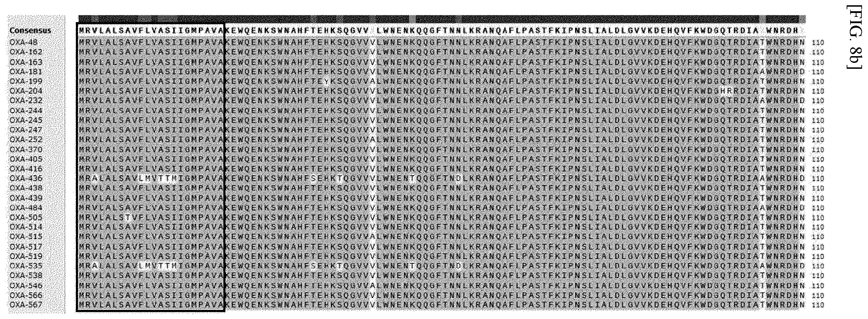

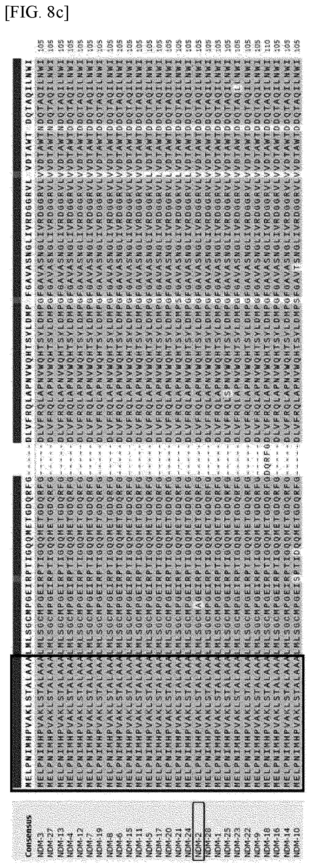

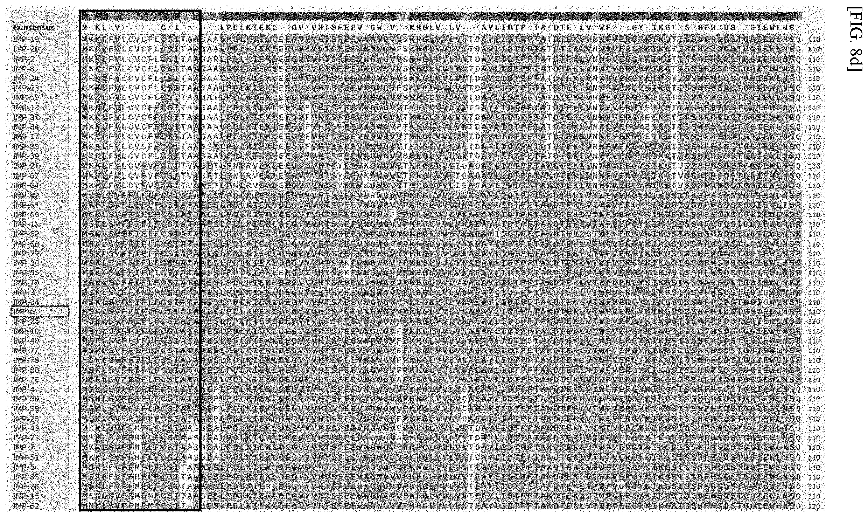

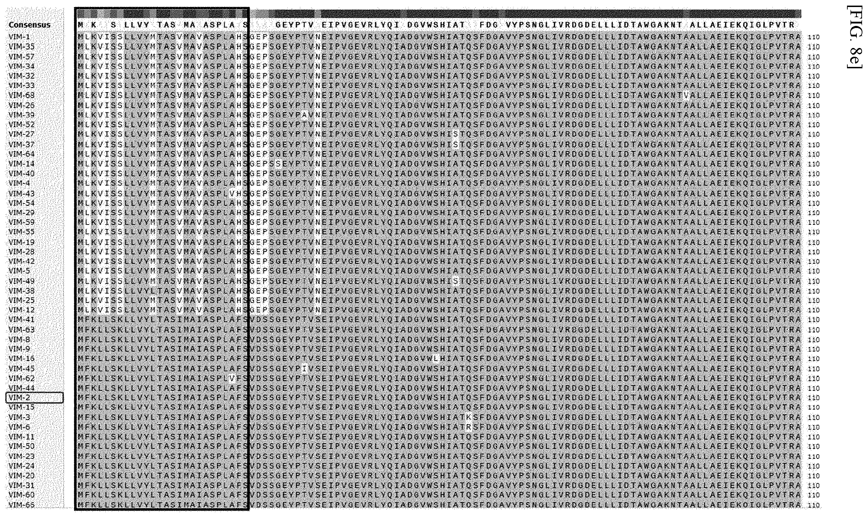

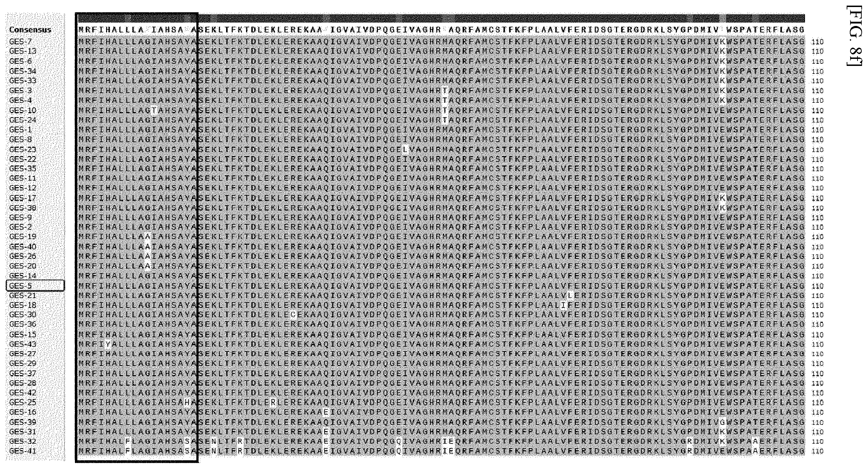

Based on the MS2 results obtained in Example 5, multiple alignment analysis was performed on a total of 43 KPC subtype proteins (FIG. 8a), 660 OXA subtype proteins (FIG. 8b), 27 NDM subtype proteins (FIG. 8c), 79 IMP subtype proteins (FIG. 8d), 66 VIM subtype proteins (FIG. 8e) and 43 GES subtype proteins (FIG. 8f) known to date in the National Center for Biotechnology Information (NCBI) database.

### KPC and OXA proteins

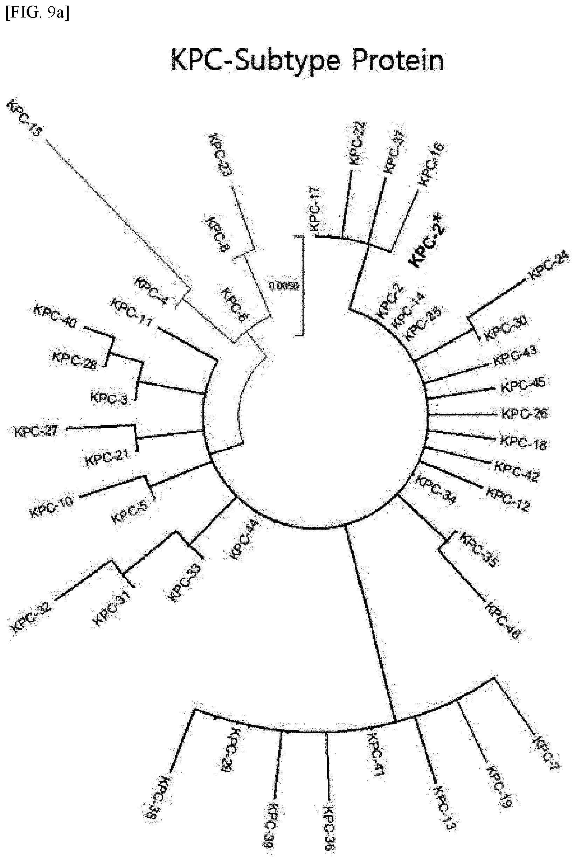

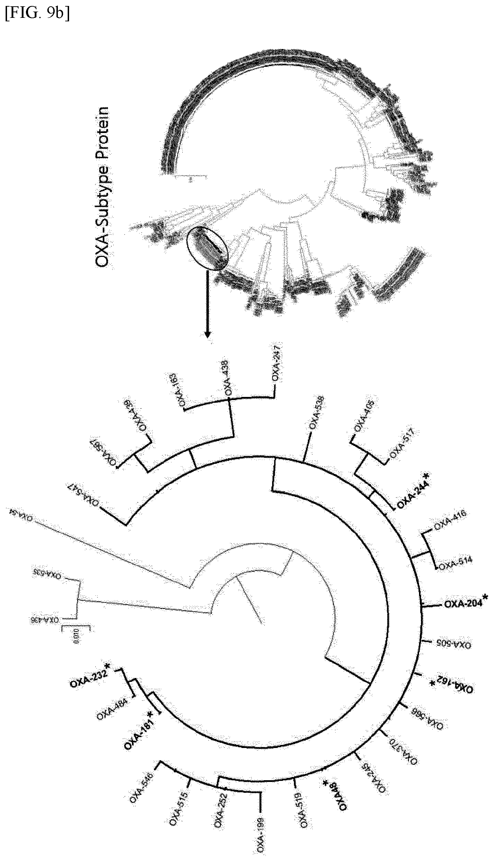

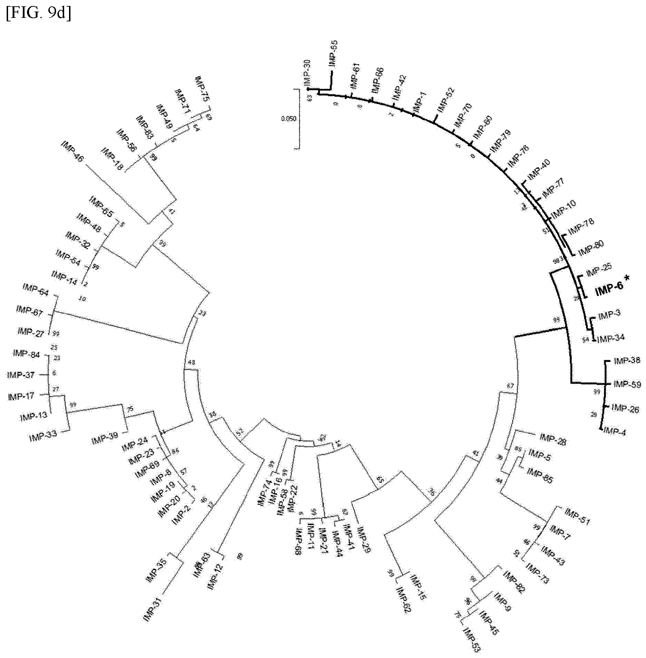

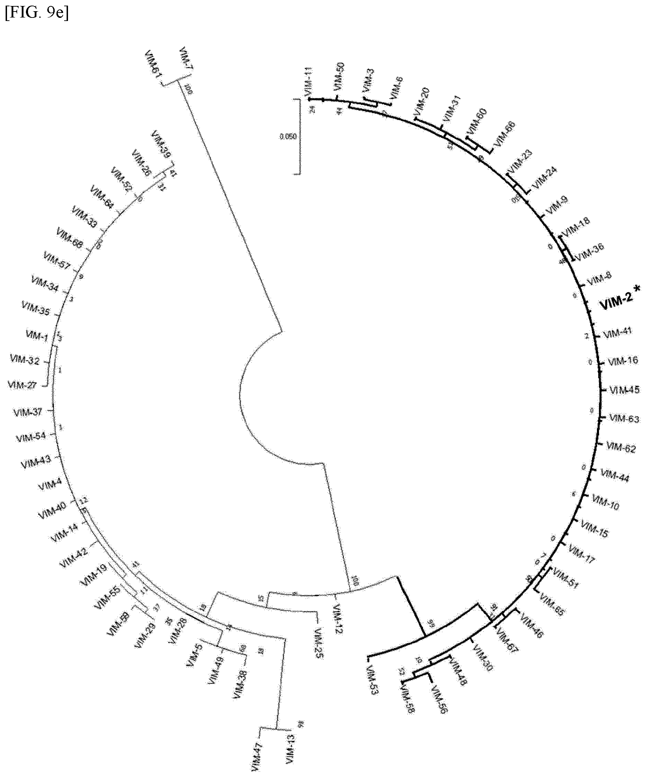

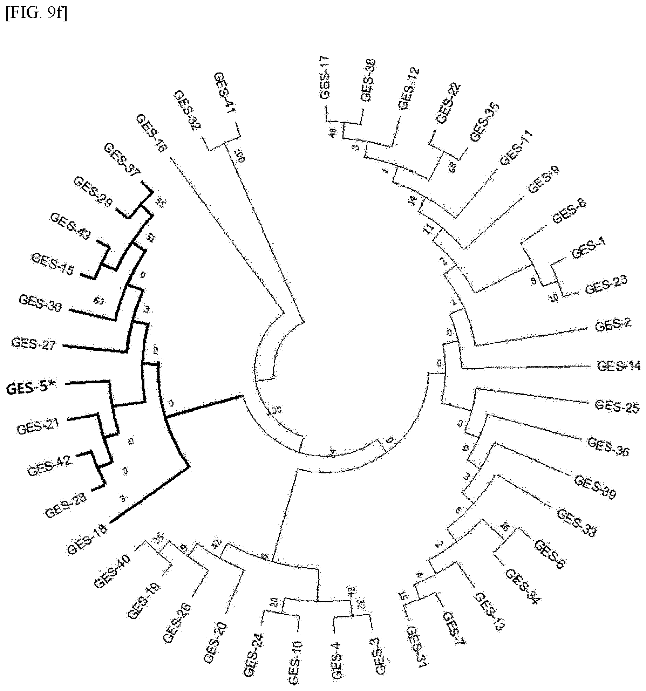

It was confirmed that 97.3% or more of the full-length amino acid sequences of 42 proteins including KPC-2 are conserved, and for the OXA protein, 91.3% or more of the full-length amino acid sequences of 30 proteins including OXA-48 having resistance to carbapenem antibiotics are conversed. In order to identify the characteristics of each protein, phylogenetic tree analysis of KPC and OXA was performed through the MEGA X program (FIGS. 9a and 9b). Specifically, 43 KPC subtype proteins all contained the same N-terminal peptide (1 to 21 a.a.), and among them, 35 subtype proteins including KPC-2 were each composed of a sequence consisting of 293 amino acids. For OXA, 30 OXA proteins comprising the same N-terminal peptide (1 to 22 a.a.) were identified, and 23 OXA proteins were characterized by the same sequence as OXA-48 composed of a sequence consisting of 265 amino acids.

### MBL proteins and GES protein

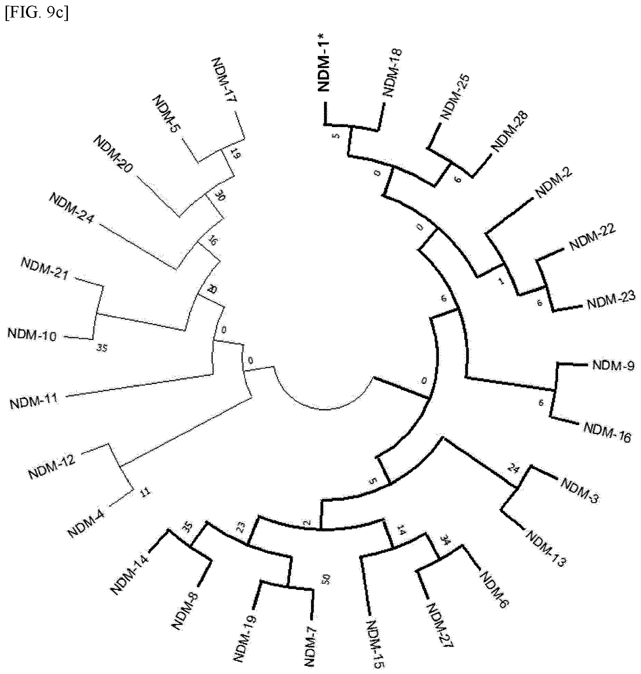

It was confirmed that sequence similarities were 90.9% (NDM), 44.8% (IMP), 63.5% (VIM), and 86.5% (GES). As subtype proteins having the same N-terminus, 27 NDM subtype proteins, 23 IMP subtype proteins (89.4%), 26 VIM subtype proteins (92.1%), and 35 GES subtype proteins (87.9%) were identified. As subtype proteins having the same peptide number as the N-terminal sequence and the same full-length amino acid sequence, 26 NDM subtype proteins (92.6%), 23 IMP subtype proteins (89.4%), 25 VIM subtype proteins (93.6%), and 34 GES subtype proteins (88.9%) were identified. In order to identify the characteristics of each protein, phylogenetic tree analysis was performed on the subtype proteins of the MBL protein and the GES protein through the MEGA X program (FIGS. 9c to 9f). As a result, it could be confirmed that the subgroups each containing NDM-1, IMP-6, VIM-2 and GES-5 were grouped in the phylogenetic tree analysis, like the results confirmed in the multiple alignment analysis.

### Example 7. Target protein identification using mass spectrometry (top-down method)

To determine the mass value of the active protein expressed in the strain, top-down mass spectrometry was performed. To confirm the exact mass value of the protein, a sample obtained by partial purification from the crude protein extract derived from the strain was used, and an LC-MS/MS system (Nano-LC and Q-Exactive HF-X mass spectrometry system) capable of top-down analysis was used.

### (1) Mass spectrometry of purified protein

About 0.5 µg of the partially purified protein sample was injected. Analysis was performed using a direct infusion method without a column and using a nano-flow pump. The sample analysis conditions used in this case are as follows.
- buffer A: 0.1% formic acid in water
- sample analysis: from 0 to 10 min, 100% fixed buffer (A) 100%, 4 µL/min fixed flow rate

### (2) Mass spectrometry of target proteins using high-resolution mass spectrometry

Using the protein mode analysis method with the Q-Exactive HF-X mass spectrometer, the mass values of the intact proteins and the tandem mass spectra of the proteins were obtained and identified. The parameters used in this case are as follows.
- Resolution: use of Full MS 120,000, MS2 60,000 or 120,000
- Full MS: 620 to 2,400 m/z, 100 msec
- MS2: use of 1 or 2 microscans, 1,000 msec, NCE 50; ionized materials with a charge state of 1 to 8 were excluded from MS2 analysis.

Software for identifying proteins based on top-down data, 'Informed Proteomics' developed by US PNNL (Pacific Northwest National Laboratory) was used.

### KPC and OXA proteins

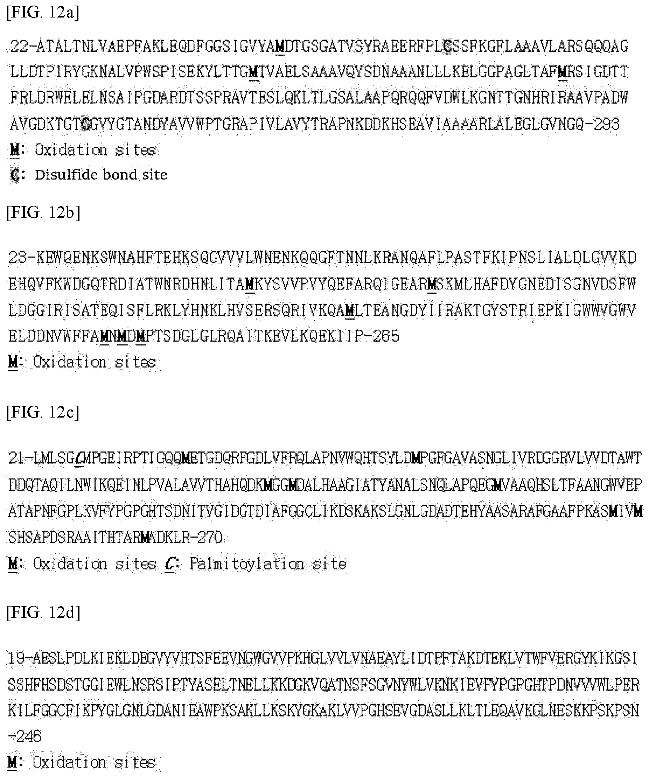

Several multi-charged KPC protein (z = +13 to +20) or OXA protein (z = +22 to +37) peaks appeared (FIGS. 10a and 10b), and it was confirmed that a representative mass value obtained by deconvolution of the peaks was an average molecular weight of 28,718.13 m/z × z, corresponding to a monoisotopic mass of 28,700.69 m/z × z. The confirmed representative mass value represents the mass value of the KPC protein in a state in which there is a disulfide bond between Cys68 and Cys237. In addition, other peaks caused by methionine oxidation were observed (e.g., polypeptides with one oxidized methionine show an average molecular weight of 28,718 + 16 m/z × z). They partially coincided with the positions of oxidized methionine residues (49, 116 and 151 for KPC, and 115, 138, 195, 237, 239 and 241 for OXA) within the KPC or OXA protein sequence obtained through in-gel digestion (bottom-up method). Polypeptides including the N-terminal sequence consisting of residues 1-21 for KPC or residues 1-22 for OXA were not observed even in the analysis based on the top-down method. When each N-terminal sequence was excluded, both KPC (22-293 a.a.) and OXA (23-265 a.a.) showed a sequence coverage of 100% (FIGS. 12a and 12b).

### MBL proteins and GES protein

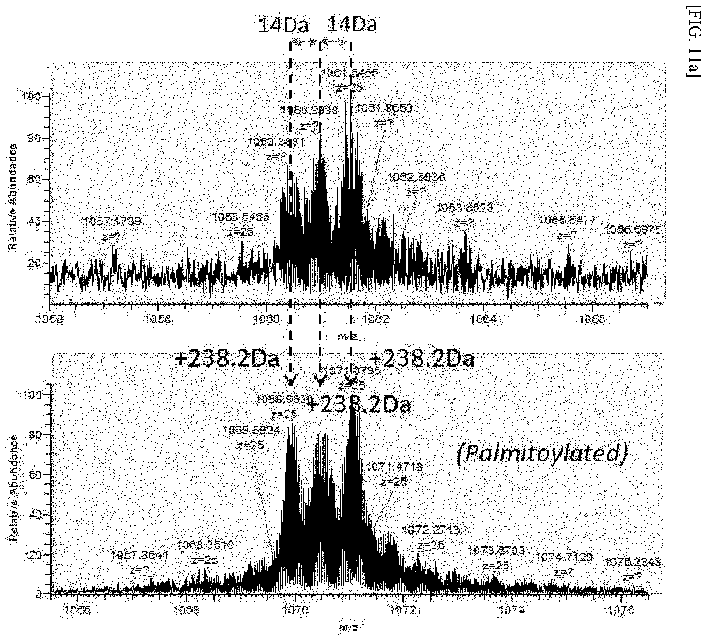

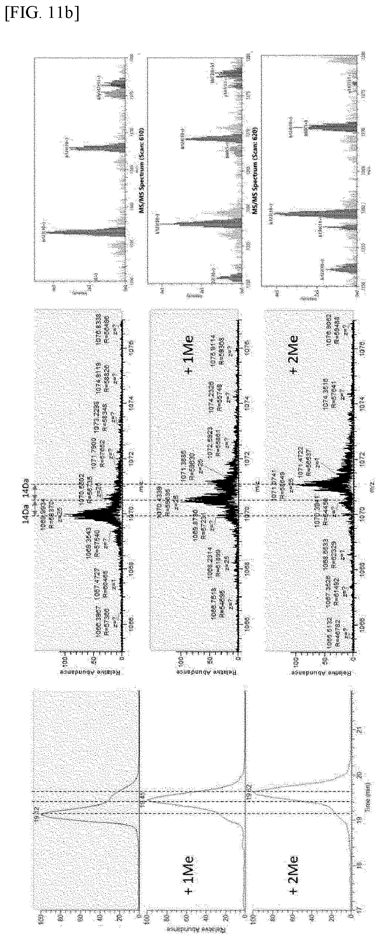

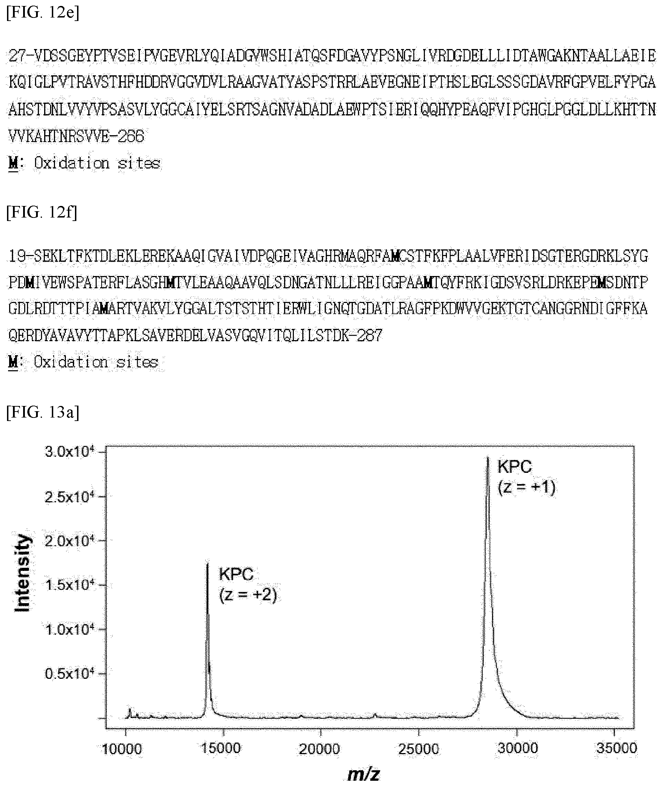

Several peaks of multi-charged MBL proteins or GES protein appeared (FIGS. 10c, 10e, 10g and 10i), and it was confirmed that, for NDM, a representative mass value obtained by deconvolution of the peaks was an average molecular weight of 26,724 m/z × z, corresponding to a monoisotopic mass of 26,707 m/z × z. It was confirmed that the average molecular weight for IMP was 25082.68 m/z × z, corresponding to a monoisotopic mass of 25,067.19 m/z × z, the average molecular weight for VIM was 25,515.42 m/z × z, corresponding to a monoisotopic mass of 25,499.92 m/z × z, and the average molecular weight for GES was 29,245.15 m/z × z, corresponding to a monoisotopic mass of 29,226.92 m/z × z. It was confirmed that the identified representative mass value for NDM corresponded to a palmitoylated protein type (FIG. 11a). In addition, other peaks caused by methionine oxidation and methylation were observed (for example, polypeptides with one oxidized methionine show an average molecular weight of 26724 + 16 m/z × z, and polypeptides with one methylation show an average molecular weight of 26724 + 14 m/z × z) (FIG. 11b). On the other hand, for IMP and VIM, no specific protein modification was found. For GES, a disulfide bond between Cys63 and Cys233 was found, and other peaks caused by methionine oxidation were also observed. They partially coincided with the positions of oxidized methionine residues (39, 67, 126, 129, 245, 248 and 265 for NDM, and 62, 95, 112, 143, 164, 181 for GES) within the NDM or GES protein sequence obtained through in-gel digestion (bottom-up method). Polypeptides including the N-terminal sequence consisting of residues 1-19 or 1-20 for NDM, residues 1-18 for IMP, residues 1-26 for VIM, or residues 1-18 for GES were not observed even in the analysis based on the top-down method. When each N-terminal sequence was excluded, sequence coverage of 100% was identified in residues 21-270 for NDM protein (FIG. 12c), residues 19-246 for IMP protein (FIG. 12d), and residues 27-266 for VIM protein (FIG. 12e). In addition, for GES protein, sequence coverage of 100% was identified in residues 19-287 (FIG. 12f).

### (3) Mass spectrometry of target proteins using low-resolution mass spectrometry

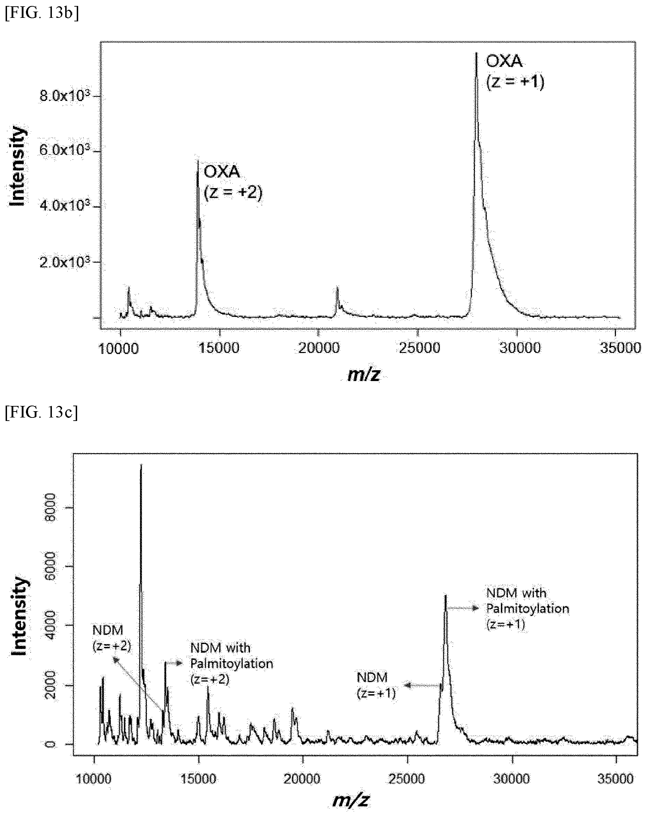

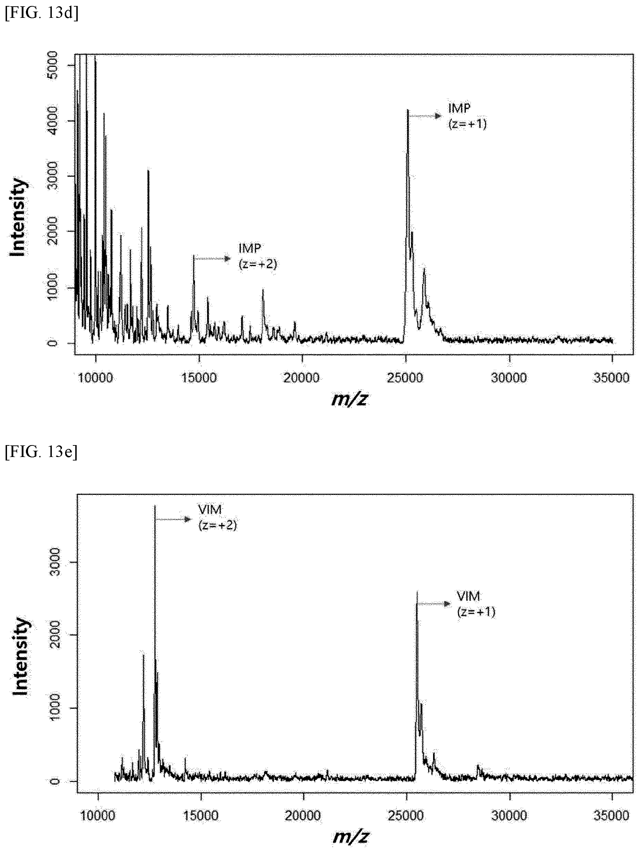

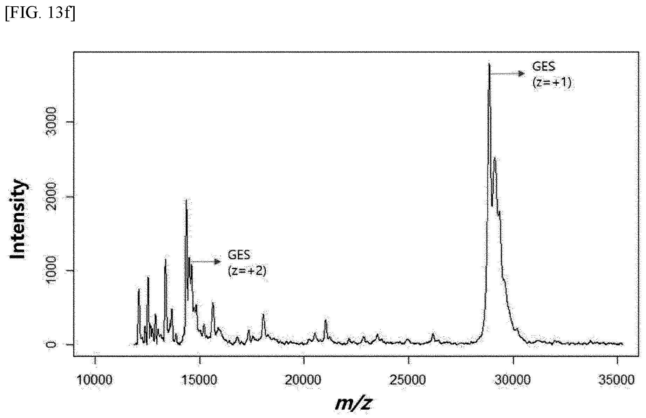

The mass spectrometry spectrum of each protein was obtained using a low-resolution mass spectrometer (1, SciEX 4800; 2, Bruker Biotyper MALDI-TOF MS system). First, 1 µL of a sinapinic acid (SA, present at 10 mg/mL in 0.1% TFA/50% acetonitrile) matrix and about 100 ng of each protein were placed on the plate spot, dried completely, and subjected to mass spectrometry. As protein samples, all of the colonies cultured in solid culture, cells harvested in liquid culture, crude extract and crude enzyme solution after cell lysis, and purified proteins obtained after purification can be used. In this case, the maximum energy used was 30%, random position acquisition was performed, a total of 2,000 laser shots (40 shots per time) were irradiated, and each spectral data was cumulatively obtained. Mass spectrometry spectra were obtained for the range of 10,000 to 40,000 m/z (KPC and OXA) or 35,000 m/z (MBL and GES), and each target protein with a charge state of +1 as well as each target protein with a charge state of +2 were simultaneously detected (FIG. 13). As a result of low-resolution mass spectrometry, both charge states of +1 and +2 were detected for all the three MBL proteins and the GES protein. In particular, for the NDM protein which is a protein anchored to the cell membrane, both a molecular weight corresponding to palmitoylation (average mass = 238.4136, monoisotopic mass = 238.22966) and a molecular weight corresponding to non-palmitoylation were identified.

### (4) Comparison of mass values of protein subtypes

The exact mass values of the KPC, OXA, MBL and GES proteins were confirmed through the above-described method, and the mass values of the active proteins from which the N-terminal peptide has been removed could be confirmed based on the confirmed mass values. Thus, for all the KPC, OXA, MBL and GES proteins found in the NCBI, the exact mass values of the active proteins can be confirmed through high-resolution or low-resolution mass spectrometry, and it is possible to rapidly and accurately identify various types of subtype proteins through mass spectrometry (Tables 8 to 13).

**[Table 8] Mass data of KPC protein**

| Sub type s | Full-length KPC protein | | | Full-length KPC protein (containing disulfide bond) | | | Active protein | | | Active protein (containing disulfide bond) | | | N-termin us |
|---|---|---|---|---|---|---|---|---|---|---|---|---|---|
| | Da | Average molecular weight | Monoiso topic mass | Da | Average molecular weight | Monois otopic mass | Da | Average molecula r weight | Monois otopic mass | Da | Average molecula r weight | Monois otopic mass | |
| 2 | 31115 | 31115.35 | 31095.9 8 | 311 13 | 31113.33 | 31093. 96 | 2872 0 | 28720.43 | 28702.7 1 | 2871 8 | 28718.41 | 28700.6 9 | 1~ 21aa |
| 3 | 31141 | 31141.39 | 31121.9 9 | 311 39 | 31139.37 | 31119. 97 | 2874 6 | 28746.46 | 28728.7 1 | 2874 4 | 28744.44 | 28726.6 9 | 1~ 21aa |
| 4 | 31132 | 31132.34 | 31112.9 9 | 311 30 | 31130.32 | 31110. 97 | 2873 7 | 28737.42 | 28719.7 1 | 2873 5 | 28735.40 | 28717.6 9 | 1~ 21aa |
| 5 | 31174 | 31174.42 | 31155.0 3 | 311 72 | 31172.40 | 31153. 01 | 2878 0 | 28779.50 | 28761.7 6 | 2877 7 | 28777.48 | 28759.7 4 | 1~ 21aa |
| 6 | 31073 | 31073.27 | 31053.94 | 31071 | 31071.25 | 31051.92 | 28678 | 28678.35 | 28660.66 | 28676 | 28676.33 | 28658.64 | 1~21aa |
| 7 | 31123 | 31123.35 | 31104.0 3 | 311 21 | 31121.33 | 31102. 01 | 2872 8 | 28728.43 | 28710.7 6 | 2872 6 | 28726.41 | 28708.7 4 | 1~ 21aa |
| 8 | 31099 | 31099.30 | 31079.9 4 | 310 97 | 31097.28 | 31077. 92 | 2870 4 | 28704.38 | 28686.6 7 | 2870 2 | 28702.36 | 28684.6 5 | 1~ 21aa |
| 10 | 31200 | 31200.46 | 31181.0 4 | 311 98 | 31198.44 | 31179. 02 | 2880 6 | 28805.53 | 28787.7 6 | 2880 4 | 28803.51 | 28785.7 4 | 1~ 21aa |
| 11 | 31131 | 31131.39 | 31112.0 2 | 311 29 | 31129.37 | 31110. 00 | 2873 6 | 28736.47 | 28718.7 4 | 2873 4 | 28734.45 | 28716.7 2 | 1~ 21aa |
| 12 | 31133 | 31133.38 | 31113.9 4 | 311 31 | 31131.36 | 31111. 92 | 2873 8 | 28738.46 | 28720.6 7 | 2873 6 | 28736.44 | 28718.6 5 | 1~ 21aa |
| 13 | 31083 | 31083.35 | 31063.9 8 | 310 81 | 31081.33 | 31061. 96 | 2868 8 | 28688.43 | 28670.7 1 | 2868 6 | 28686.41 | 28668.6 9 | 1~ 21aa |
| 14 | 30957 | 30957.19 | 30937.9 2 | 309 55 | 30955.17 | 30935. 90 | 2856 2 | 28562.27 | 28544.6 4 | 2856 0 | 28560.25 | 28542.6 2 | 1~ 21aa |
| 15 | 31272 | 31271.58 | 31252.1 1 | 312 70 | 31269.56 | 31250. 09 | 2887 7 | 28876.66 | 28858.8 4 | 2887 5 | 28874.64 | 28856.8 2 | 1~ 21aa |
| 16 | 31071 | 31071.29 | 31051.9 8 | 310 69 | 31069.27 | 31049. 96 | 2867 6 | 28676.37 | 28658.7 0 | 2867 4 | 28674.35 | 28656.6 8 | 1~ 21aa |
| 17 | 31081 | 31081.33 | 31062.0 0 | 310 79 | 31079.31 | 31059. 98 | 2868 6 | 28686.41 | 28668.7 3 | 2868 4 | 28684.39 | 28666.7 1 | 1~ 21aa |
| 18 | 31129 | 31129.38 | 31110.0 0 | 311 27 | 31127.36 | 31107. 98 | 2872 0 | 28720.43 | 28702.7 1 | 2871 8 | 28718.41 | 28700.6 9 | 1~ 21aa |
| 19 | 31128 | 31128.39 | 31108.9 9 | 311 26 | 31126.37 | 31106. 97 | 2873 3 | 28733.47 | 28715.7 2 | 2873 1 | 28731.45 | 28713.7 0 | 1~ 21aa |
| 21 | 31085 | 31085.32 | 31066.0 1 | 310 83 | 31083.30 | 31063. 99 | 2869 0 | 28690.40 | 28179.5 9 | 2868 8 | 28688.38 | 28177.5 7 | 1 ~ 21aa |
| 22 | 30952 | 30952.17 | 30932.9 4 | 309 50 | 30950.15 | 30930. 92 | 2855 7 | 28557.25 | 28539.6 7 | 2855 5 | 28555.23 | 28537.6 5 | 1 ~ 21aa |
| 23 | 31113 | 31113.33 | 31093.9 6 | 311 11 | 31111.31 | 31091. 94 | 2871 8 | 28718.41 | 28700.6 8 | 2871 6 | 28716.39 | 28698.6 6 | 1 ~ 21aa |
| 24 | 31056 | 31056.28 | 31036.9 4 | 310 54 | 31054.26 | 31034. 92 | 2872 0 | 28720.43 | 28702.7 1 | 2871 8 | 28718.41 | 28700.6 9 | 1 ~ 21aa |
| 25 | 31358 | 31357.63 | 31338.1 1 | 313 56 | 31355.61 | 31336. 09 | 2896 3 | 28962.70 | 28944.8 4 | 2896 1 | 28960.68 | 28942.8 2 | 1 ~ 21aa |
| 26 | 31131 | 31131.35 | 31111.9 8 | 311 29 | 31129.33 | 31109. 96 | 2872 0 | 28720.43 | 28702.7 1 | 2871 8 | 28718.41 | 28700.6 9 | 1 ~ 21aa |
| 27 | 31111 | 31111.36 | 31092.0 1 | 311 09 | 31109.34 | 31089. 99 | 2871 6 | 28716.44 | 28698.7 4 | 2871 4 | 28714.42 | 28696.7 2 | 1 ~ 21aa |
| 28 | 30983 | 30983.23 | 30963.9 2 | 309 81 | 30981.21 | 30961. 90 | 2858 8 | 28588.31 | 28570.6 5 | 2858 6 | 28586.29 | 28568.6 3 | 1 ~ 21aa |
| 29 | 31500 | 31499.74 | 31480.1 4 | 314 98 | 31497.72 | 31478. 12 | 2910 5 | 29104.82 | 29086.8 6 | 2910 3 | 29102.80 | 29084.8 4 | 1 ~ 21aa |
| 30 | 31096 | 31096.30 | 31076.9 4 | 310 94 | 31094.28 | 31074. 92 | 2872 0 | 28720.43 | 28702.7 1 | 2871 8 | 28718.41 | 28700.6 9 | 1 ~ 21aa |
| 31 | 31189 | 31189.47 | 31170.0 3 | 311 87 | 31187.45 | 31168. 01 | 2879 5 | 28794.55 | 28776.7 5 | 2879 3 | 28792.53 | 28774.7 3 | 1 ~ 21aa |
| 32 | 31220 | 31219.56 | 31200.0 2 | 312 18 | 31217.54 | 31198. 00 | 2882 5 | 28824.64 | 28806.7 4 | 2882 3 | 28822.62 | 28804.7 2 | 1 ~ 21aa |
| 33 | 31163 | 31163.44 | 31144.02 | 31161 | 31161.42 | 31142.00 | 28769 | 28768.52 | 28750.75 | 28767 | 28766.50 | 28748.73 | 1 ~21aa |
| 34 | 32026 | 32026.29 | 32006.4 0 | 320 24 | 32024.27 | 32004. 38 | 2963 1 | 29631.37 | 29613.1 2 | 2962 9 | 29629.35 | 29611.1 0 | 1 ~ 21aa |
| 35 | 31099 | 31099.31 | 31079.9 5 | 310 97 | 31097.29 | 31077. 93 | 2870 4 | 28704.39 | 28686.6 8 | 2870 2 | 28702.37 | 28684.6 6 | 1 ~ 21aa |
| 36 | 31155 | 31155.41 | 31136.0 0 | 311 53 | 31153.39 | 31133. 98 | 2876 0 | 28760.49 | 28742.7 3 | 2875 8 | 28758.47 | 28740.7 1 | 1 ~ 21aa |
| 37 | 31051 | 31051.31 | 31032.0 2 | 310 49 | 31049.29 | 31030. 00 | 2865 6 | 28656.39 | 28638.7 5 | 2865 4 | 28654.37 | 28636.7 3 | 1 ~ 21aa |
| 38 | 31113 | 31113.33 | 31093.9 6 | 311 11 | 31111.31 | 31091. 94 | 2871 8 | 28718.41 | 28700.6 8 | 2871 6 | 28716.39 | 28698.6 6 | 1 ~ 21aa |
| 39 | 31171 | 31171.41 | 31152.0 0 | 311 69 | 31169.39 | 31149. 98 | 2877 6 | 28776.49 | 28758.7 2 | 2877 4 | 28774.47 | 28756.7 0 | 1 ~ 21aa |
| 40 | 31370 | 31369.63 | 31350.1 0 | 313 68 | 31367.61 | 31348. 08 | 2897 5 | 28974.71 | 28956.8 3 | 2897 3 | 28972.69 | 28954.8 1 | 1 ~ 21aa |
| 41 | 31481 | 31480.78 | 31461.1 8 | 314 79 | 31478.76 | 31459. 16 | 2908 6 | 29085.86 | 29067.9 0 | 2908 4 | 29083.84 | 29065.8 8 | 1 ~ 21aa |
| 42 | 31085 | 31085.32 | 31065.9 7 | 310 83 | 31083.30 | 31063. 95 | 2869 0 | 28690.40 | 28672.7 0 | 2868 8 | 28688.38 | 28670.6 8 | 1 ~ 21aa |
| 43 | 31143 | 31143.41 | 31124.0 3 | 311 41 | 31141.39 | 31122. 01 | 2874 8 | 28748.49 | 28730.7 5 | 2874 6 | 28746.47 | 28728.7 3 | 1 ~ 21aa |
| 44 | 32828 | 32828.19 | 32807.8 1 | 328 26 | 32826.17 | 32805. 79 | 3043 3 | 30433.27 | 30414.5 4 | 3043 1 | 30431.25 | 30412.5 2 | 1 ~ 21aa |
| 45 | 31142 | 31142.42 | 31123.0 3 | 311 40 | 31140.40 | 31121. 01 | 2874 8 | 28747.50 | 28729.7 6 | 2874 5 | 28745.48 | 28727.7 4 | 1 ~ 21aa |
| 46 | 31125 | 31125.34 | 31105.9 6 | 311 23 | 31123.32 | 31103. 94 | 2873 0 | 28730.42 | 28712.6 8 | 2872 8 | 28728.40 | 28710.6 6 | 1~ 21aa |

**[Table 9] Mass data of OXA protein**

| Subtypes | Full-length OXA protein | | | Active protein | | | |
|---|---|---|---|---|---|---|---|
| | Da | Average molecular weight | Monoisotopic mass | Da | Average molecular weight | Monoisotopic mass | N-terminus |
| 48 | 30359 | 30358.76 | 30339.55 | 28147 | 28146.97 | 28129.28 | 1 ~ 22aa |
| 54 | 30280 | 30279.68 | 30260.45 | 28098 | 28097.89 | 28080.23 | 1 ~ 22aa |
| 162 | 30329 | 30328.74 | 30309.54 | 28117 | 28116.94 | 28099.27 | 1 ~ 22aa |
| 163 | 29891 | 29891.19 | 29872.27 | 27679 | 27679.40 | 27662.00 | 1 ~ 22aa |
| 181 | 30313 | 30312.74 | 30293.55 | 28172 | 28172.02 | 28154.31 | 1 ~ 22aa |
| 199 | 30299 | 30298.71 | 30279.52 | 28158 | 28157.99 | 28140.29 | 1 ~ 22aa |
| 204 | 30423 | 30422.85 | 30403.60 | 28282 | 28282.14 | 28264.37 | 1 ~ 22aa |
| 232 | 30244 | 30243.63 | 30224.48 | 28032 | 28031.83 | 28014.21 | 1 ~ 22aa |
| 244 | 30260 | 30259.63 | 30240.47 | 28048 | 28047.83 | 28030.20 | 1 ~ 22aa |
| 245 | 30393 | 30392.82 | 30373.57 | 28252 | 28252.11 | 28234.34 | 1 ~ 22aa |
| 247 | 29814 | 29814.11 | 29795.25 | 27673 | 27673.39 | 27656.02 | 1 ~ 22aa |
| 252 | 30331 | 30330.71 | 30311.52 | 28190 | 28189.99 | 28172.29 | 1 ~ 22aa |
| 370 | 30401 | 30400.80 | 30381.56 | 28260 | 28260.08 | 28242.33 | 1 ~ 22aa |
| 405 | 29859 | 29859.19 | 29840.28 | 27718 | 27718.48 | 27701.04 | 1 ~ 22aa |
| 416 | 30401 | 30400.84 | 30381.60 | 28260 | 28260.13 | 28242.37 | 1 ~ 22aa |
| 436 | 30285 | 30284.74 | 30265.51 | 28126 | 28125.97 | 28108.35 | 1 ~ 22aa |
| 438 | 30111 | 30111.42 | 30092.35 | 27900 | 27899.62 | 27882.08 | 1 ~ 22aa |
| 439 | 29865 | 29865.16 | 29846.26 | 27653 | 27653.36 | 27635.99 | 1 ~ 22aa |
| 484 | 30214 | 30213.60 | 30194.47 | 28002 | 28001.80 | 27984.20 | 1 ~ 22aa |
| 505 | 30389 | 30388.79 | 30369.56 | 28147 | 28146.97 | 28129.28 | 1 ~ 22aa |
| 514 | 30387 | 30386.82 | 30367.58 | 28175 | 28175.02 | 28157.31 | 1 ~ 22aa |
| 515 | 30361 | 30360.73 | 30341.53 | 28149 | 28148.94 | 28131.26 | 1 ~ 22aa |
| 517 | 30088 | 30088.47 | 30069.42 | 27877 | 27876.68 | 27859.15 | 1 ~ 22aa |
| 519 | 30373 | 30372.79 | 30353.57 | 28161 | 28160.99 | 28143.30 | 1 ~ 22aa |
| 535 | 30185 | 30184.62 | 30165.45 | 27955 | 27954.78 | 27937.26 | 1 ~ 22aa |
| 538 | 30363 | 30362.75 | 30343.53 | 28151 | 28150.96 | 28133.26 | 1 ~ 22aa |
| 546 | 30343 | 30342.76 | 30323.56 | 28131 | 28130.97 | 28113.29 | 1 ~ 22aa |
| 547 | 30427 | 30426.88 | 30407.63 | 28215 | 28215.09 | 28197.36 | 1 ~ 22aa |
| 566 | 30403 | 30402.77 | 30383.54 | 28191 | 28190.98 | 28173.27 | 1 ~ 22aa |
| 567 | 30190 | 30189.58 | 30170.47 | 27978 | 27977.78 | 27960.2 | 1 ~ 22aa |

**[Table 10] Mass data of NDM protein**

| Subtypes | Full-length NDM protein | | | Active protein (N-terminal residues 1-19) | | | Active protein (N-terminal residues 1-20) | | |
|---|---|---|---|---|---|---|---|---|---|
| | Da | Average molecular weight | Monoisoto pic mass | Da | Average molecular weight | Monoisotop ic mass | Da | Average molecular weight | Monoisotopic mass |
| 1 | 28499 | 28499.46 | 28481.22 | 26510 | 26510.04 | 26493.16 | 26439 | 26438.96 | 26422.12 |
| 2 | 28473 | 28473.42 | 28455.20 | 26484 | 26484.00 | 26467.14 | 26413 | 26412.92 | 26396.10 |
| 3 | 28498 | 28498.48 | 28480.23 | 26509 | 26509.05 | 26492.17 | 26438 | 26437.98 | 26421.14 |
| 4 | 28481 | 28481.43 | 28463.26 | 26492 | 26492.01 | 26475.20 | 26421 | 26420.93 | 26404.16 |
| 5 | 28495 | 28495.45 | 28477.27 | 26506 | 26506.03 | 26489.22 | 26435 | 26434.95 | 26418.18 |
| 6 | 28528 | 28527.51 | 28509.25 | 26538 | 26538.09 | 26521.19 | 26467 | 26467.01 | 26450.15 |
| 7 | 28480 | 28480.44 | 28462.27 | 26491 | 26491.02 | 26474.22 | 26420 | 26419.94 | 26403.18 |
| 8 | 28423 | 28423.39 | 28405.25 | 26434 | 26433.97 | 26417.19 | 26363 | 26362.89 | 26346.16 |
| 9 | 28499 | 28498.52 | 28480.27 | 26509 | 26509.10 | 26492.21 | 26438 | 26438.02 | 26421.17 |
| 10 | 28648 | 28647.58 | 28629.25 | 26658 | 26658.15 | 26641.19 | 26587 | 26587.08 | 26570.16 |
| 11 | 28467 | 28467.40 | 28449.24 | 26478 | 26477.98 | 26461.18 | 26407 | 26406.90 | 26390.15 |
| 12 | 28539 | 28539.46 | 28521.26 | 26550 | 26550.04 | 26533.21 | 26479 | 26478.96 | 26462.17 |
| 13 | 28480 | 28480.44 | 28462.27 | 26491 | 26491.02 | 26474.22 | 26420 | 26419.94 | 26403.18 |
| 14 | 28441 | 28441.42 | 28423.21 | 26452 | 26452.00 | 26435.15 | 26381 | 26380.92 | 26364.11 |
| 15 | 28509 | 28509.48 | 28491.29 | 26520 | 26520.06 | 26503.23 | 26449 | 26448.98 | 26432.19 |
| 16 | 28480 | 28480.41 | 28462.17 | 26491 | 26490.99 | 26474.11 | 26420 | 26419.91 | 26403.08 |
| 17 | 28495 | 28494.51 | 28476.33 | 26505 | 26505.09 | 26488.27 | 26434 | 26434.01 | 26417.23 |
| 18 | 29103 | 29103.10 | 29084.49 | 27114 | 27113.67 | 27096.43 | 27043 | 27042.60 | 27025.40 |
| 19 | 28509 | 28508.50 | 28490.31 | 26519 | 26519.07 | 26502.25 | 26448 | 26448.00 | 26431.21 |
| 20 | 28476 | 28476.41 | 28458.23 | 26487 | 26486.99 | 26470.17 | 26416 | 26415.91 | 26399.14 |
| 21 | 28525 | 28525.48 | 28507.28 | 26536 | 26536.06 | 26519.23 | 26465 | 26464.98 | 26448.19 |
| 22 | 28481 | 28481.43 | 28463.26 | 26492 | 26492.01 | 26475.20 | 26421 | 26420.93 | 26404.16 |
| 23 | 28499 | 28499.46 | 28481.22 | 26510 | 26510.04 | 26493.16 | 26439 | 26438.96 | 26422.12 |
| 24 | 28513 | 28513.49 | 28495.23 | 26524 | 26524.07 | 26507.17 | 26453 | 26452.99 | 26436.14 |
| 25 | 28515 | 28515.46 | 28497.21 | 26526 | 26526.04 | 26509.15 | 26455 | 26454.96 | 26438.11 |
| 27 | 28527 | 28526.53 | 28508.26 | 26537 | 26537.11 | 26520.20 | 26466 | 26466.03 | 26449.17 |
| 28 | 28528 | 28527.51 | 28509.25 | 26538 | 26538.09 | 26521.19 | 26467 | 26467.01 | 26450.15 |

**[Table 11] Mass data of IMP protein**

| Subtypes | Full-length IMP protein | | | Active form of IMP protein | | | |
|---|---|---|---|---|---|---|---|
| | Da | Average molecular weight | Monoisotopic mass | Da | Average molecular weight | Monoisotopic mass | N-terminus |
| 1 | 27120 | 27120.19 | 27103.24 | 25113 | 25112.71 | 25097.2 | 1 ~ 18aa |
| 2 | 27180 | 27180.25 | 27163.13 | 25151 | 25150.65 | 25135.08 | 1 ~ 19aa |
| 3 | 27018 | 27018.1 | 27001.21 | 25011 | 25010.62 | 24995.17 | 1 ~ 18aa |
| 4 | 27087 | 27087.2 | 27070.24 | 25080 | 25079.72 | 25064.2 | 1 ~ 18aa |
| 5 | 27176 | 27176.24 | 27159.12 | 25020 | 25019.56 | 25004.05 | 1 ~ 19aa |
| 6 | 27090 | 27090.16 | 27073.23 | 25083 | 25082.68 | 25067.19 | 1 ~ 18aa |
| 7 | 27134 | 27134.24 | 27117.21 | 25025 | 25024.6 | 25009.15 | 1 ~ 19aa |
| 8 | 27053 | 27053.06 | 27036.02 | 24952 | 24952.38 | 24936.94 | 1 ~ 20aa |
| 9 | 27277 | 27277 .22 | 27260.01 | 25192 | 25191.61 | 25175.97 | 1 ~ 18aa |
| 10 | 27168 | 27168.23 | 27151.24 | 25161 | 25160.75 | 25145.2 | 1 ~ 18aa |
| 11 | 27061 | 27061.01 | 27044.05 | 24973 | 24973.34 | 24957.93 | 1 ~ 19aa |
| 12 | 27101 | 27101.29 | 27084.27 | 24988 | 24987.6 | 24972.09 | 1 ~ 19aa |
| 13 | 27212 | 27212.26 | 27195.02 | 25078 | 25077.56 | 25061.95 | 1 ~ 20aa |
| 14 | 27441 | 27440.57 | 27423.32 | 25365 | 25364.9 | 25349.23 | 1 ~ 18aa |
| 15 | 27139 | 27139.07 | 27121.93 | 25011 | 25011.44 | 24995.94 | 1 ~ 19aa |
| 16 | 27298 | 27298.24 | 27281.06 | 25268 | 25267.62 | 25251.96 | 1 ~ 18aa |
| 17 | 27141 | 27141.14 | 27123.95 | 25006 | 25006.44 | 24990.88 | 1 ~ 20aa |
| 18 | 27199 | 27199.28 | 27182.22 | 25186 | 25185.69 | 25170.14 | 1 ~ 18aa |
| 19 | 27095 | 27095.14 | 27078.06 | 24994 | 24994.46 | 24978.98 | 1 ~ 20aa |
| 20 | 27143 | 27143.19 | 27126.06 | 25043 | 25042.5 | 25026.98 | 1 ~ 20aa |
| 21 | 27033 | 27032.96 | 27016.02 | 24945 | 24945.29 | 24929.9 | 1 ~ 19aa |
| 22 | 27224 | 27224.16 | 27207.03 | 25208 | 25207.56 | 25191.95 | 1 ~ 18aa |
| 23 | 27101 | 27101.11 | 27084.02 | 25000 | 25000.42 | 24984.94 | 1 ~ 20aa |
| 24 | 27081 | 27081.08 | 27064.02 | 24980 | 24980.39 | 24964.94 | 1 ~ 20aa |
| 25 | 27120 | 27120.19 | 27103.24 | 25113 | 25112.71 | 25097.2 | 1 ~ 18aa |
| 26 | 27135 | 27135.24 | 27118.24 | 25128 | 25127.76 | 25112.2 | 1 ~ 18aa |
| 27 | 27303 | 27303.26 | 27286.12 | 25216 | 25215.59 | 25200 | 1 ~ 19aa |
| 28 | 27139 | 27139.19 | 27122.09 | 24983 | 24982.5 | 24967.02 | 1 ~ 19aa |
| 29 | 27219 | 27219.17 | 27202.09 | 25115 | 25114.53 | 25098.96 | 1 ~ 19aa |
| 30 | 27119 | 27119.25 | 27102.29 | 25112 | 25111.77 | 25096.25 | 1 ~ 18aa |
| 31 | 27176 | 27176.19 | 27159.15 | 25116 | 25115.56 | 25100.01 | 1 ~ 18aa |
| 32 | 27490 | 27489.64 | 27472.34 | 25414 | 25413.98 | 25398.25 | 1 ~ 18aa |
| 33 | 27211 | 27211.26 | 27194.09 | 25205 | 25204.69 | 25189.08 | 1 ~ 18aa |
| 34 | 27048 | 27048.12 | 27031.22 | 25041 | 25040.64 | 25025.18 | 1 ~ 18aa |
| 35 | 27257 | 27257.13 | 27240.07 | 25139 | 25139.45 | 25123.91 | 1 ~ 19aa |
| 37 | 27388 | 27388.44 | 27371.1 | 25254 | 25253.74 | 25238.03 | 1 ~ 20aa |
| 38 | 27057 | 27057.17 | 27040.23 | 25050 | 25049.69 | 25034.19 | 1 ~ 18aa |
| 39 | 27011 | 27011.07 | 26994.03 | 24910 | 24910.39 | 24894.95 | 1 ~ 20aa |
| 40 | 27108 | 27108.13 | 27091.2 | 25101 | 25100.65 | 25085.16 | 1 ~ 18aa |
| 41 | 27109 | 27109.05 | 27092.05 | 25021 | 25021.38 | 25005.93 | 1 ~ 19aa |
| 42 | 27219 | 27219.32 | 27202.32 | 25212 | 25211.84 | 25196.28 | 1 ~ 18aa |
| 43 | 27182 | 27182.28 | 27165.21 | 25073 | 25072.65 | 25057.15 | 1 ~ 19aa |
| 44 | 27049 | 27048.96 | 27032.01 | 24961 | 24961.29 | 24945.9 | 1 ~ 19aa |
| 45 | 27247 | 27247.2 | 27230 | 25105 | 25104.53 | 25088.94 | 1 ~ 19aa |
| 46 | 26841 | 26840.97 | 26824.13 | 24831 | 24831.37 | 24816.02 | 1 ~ 18aa |
| 48 | 27429 | 27428.51 | 27411.29 | 25353 | 25352.85 | 25337.19 | 1 ~ 18aa |
| 49 | 27247 | 27247.32 | 27230.22 | 25234 | 25233.73 | 25218.14 | 1 ~ 18aa |
| 51 | 27104 | 27104.21 | 27087.2 | 24995 | 24994.58 | 24979.14 | 1 ~ 19aa |
| 52 | 27078 | 27078.11 | 27061.19 | 25071 | 25070.63 | 25055.15 | 1 ~ 18aa |
| 53 | 27237 | 27237.16 | 27219.98 | 25094 | 25094.49 | 25078.92 | 1 ~ 19aa |
| 54 | 27427 | 27426.54 | 27409.31 | 25351 | 25350.88 | 25335.21 | 1 ~ 18aa |
| 55 | 27147 | 27147.34 | 27130.36 | 25174 | 25173.88 | 25158.3 | 1 ~ 18aa |
| 56 | 27169 | 27169.25 | 27152.21 | 25156 | 25155.66 | 25140.13 | 1 ~ 18aa |
| 58 | 27272 | 27272.2 | 27255.03 | 25199 | 25198.56 | 25182.93 | 1 ~ 19aa |
| 59 | 27136 | 27136.27 | 27119.26 | 25129 | 25128.79 | 25113.22 | 1 ~ 18aa |
| 60 | 27119 | 27119.25 | 27102.29 | 25112 | 25111.77 | 25096.25 | 1 ~ 18aa |
| 61 | 27119 | 27119.24 | 27102.28 | 25112 | 25111.76 | 25096.24 | 1 ~ 18aa |
| 62 | 27109 | 27109.04 | 27091.92 | 24981 | 24981.42 | 24965.93 | 1 ~ 19aa |
| 63 | 27131 | 27131.31 | 27114.28 | 25018 | 25017.62 | 25002.1 | 1 ~ 19aa |
| 64 | 27317 | 27317.29 | 27300.14 | 25216 | 25215.59 | 25200 | 1 ~ 19aa |
| 65 | 27411 | 27410.54 | 27393.31 | 25335 | 25334.88 | 25319.22 | 1 ~ 18aa |
| 66 | 27168 | 27168.23 | 27151.24 | 25161 | 25160.75 | 25145.2 | 1 ~ 18aa |
| 67 | 27319 | 27319.26 | 27302.11 | 25232 | 25231.59 | 25216 | 1 ~ 19aa |
| 68 | 27031 | 27030.98 | 27014.04 | 24872 | 24872.24 | 24856.89 | 1 ~ 20aa |
| 69 | 27083 | 27083.09 | 27066.03 | 24982 | 24982.4 | 24966.95 | 1 ~ 20aa |
| 70 | 27120 | 27120.19 | 27103.24 | 25113 | 25112.71 | 25097.2 | 1 ~ 18aa |
| 71 | 27217 | 27217.3 | 27200.21 | 25204 | 25203.71 | 25188.13 | 1 ~ 18aa |
| 73 | 27106 | 27106.18 | 27089.18 | 24796 | 24796.35 | 24781.04 | 1 ~ 21aa |
| 74 | 27346 | 27346.28 | 27329.06 | 25259 | 25258.61 | 25242.94 | 1 ~ 19aa |
| 75 | 27227 | 27227.34 | 27210.23 | 25214 | 25213.74 | 25198.15 | 1 ~ 18aa |
| 76 | 27092 | 27092.13 | 27075.21 | 25085 | 25084.65 | 25069.17 | 1 ~ 18aa |
| 77 | 27142 | 27142.15 | 27125.19 | 25135 | 25134.67 | 25119.15 | 1 ~ 18aa |
| 78 | 27138 | 27138.21 | 27121.23 | 25131 | 25130.73 | 25115.19 | 1~18aa |
| 79 | 27148 | 27148.2 | 27131.25 | 25141 | 25140.72 | 25125.21 | 1~18aa |
| 80 | 27152 | 27152.23 | 27135.25 | 25145 | 25144.75 | 25129.2 | 1~18aa |
| 82 | 27255 | 27255.22 | 27238.03 | 25113 | 25112.56 | 25096.98 | 1~19aa |
| 83 | 27185 | 27185.25 | 27168.21 | 25172 | 25171.66 | 25156.13 | 1 ~ 18aa |
| 84 | 27260 | 27260.31 | 27243.02 | 25126 | 25125.61 | 25109.95 | 1~20aa |
| 85 | 27146 | 27146.22 | 27129.11 | 24990 | 24989.53 | 24974.04 | 1~19aa |

**[Table 12] Mass data of VIM protein**

| Subtypes | Full-length VIM protein | | | Active form of VIM protein | | | |
|---|---|---|---|---|---|---|---|
| | Da | Average molecular weight | Monoisotopic mass | Da | Average molecular weight | Monoisotopic mass | N-terminus |
| 1 | 28024 | 28024.46 | 28007.22 | 25322 | 25322.13 | 25306.78 | 1~26aa |
| 2 | 28327 | 28326.94 | 28309.51 | 25515 | 25515.42 | 25499.92 | 1~26aa |
| 3 | 28300 | 28299.96 | 28282.53 | 25488 | 25488.44 | 25472.94 | 1~26aa |
| 4 | 28094 | 28093.57 | 28076.29 | 25391 | 25391.24 | 25375.85 | 1~26aa |
| 5 | 28042 | 28041.57 | 28024.32 | 25339 | 25339.25 | 25323.88 | 1~26aa |
| 6 | 28328 | 28327.97 | 28310.54 | 25516 | 25516.45 | 25500.95 | 1~26aa |
| 7 | 28112 | 28111.92 | 28094.63 | 25464 | 25463.74 | 25448.26 | 1~25aa |
| 8 | 28297 | 28296.91 | 28279.5 | 25485 | 25485.39 | 25469.91 | 1 ~ 26aa |
| 9 | 28339 | 28338.99 | 28321.54 | 25527 | 25527.47 | 25511.95 | 1~26aa |
| 10 | 28343 | 28342.94 | 28325.5 | 25531 | 25531.42 | 25515.91 | 1~26aa |
| 11 | 28300 | 28299.91 | 28282.5 | 25488 | 25488.39 | 25472.91 | 1~26aa |
| 12 | 28117 | 28116.55 | 28099.25 | 25414 | 25414.23 | 25398.81 | 1~26aa |
| 13 | 28220 | 28219.63 | 28202.29 | 25455 | 25455.24 | 25439.83 | 1 ~ 26aa |
| 14 | 28124 | 28123.59 | 28106.3 | 25421 | 25421.27 | 25405.86 | 1 ~ 26aa |
| 15 | 28311 | 28310.94 | 28293.51 | 25499 | 25499.42 | 25483.92 | 1 ~ 26aa |
| 16 | 28353 | 28353.02 | 28335.56 | 25542 | 25541.5 | 25525.97 | 1 ~ 26aa |
| 17 | 28345 | 28344.97 | 28327.46 | 25515 | 25515.42 | 25499.92 | 1 ~ 26aa |
| 18 | 27941 | 27940.58 | 27923.37 | 25129 | 25129.06 | 25113.79 | 1 ~ 26aa |
| 19 | 28108 | 28107.64 | 28090.35 | 25405 | 25405.31 | 25389.9 | 1 ~ 26aa |
| 20 | 28346 | 28345.98 | 28328.55 | 25534 | 25534.46 | 25518.96 | 1 ~ 26aa |
| 23 | 28258 | 28257.83 | 28240.44 | 25446 | 25446.31 | 25430.85 | 1 ~ 26aa |
| 24 | 28284 | 28283.91 | 28266.49 | 25472 | 25472.39 | 25456.9 | 1 ~ 26aa |
| 25 | 28147 | 28146.76 | 28129.42 | 25444 | 25444.43 | 25428.98 | 1 ~ 26aa |
| 26 | 28000 | 28000.47 | 27983.25 | 25298 | 25298.15 | 25282.81 | 1 ~ 26aa |
| 27 | 28040 | 28040.46 | 28023.22 | 25338 | 25338.13 | 25322.78 | 1 ~ 26aa |
| 28 | 28070 | 28069.58 | 28052.32 | 25367 | 25367.26 | 25351.88 | 1 ~ 26aa |
| 29 | 28058 | 28057.57 | 28040.32 | 25355 | 25355.25 | 25339.88 | 1 ~ 26aa |
| 30 | 28354 | 28353.96 | 28336.52 | 25542 | 25542.44 | 25526.93 | 1 ~ 26aa |
| 31 | 28320 | 28319.95 | 28302.54 | 25508 | 25508.43 | 25492.96 | 1 ~ 26aa |
| 32 | 27966 | 27966.42 | 27949.22 | 25264 | 25264.1 | 25248.78 | 1 ~ 26aa |
| 33 | 28008 | 28008.46 | 27991.23 | 25306 | 25306.13 | 25290.79 | 1 ~ 26aa |
| 34 | 28038 | 28038.48 | 28021.24 | 25336 | 25336.16 | 25320.8 | 1 ~ 26aa |
| 35 | 28054 | 28054.48 | 28037.23 | 25352 | 25352.16 | 25336.79 | 1 ~ 26aa |
| 36 | 28355 | 28354.99 | 28337.55 | 25543 | 25543.47 | 25527.96 | 1 ~ 26aa |
| 37 | 28110 | 28109.56 | 28092.29 | 25407 | 25407.24 | 25391.85 | 1 ~ 26aa |
| 38 | 28052 | 28051.59 | 28034.4 | 25367 | 25367.3 | 25351.91 | 1 ~ 26aa |
| 39 | 27970 | 27970.45 | 27953.24 | 25268 | 25268.12 | 25252.8 | 1 ~ 26aa |
| 40 | 28122 | 28121.62 | 28104.32 | 25419 | 25419.29 | 25403.88 | 1 ~ 26aa |
| 41 | 28326 | 28325.95 | 28308.52 | 25514 | 25514.43 | 25498.93 | 1 ~ 26aa |
| 42 | 28038 | 28038.48 | 28021.24 | 25336 | 25336.16 | 25320.8 | 1 ~ 26aa |
| 43 | 28122 | 28121.62 | 28104.32 | 25391 | 25391.24 | 25375.85 | 1 ~ 26aa |
| 44 | 28313 | 28312.87 | 28295.46 | 25501 | 25501.35 | 25485.87 | 1 ~ 26aa |
| 45 | 28339 | 28338.99 | 28321.54 | 25527 | 25527.47 | 25511.95 | 1 ~ 26aa |
| 46 | 28333 | 28332.9 | 28315.46 | 25487 | 25487.36 | 25471.89 | 1 ~ 26aa |
| 47 | 28240 | 28239.62 | 28222.26 | 25455 | 25455.24 | 25439.83 | 1 ~ 26aa |
| 48 | 28361 | 28360.96 | 28343.49 | 25515 | 25515.42 | 25499.92 | 1 ~ 26aa |
| 49 | 28058 | 28057.57 | 28040.32 | 25355 | 25355.25 | 25339.88 | 1 ~ 26aa |
| 50 | 28257 | 28256.88 | 28239.48 | 25445 | 25445.36 | 25429.89 | 1 ~ 26aa |
| 51 | 28355 | 28354.99 | 28337.54 | 25543 | 25543.47 | 25527.95 | 1 ~ 26aa |
| 52 | 28044 | 28043.5 | 28026.27 | 25341 | 25341.18 | 25325.82 | 1 ~ 26aa |
| 53 | 28210 | 28209.74 | 28192.41 | 25487 | 25487.36 | 25471.89 | 1 ~ 26aa |
| 54 | 28067 | 28066.54 | 28049.28 | 25364 | 25364.22 | 25348.84 | 1 ~ 26aa |
| 55 | 28127 | 28126.68 | 28109.39 | 25424 | 25424.36 | 25408.95 | 1 ~ 26aa |
| 56 | 28243 | 28242.82 | 28225.44 | 25458 | 25458.37 | 25442.9 | 1 ~ 26aa |
| 57 | 28024 | 28024.46 | 28007.22 | 25322 | 25322.13 | 25306.78 | 1 ~ 26aa |
| 58 | 28304 | 28303.9 | 28286.47 | 25458 | 25458.37 | 25442.9 | 1 ~ 26aa |
| 59 | 28044 | 28043.5 | 28026.27 | 25341 | 25341.18 | 25325.82 | 1 ~ 26aa |
| 60 | 28303 | 28302.96 | 28285.53 | 25491 | 25491.44 | 25475.94 | 1 ~ 26aa |
| 61 | 28161 | 28160.99 | 28143.65 | 25513 | 25512.82 | 25497.28 | 1 ~ 25aa |
| 62 | 28355 | 28354.99 | 28337.54 | 25515 | 25515.42 | 25499.92 | 1 ~ 26aa |
| 63 | 28327 | 28326.94 | 28309.51 | 25515 | 25515.42 | 25499.92 | 1 ~ 26aa |
| 64 | 28051 | 28050.54 | 28033.28 | 25348 | 25348.21 | 25332.83 | 1 ~ 26aa |
| 65 | 28327 | 28326.94 | 28309.51 | 25515 | 25515.42 | 25499.92 | 1 ~ 26aa |
| 66 | 28329 | 28329.04 | 28311.58 | 25518 | 25517.52 | 25502 | 1 ~ 26aa |
| 67 | 28299 | 28298.88 | 28281.48 | 25487 | 25487.36 | 25471.89 | 1 ~ 26aa |
| 68 | 28053 | 28052.51 | 28035.26 | 25350 | 25350.19 | 25334.81 | 1 ~ 26aa |

**[Table 13] Mass data of GES protein**

| Subty pes | Full-length GES protein | | | Full-length GES protein (containing disulfide bond) | | | Active protein | | | Active protein (containing disulfide bond) | | |
|---|---|---|---|---|---|---|---|---|---|---|---|---|
| | Da | Averag e molecul ar weight | Monoisotopi c mass | Da | Averag e molecul ar weight | Monoisotopi c mass | Da | Averag e molecul ar weight | Monoisotopi c mass | Da | Averag e molecul ar weight | Monoisotopi c mass |
| 1 | 311 54 | 31154.4 8 | 31134.97 | 311 52 | 31152.4 6 | 31132.95 | 292 17 | 29217.1 4 | 29198.92 | 292 15 | 29215.1 2 | 29196.90 |
| 2 | 312 12 | 31211.5 3 | 31191.99 | 312 10 | 31209.5 1 | 31189.97 | 292 74 | 29274.1 9 | 29255.94 | 292 72 | 29272.1 7 | 29253.92 |
| 3 | 311 23 | 31123.4 5 | 31104.03 | 311 21 | 31121.4 3 | 31102.01 | 291 86 | 29186.1 1 | 29167.97 | 291 84 | 29184.0 9 | 29165.95 |
| 4 | 311 53 | 31153.4 8 | 31134.04 | 311 51 | 31151.4 6 | 31132.02 | 292 16 | 29216.1 4 | 29197.99 | 292 14 | 29214.1 2 | 29195.97 |
| 5 | 311 85 | 31184.5 0 | 31164.98 | 311 82 | 31182.4 8 | 31162.96 | 292 47 | 29247.1 7 | 29228.93 | 292 45 | 29245.1 5 | 29226.91 |
| 6 | 311 84 | 31183.5 6 | 31164.03 | 311 82 | 31181.5 4 | 31162.01 | 292 46 | 29246.2 3 | 29227.98 | 292 44 | 29244.2 1 | 29225.96 |
| 7 | 311 54 | 31153.5 4 | 31134.02 | 311 52 | 31151.5 2 | 31132.00 | 292 16 | 29216.2 0 | 29197.97 | 292 14 | 29214.1 8 | 29195.95 |
| 8 | 311 97 | 31196.5 6 | 31177.01 | 311 95 | 31194.5 4 | 31174.99 | 292 59 | 29259.2 2 | 29240.96 | 292 57 | 29257.2 0 | 29238.94 |
| 9 | 311 85 | 31184.5 0 | 31164.98 | 311 82 | 31182.4 8 | 31162.96 | 292 47 | 29247.1 7 | 29228.93 | 292 45 | 29245.1 5 | 29226.91 |
| 10 | 311 28 | 31128.4 0 | 31108.91 | 311 26 | 31126.3 8 | 31106.89 | 292 03 | 29203.1 2 | 29184.90 | 292 01 | 29201.1 0 | 29182.88 |
| 11 | 311 69 | 31168.5 0 | 31148.98 | 311 66 | 31166.4 8 | 31146.96 | 292 31 | 29231.1 7 | 29212.93 | 292 29 | 29229.1 5 | 29210.91 |
| 12 | 311 38 | 31138.4 8 | 31118.97 | 311 36 | 31136.4 6 | 31116.95 | 292 01 | 29201.1 4 | 29182.92 | 291 99 | 29199.1 2 | 29180.90 |
| 13 | 312 11 | 31210.5 9 | 31191.04 | 312 09 | 31208.5 7 | 31189.02 | 292 73 | 29273.2 5 | 29254.99 | 292 71 | 29271.2 3 | 29252.97 |
| 14 | 311 99 | 31198.5 3 | 31178.99 | 311 97 | 31196.5 1 | 31176.97 | 292 61 | 29261.2 0 | 29242.94 | 292 59 | 29259.1 8 | 29240.92 |
| 15 | 311 74 | 31174.4 7 | 31154.96 | 311 72 | 31172.4 5 | 31152.94 | 292 37 | 29237.1 3 | 29218.90 | 292 35 | 29235.1 1 | 29216.88 |
| 16 | 311 85 | 31185.4 9 | 31165.96 | 311 83 | 31183.4 7 | 31163.94 | 292 48 | 29248.1 5 | 29229.91 | 292 46 | 29246.1 3 | 29227.89 |
| 17 | 311 68 | 31167.5 6 | 31148.03 | 311 66 | 31165.5 4 | 31146.01 | 292 30 | 29230.2 3 | 29211.98 | 292 28 | 29228.2 1 | 29209.96 |
| 18 | 311 | 31198.5 | 31178.99 | 311 | 31196.5 | 31176.97 | 292 | 29261.2 | 29242.94 | 292 | 29259.1 | 29240.92 |
| | 99 | 3 | | 97 | 1 | | 61 | 0 | | 59 | 8 | |
| 19 | 311 83 | 31182.5 3 | 31163.00 | 311 81 | 31180.5 1 | 31160.98 | 292 31 | 29231.1 7 | 29212.93 | 292 29 | 29229.1 5 | 29210.91 |
| 20 | 311 99 | 31198.5 3 | 31178.99 | 311 97 | 31196.5 1 | 31176.97 | 292 47 | 29247.1 7 | 29228.93 | 292 45 | 29245.1 5 | 29226.91 |
| 21 | 311 50 | 31150.4 9 | 31130.99 | 311 48 | 31148.4 7 | 31128.97 | 292 13 | 29213.1 5 | 29194.94 | 292 11 | 29211.1 3 | 29192.92 |
| 22 | 311 50 | 31150.4 7 | 31131.02 | 311 48 | 31148.4 5 | 31129.00 | 292 13 | 29213.1 4 | 29194.97 | 292 11 | 29211.1 2 | 29192.95 |
| 23 | 311 54 | 31154.4 8 | 31134.97 | 311 52 | 31152.4 6 | 31132.95 | 292 17 | 29217.1 4 | 29198.92 | 292 15 | 29215.1 2 | 29196.90 |
| 24 | 311 54 | 31154.4 2 | 31134.98 | 311 52 | 31152.4 0 | 31132.96 | 292 17 | 29217.0 8 | 29198.93 | 292 15 | 29215.0 6 | 29196.91 |
| 25 | 311 86 | 31186.4 8 | 31166.98 | 311 84 | 31184.4 6 | 31164.96 | 292 75 | 29275.1 8 | 29256.93 | 292 73 | 29273.1 6 | 29254.91 |
| 26 | 311 69 | 31168.5 0 | 31148.98 | 311 66 | 31166.4 8 | 31146.96 | 292 17 | 29217.1 4 | 29198.92 | 292 15 | 29215.1 2 | 29196.90 |
| 27 | 312 16 | 31215.5 2 | 31195.98 | 312 14 | 31213.5 0 | 31193.96 | 292 78 | 29278.1 8 | 29259.93 | 292 76 | 29276.1 6 | 29257.91 |
| 28 | 311 84 | 31183.5 2 | 31163.99 | 311 82 | 31181.5 0 | 31161.97 | 292 46 | 29246.1 8 | 29227.94 | 292 44 | 29244.1 6 | 29225.92 |
| 29 | 311 58 | 31158.4 7 | 31138.96 | 311 56 | 31156.4 5 | 31136.94 | 292 21 | 29221.1 3 | 29202.91 | 292 19 | 29219.1 1 | 29200.89 |
| 30 | 311 31 | 31131.4 6 | 31111.88 | 311 29 | 31129.4 4 | 31109.86 | 291 94 | 29194.1 2 | 29175.83 | 291 92 | 29192.1 0 | 29173.81 |
| 31 | 311 55 | 31154.5 2 | 31135.00 | 311 53 | 31152.5 0 | 31132.98 | 292 17 | 29217.1 9 | 29198.95 | 292 15 | 29215.1 7 | 29196.93 |
| 32 | 312 33 | 31233.4 8 | 31214.02 | 312 31 | 31231.4 6 | 31212.00 | 293 38 | 29338.2 3 | 29320.02 | 293 36 | 29336.2 1 | 29318.00 |
| 33 | 311 54 | 31153.5 4 | 31134.02 | 311 52 | 31151.5 2 | 31132.00 | 292 16 | 29216.2 0 | 29197.97 | 292 14 | 29214.1 8 | 29195.95 |
| 34 | 311 69 | 31169.4 9 | 31149.98 | 311 67 | 31167.4 7 | 31147.96 | 292 32 | 29232.1 6 | 29213.93 | 292 30 | 29230.1 4 | 29211.91 |
| 35 | 311 65 | 31164.5 0 | 31145.04 | 311 62 | 31162.4 8 | 31143.02 | 292 27 | 29227.1 6 | 29208.99 | 292 25 | 29225.1 4 | 29206.97 |
| 36 | 311 84 | 31183.5 6 | 31164.03 | 311 82 | 31181.5 4 | 31162.01 | 292 46 | 29246.2 3 | 29227.98 | 292 44 | 29244.2 1 | 29225.96 |
| 37 | 311 88 | 31188.4 9 | 31168.97 | 311 86 | 31186.4 7 | 31166.95 | 292 51 | 29251.1 6 | 29232.92 | 292 49 | 29249.1 4 | 29230.90 |
| 38 | 311 40 | 31139.5 1 | 31119.99 | 311 37 | 31137.4 9 | 31117.97 | 292 02 | 29202.1 7 | 29183.94 | 292 00 | 29200.1 5 | 29181.92 |
| 39 | 311 12 | 31112.4 4 | 31092.95 | 311 10 | 31110.4 2 | 31090.93 | 291 75 | 29175.1 0 | 29156.90 | 291 73 | 29173.0 8 | 29154.88 |
| 40 | 312 13 | 31212.5 6 | 31193.01 | 312 11 | 31210.5 4 | 31190.99 | 292 61 | 29261.2 0 | 29242.94 | 292 59 | 29259.1 8 | 29240.92 |
| 41 | 312 64 | 31264.4 5 | 31244.98 | 312 62 | 31262.4 3 | 31242.96 | 293 69 | 29369.2 0 | 29350.98 | 293 67 | 29367.1 8 | 29348.96 |
| 42 | 315 98 | 31597.9 8 | 31578.21 | 315 96 | 31595.9 6 | 31576.19 | 296 61 | 29660.6 5 | 29642.16 | 296 59 | 29658.6 3 | 29640.14 |
| 43 | 312 | 31200.5 | 31180.96 | 311 | 31198.4 | 31178.94 | 292 | 29237.1 | 29218.90 | 292 | 29235.1 | 29216.88 |
| | 01 | 0 | | 98 | 8 | | 37 | 3 | | 35 | 1 | |

### Example 8: Genotype identification and protein identification of MBL proteins and GES derived from clinical strains

In order to identify clinical strain-derived NDM, IMP and VIM (MBL proteins) and GES (Class A carbapenem protein), a strain confirmed as positive for CRE (Carbapenem-Resistant Enterobacteriaceae) was collected. The collected strain was genotyped for each gene using the PCR method.

The strain whose genotype was confirmed was cultured using LB liquid medium, and whether MBL proteins (NDM, IMP and VIM) and GES protein were expressed was analyzed by SDS-PAGE gel analysis (FIG. 2).

The clinical strain-derived MBL proteins and GES protein were digested by the in-gel digestion method and identified by the Bottom-Up method using the Q-Exactive HF-X plus system. It was confirmed that, for NDM, the sequence coverage for the identified peptides was 61.11% (165/270) in the full-length protein sequence and 65.74% (165/251) in the active form from which the N-terminus has been removed. It was confirmed that, for IMP and VIM, the sequence coverages for the identified peptides were 22.76% (56/246) and 44.74% (119/266), respectively, in the full-length protein sequences, and 24.56% (56/228) and 49.58% (119/240), respectively, in the active forms from which the N-terminus has been removed. In addition, it was confirmed that, for GES, the sequence coverage for the identified peptides were 67.25% (193/287) in the full-length amino acid sequence, and 71.75% (193/269) in the active protein sequence.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method for detecting in a biological sample a pathogenic strain having resistance to carbapenem antibiotics, comprising:
(a) isolating a protein expressed by a pathogenic strain in a biological sample isolated from a subject; and
(b) performing top-down mass spectrometry on the isolated protein,
wherein it is determined that the pathogenic strain having resistance to carbapenem antibiotics is present in the biological sample, when a protein having the same mass as *Klebsiella pneumoniae* carbapenemase (KPC) or OXA carbapenemase from which 21 or 22 amino acid residues at the N-terminus have been removed is detected as a result of the mass spectrometry or when a protein having the same mass as at least one carbapenemase selected from the group consisting of New Delhi Metallobeta-lactamase NDM), imipenemase (IMP), Verona integron-borne metallo-β-lactamase (VIM) and Guiana extended spectrum β-lactamase (GES), from which 18, 19, 20, 21 or 26 amino acid residues at the N-terminus have been removed, is detected as a result of the mass spectrometry.

2. The method of claim 1, further comprising performing ion exchange chromatography on the protein isolated in step (a).

3. The method of claim 2, wherein the ion exchange chromatography is any one selected from the group consisting of anion exchange chromatography, cation exchange chromatography, and a sequential combination thereof.

4. The method of claim 1, wherein step (a) is performed by adding a surfactant to the biological sample.

5. The method of claim 4, wherein step (a) is performed by additionally adding a lysis buffer to the biological sample.

6. The method of claim 4, further comprising a sonication step between step (a) and step (b).

7. The method of claim 1, wherein step (b) is performed using a mass spectrometry method selected from the group consisting of matrix-assisted laser desorption/ionization time of flight (MALDI-TOF) mass spectrometry, surface enhanced laser desorption/ionization time of flight (SELDI-TOF) mass spectrometry, electrospray ionization time-of-flight (ESI-TOF) mass spectrometry, liquid chromatography-mass spectrometry (LC-MS), and liquid chromatography-mass spectrometry/mass spectrometry (LC-MS/MS).

8. The method of claim 7, wherein step (b) is performed using matrix-assisted laser desorption/ionization time of flight (MALDI-TOF) mass spectrometry.

9. The method of claim 1, wherein the carbapenemase is a KPC protein, and it is determined that the pathogenic strain having resistance to carbapenem antibiotics is present in the biological sample, when a protein having the same mass as the KPC protein from which 21 amino acid residues at the N-terminus have been removed is detected as a result of the mass spectrometry.

10. The method of claim 1, wherein the carbapenemase is an OXA protein, and it is determined that the pathogenic strain having resistance to carbapenem antibiotics is present in the biological sample, when a protein having the same mass as the OXA protein from which 22 amino acid residues at the N-terminus have been removed is detected as a result of the mass spectrometry.

11. The method of claim 1, wherein the carbapenemase is an NDM protein, and it is determined that the pathogenic strain having resistance to carbapenem antibiotics is present in the biological sample, when a protein having the same mass as the NDM protein from which 19 or 20 amino acid residues at the N-terminus have been removed is detected as a result of the mass spectrometry.

12. The method of claim 1, wherein the carbapenemase is an IMP protein, and it is determined that the pathogenic strain having resistance to carbapenem antibiotics is present in the biological sample, when a protein having the same mass as the IMP protein from which 18 to 21 amino acid residues at the N-terminus have been removed is detected as a result of the mass spectrometry.

13. The method of claim 1, wherein the carbapenemase is a VIM protein, and it is determined that the pathogenic strain having resistance to carbapenem antibiotics is present in the biological sample, when a protein having the same mass as the VIM protein from which 25 or 26 amino acid residues at the N-terminus have been removed is detected as a result of the mass spectrometry.

14. The method of claim 1, wherein the carbapenemase is a GES protein, and it is determined that the pathogenic strain having resistance to carbapenem antibiotics is present in the biological sample, when a protein having the same mass as the GES protein from which 18 amino acid residues at the N-terminus have been removed is detected as a result of the mass spectrometry.

15. The method of claim 1, wherein it is determined that the pathogenic strain having resistance to carbapenem antibiotics is present in the biological sample, when one or more mass values (m/z × z) selected from the group consisting of 28720, 28746, 28737, 28780, 28678, 28728, 28704, 28806, 28736, 28738, 28688, 28562, 28877, 28676, 28686, 28733, 28690, 28557, 28718, 28963, 28716, 28588, 29105, 28795, 28825, 28769, 29631, 28760, 28656, 28776, 28975, 29086, 28748, 30433, 28730 and values within the ranges of these values ±5 are detected as a result of the mass spectrometry.

16. The method of claim 15, wherein the pathogenic strain having resistance to carbapenem antibiotics is a KPC protein-producing strain.

17. The method of claim 15, wherein the mass values (m/z × z) additionally include a mass value that increased by 16 from each mass value.

18. The method of claim 15 or 17, wherein the mass values (m/z × z) additionally include a mass value that decreased by 2 from each mass value.

19. The method of claim 1, wherein it is determined that the pathogenic strain having resistance to carbapenem antibiotics is present in the biological sample, when one or more mass values (m/z × z) selected from the group consisting of 28147, 28098, 28117, 27679, 28172, 28158, 28282, 28032, 28048, 28252, 27673, 28190, 28260, 27718, 28126, 27900, 27653, 28002, 28175, 28149, 27877, 28161, 27955, 28151, 28131, 28215, 28191, 27978 and values within the ranges of these values ±5 are detected as a result of the mass spectrometry.

20. The method of claim 19, wherein the pathogenic strain having resistance to carbapenem antibiotics is an OXA protein-producing strain.

21. The method of claim 19, wherein the mass values (m/z × z) additionally include a mass value that increased by 16, 32 or 48 from each mass value.

22. The method of claim 1, wherein it is determined that the pathogenic strain having resistance to carbapenem antibiotics is present in the biological sample, when one or more mass values (m/z × z) selected from the group consisting of 26439, 26413, 26438, 26421, 26435, 26467, 26420, 26363, 26587, 26407, 26479, 26381, 26449, 26434, 27043, 26448, 26416, 26465, 26453, 26455, 26466, 26510, 26484, 26509, 26492, 26506, 26538, 26491, 26434, 26658, 26478, 26550, 26452, 26520, 26505, 27114, 26519, 26487, 26536, 26524, 26526, 26537 and values within the ranges of these values ±5 are detected as a result of the mass spectrometry.

23. The method of claim 22, wherein the pathogenic strain having resistance to carbapenem antibiotics is an NDM protein-producing strain.

24. The method of claim 22, wherein the mass values (m/z × z) additionally include a mass value that increased by 16 or 32 from each mass value.

25. The method of claim 22 or 24, wherein the mass values (m/z × z) additionally include a mass value that increased by 14, 28 or 42 from each mass value.

26. The method of claim 22 or 24, wherein the mass values (m/z × z) additionally include a mass value that increased by 238 from each mass value.

27. The method of claim 25, wherein the mass values (m/z × z) additionally include a mass value that increased by 238 from each mass value.

28. The method of claim 1, wherein it is determined that the pathogenic strain having resistance to carbapenem antibiotics is present in the biological sample, when one or more mass values (m/z × z) selected from the group consisting of 25113, 25151, 25011, 25080, 25020, 25083, 25025, 24952, 25192, 25161, 24973, 24988, 25078, 25365, 25268, 25006, 25186, 24994, 25043, 24945, 25208, 25000, 24980, 25128, 25216, 24983, 25115, 25112, 25116, 25414, 25205, 25041, 25139, 25254, 25050, 24910, 25101, 25021, 25212, 25073, 24961, 25105, 24831, 25353, 25234, 24995, 25071, 25094, 25351, 25174, 25156, 25199, 25129, 24981, 25018, 25335, 25232, 24872, 24982, 25204, 24796, 25259, 25214, 25085, 25135, 25131, 25141, 25145, 25172, 25126, 24990 and values within the ranges of these values ±5 are detected as a result of the mass spectrometry.

29. The method of claim 28, wherein the pathogenic strain having resistance to carbapenem antibiotics is an IMP protein-producing strain.

30. The method of claim 1, wherein it is determined that the pathogenic strain having resistance to carbapenem antibiotics is present in the biological sample, when one or more mass values (m/z × z) selected from the group consisting of 25322, 25515, 25488, 25391, 25339, 25516, 25464, 25485, 25527, 25531, 25414, 25455, 25421, 25499, 25542, 25129, 25405, 25534, 25446, 25472, 25444, 25298, 25338, 25367, 25355, 25508, 25264, 25306, 25336, 25352, 25543, 25407, 25268, 25419, 25514, 25501, 25487, 25445, 25341, 25364, 25424, 25458, 25491, 25513, 25348, 25518, 25350 and values within the ranges of these values ±5 are detected as a result of the mass spectrometry.

31. The method of claim 30, wherein the pathogenic strain having resistance to carbapenem antibiotics is a VIM protein-producing strain.

32. The method of claim 1, wherein the pathogenic strain having resistance to carbapenem antibiotics is present in the biological sample, when one or more mass values (m/z × z) selected from the group consisting of 29217, 29274, 29186, 29216, 29247, 29246, 29259, 29203, 29231, 29201, 29273, 29261, 29237, 29248, 29230, 29213, 29275, 29278, 29221, 29194, 29338, 29232, 29227, 29251, 29202, 29175, 29369, 29661 and values within the ranges of these values ±5 are detected as a result of the mass spectrometry.

33. The method of claim 32, wherein the pathogenic strain having resistance to carbapenem antibiotics is a GES protein-producing strain.

34. The method of claim 32, wherein the mass values (m/z × z) additionally include a mass value that increased by 16 or 32 from each mass value.

35. The method of claim 32 or 34, wherein the mass values (m/z × z) additionally include a mass value that decreased by 2 from each mass value.
